# EUROPEAN PATENT SPECIFICATION

(11) **EP 3 319 976 B1**
(45) Date of publication and mention of the grant of the patent: **01.09.2021**
(21) Application number: 16822088.7
(22) Date of filing: 11.07.2016
(51) Int. Cl.: A61K 9/127, C07H 21/04, C12N 15/88, A61K 48/00, A61K 9/00, A61K 47/24, A61K 31/713, A61P 11/00

(54) **COMPOSITIONS AND METHODS FOR TREATING LUNG DISEASES AND LUNG INJURY**
ZUSAMMENSETZUNGEN UND VERFAHREN ZUR BEHANDLUNG VON LUNGENERKRANKUNGEN UND LUNGENLÄSIONEN
COMPOSITIONS ET PROCÉDÉS POUR TRAITER DES MALADIES PULMONAIRES ET DES LÉSIONS PULMONAIRES

(30) Priority: 09.07.2015 US 201562190583 P
(43) Date of publication of application: 16.05.2018
(73) Proprietor: Insmed Incorporated, Bridgewater, NJ 08807-1704 (US)
(72) Inventor: CHEN, Kuan-Ju, Bridgewater, New Jersey 08807-3365 (US); LEIFER, Franziska, Bridgewater, New Jersey 08807-3365 (US); MALININ, Vladimir, Bridgewater, New Jersey 08807-3365 (US); PERKINS, Walter, Bridgewater, New Jersey 08807-3365 (US); ZHANG, Jimin, Bridgewater, New Jersey 08807-3365 (US); DIPETRILLO, Keith, Bridgewater, New Jersey 08807-3365 (US)
(74) Representative: Cooley (UK) LLP
(86) International application number: PCT/US2016/041776
(87) International publication number: WO 2017/008076

(56) References cited:
- EP-A1- 2 199 298
- WO-A1-2011/108955
- WO-A1-2014/052634
- US-A1- 2011 256 175
- US-A1- 2014 308 304

### SEQUENCE LISTING

<110> Insmed, Inc. Chen, Kuan-Ju Leifer, Franziska Malinin, Vladimir Perkins, Walter Zhang, Jimin DiPetrillo, Keith
<120> COMPOSITIONS AND METHODS FOR TREATING LUNG DISEASES AND LUNG INJURY
<130> INMD-125/01WO 315953-3361
<150> US 62/190,583
   <151> 2015-07-09
<160> 24
<170> PatentIn version 3.5
<210> 1
   <211> 2676
   <212> DNA
   <213> Homo sapiens
<400> 1
   gaattctctc tccagcagcc ctgccagatg cccgcccagc ccctgcctca ggcggggagg      60
   gcttcaggga agctcaccaa ggcagaaggg cgggagagat tgtcagagcc ccagctggtg     120
   tccagggact gaccgtgagc ctgggtgaaa gtgagttccc cgttggaggc aacagacgag     180
   gagaggatgg aaggcctggc ccccaagaat gagccctgag gttcagggag cggctggagt     240
   gagccggccc cagatctccg tccagctgcg ggtcccagag gcctgggtta cactcgcagc     300
   tcctggggga ggcccttgac gtgcctcagt tcccaaacag gaaccctggg aaggaccaga     360
   gaagtgccta ttgcgcagtg agtgcccgac acagctgcat gtggccggta tcacagggcc     420
   ctgggtaaac tgaggcaggc gacacagctg catgtggccg gtatcacagg gccctgggta     480
   aactgaggca ggcgacacag ctgcatgtgg ccggtatcac agggccctgg gtaaactgag     540
   gcaggcgaca cagctgcatg tggccgtatc acagggccct gggtaaactg aggcaggtga     600
   cacagctgca tgtggccggt atcacggggc cctggataaa cagaggcagg cgacacagct     660
   gcatgtggcc ggtatcacgg ggccctgggt aaactgaggc aggcgaggcc acccccatca     720
   agtccctcag gtctaggttt ggcaggtttg gcaaaaacac agcaacgctc ggttaaatct     780
   gaatttcggg taagtatatc ctgggcctca tttggaagag acttagatta aaaaaaaaac     840
   gtcgagacca gcccggccaa cacggtgaaa ccccgtctct actaaaaata caaaaaatta     900
   gccaggcgca gtggctcacg cctgtgatcc cagcactctg ggaggctgag gcaggcggat     960
   cacccgaggt cagatgttca agaccagcct ggccgacagg gcgaaacact gtctctacta    1020
   caaatacaaa aattagccgg gagtggtggc aggtgcctgt aatctcagct attcaggagg    1080
   ctgaggcagg agaatcactt gaacctggga ggcggaggtt gccgtgagcc gggatcacgc 1140
   caccgcactc cagcctgggc gatagagcaa gactctgtct ccaaaaaaat aaattaaaaa 1200
   acccacattg attatctgac atttgaatgc gattgtgcat cctgaatttt gtctggaggc 1260
   cccacccgag ccaatccagc gtcttgtccc ccttctcccc cttttcatca acgccctgtg 1320
   ccaggggaga ggaagtggag ggcgctggcc ggccgtgggg caatgcaacg gcctcccagc 1380
   acagggctat aagaggagcc gggcgggcac ggaggggcag agaccccgga gccccagccc 1440
   caccatgacc ctcggccgcc gactcgcgtg tcttttcctc gcctgtgtcc tgccggcctt 1500
   gctgctgggg ggtgagtttt tgagtccaac ctcccgctgc tccctctgtc ccgggttctg 1560
   tgacaggctc cttggcaggc actcagcacc cgcacccggt gtgtccccag gcaccgcgct 1620
   ggcctcggag attgtggggg gccggcgagc gcggccccac gcgtggccct tcatggtgtc 1680
   cctgcagctg cgcggaggcc acttctgcgg cgccaccctg attgcgccca acttcgtcat 1740
   gtcggccgcg cactgcgtgg cgaatgtgta agtagccggg agtgtgcgcg cccggctcgg 1800
   catctcgcac gccgcgtgcc ccgacccccg gggccgcccc tgagccccgc ctctccctcc 1860
   ccggcagaaa cgtccgcgcg gtgcgggtgg tcctgggagc ccataacctc tcgcggcggg 1920
   agcccacccg gcaggtgttc gccgtgcagc gcatcttcga aaacggctac gaccccgtaa 1980
   acttgctcaa cgacatcgtg attctccagg tgccgccggg cgggcggggg cgcaggggcg 2040
   gaggcagagc gtggggaggc atcactgccc cgtgtgacgc gctgacgatc tgtccccacc 2100
   gccacagctc aacgggtcgg ccaccatcaa cgccaacgtg caggtggccc agctgccggc 2160
   tcagggacgc cgcctgggca acggggtgca gtgcctggcc atgggctggg gccttctggg 2220
   caggaaccgt gggatcgcca gcgtcctgca ggagctcaac gtgacggtgg tgacgtccct 2280
   ctgccgtcgc agcaacgtct gcactctcgt gaggggccgg caggccggcg tctgtttcgt 2340
   acgtgccctg ggtgtccctc tgctccccac ccgctcccag cccggacttg ggcagggcct 2400
   cgcagtccag cttccccacc ttgtctgcct ccacaggggg actccggcag ccccttggtc 2460
   tgcaacgggc taatccacgg aattgcctcc ttcgtccggg gaggctgcgc ctcagggctc 2520
   taccccgatg cctttgcccc ggtggcacag tttgtaaact ggatcgactc tatcatccaa 2580
   cgctccgagg acaacccctg tccccacccc cgggacccgg acccggccag caggacccac 2640
   tgagaagggc tgcccgggtc acctcagctg cccaca 2676
<210> 2
   <211> 938
   <212> DNA
   <213> Homo sapiens
<400> 2
   gcacggaggg gcagagaccc cggagcccca gccccaccat gaccctcggc cgccgactcg        60
   cgtgtctttt cctcgcctgt gtcctgccgg ccttgctgct ggggggcacc gcgctggcct      120
   cggagattgt ggggggccgg cgagcgcggc cccacgcgtg gcccttcatg gtgtccctgc      180
   agctgcgcgg aggccacttc tgcggcgcca ccctgattgc gcccaacttc gtcatgtcgg      240
   ccgcgcactg cgtggcgaat gtaaacgtcc gcgcggtgcg ggtggtcctg ggagcccata      300
   acctctcgcg gcgggagccc acccggcagg tgttcgccgt gcagcgcatc ttcgaaaacg      360
   gctacgaccc cgtaaacttg ctcaacgaca tcgtgattct ccagctcaac gggtcggcca      420
   ccatcaacgc caacgtgcag gtggcccagc tgccggctca gggacgccgc ctgggcaacg      480
   gggtgcagtg cctggccatg ggctggggcc ttctgggcag gaaccgtggg atcgccagcg      540
   tcctgcagga gctcaacgtg acggtggtga cgtccctctg ccgtcgcagc aacgtctgca      600
   ctctcgtgag gggccggcag gccggcgtct gtttcgggga ctccggcagc cccttggtct      660
   gcaacgggct aatccacgga attgcctcct tcgtccgggg aggctgcgcc tcagggctct      720
   accccgatgc ctttgccccg gtggcacagt ttgtaaactg gatcgactct atcatccaac      780
   gctccgagga caacccctgt ccccaccccc gggacccgga cccggccagc aggacccact      840
   gagaagggct gcccgggtca cctcagctgc ccacacccac actctccagc atctggcaca      900
   ataaacattc tctgttttgt agaaaaaaaa aaaaaaaa                                938
<210> 3
   <211> 5292
   <212> DNA
   <213> Homo sapiens
<400> 3
   ttgtcagagc cccagctggt gtccagggac tgaccgtgag cctgggtgaa agtgagttcc      60
   ccgttggagg caccagacga ggagaggatg gaaggcctgg cccccaagaa tgagccctga     120
   ggttcaggag cggctggagt gagccgcccc cagatctccg tccagctgcg ggtcccagag     180
   gcctgggtta cactcggagc tcctggggga ggcccttgac gtgctcagtt cccaaacagg     240
   aaccctggga aggaccagag aagtgcctat tgcgcagtga gtgcccgaca cagctgcatg     300
   tggccggtat cacagggccc tgggtaaact gaggcaggcg acacagctgc atgtggccgg     360
   tatcacaggg ccctgggtaa actgaggcag gcgacacagc tgcatgtggc cggtatcaca     420
   gggccctggg taaactgagg caggcgacac agctgcatgt ggccggtatc acagggccct     480
   gggtaaactg aggcaggcga cacagctgca tgtggccggt atcacggggc cctggataaa     540
   cagaggcagg cgaggccacc cccatcaagt ccctcaggtc taggtttggc caggtttgga     600
   aaaacacagc aacgctcggt aaatctgaat ttcgggtaag tatatcctgg gcctcatttg     660
   gaagagactt agattaaaaa aaaaacgtcg agaccagccc ggccaacacg tgaaaccccg     720
   tctctactaa aaatacaaaa aattagccag gcgcagtgct cacgcctgtg atcccagcac     780
   tctgggaggt gaggcaggcg gatcacccga ggtcagctgt tcaagaccag cctggccgag 840
   tgggcgaaac actgtctcta ctacaaatac aaaaattagc cgggagtgga ggcaggtgcc 900
   tgtaatctca gctattcagg aggctgaggc aggagaatca cttgaacctg ggaggcggag 960
   gttgccgtga gccgggatca cgccaccgca ctccagcctg ggcgatagag caagactctg 1020
   tctccaaaaa aataaattaa aaaacccaca ttgattatct gacatttgaa tgcgattgtg 1080
   catcctgaat tttgtctgga ggccccaccc gagccaatcc agcgtcttgt cccccttctc 1140
   ccccttttca tcaacgcctg tgccagggga gaggaagtgg agggcgctgg ccggccgtgg 1200
   ggcaatgcaa cggcctccca gcacagggct ataagaggag ccgggcgggc acggaggggc 1260
   agagaccccg gagccccagc cccaccatga ccctcggccg ccgactcgcg tgtcttttcc 1320
   tcgcctgtgt cctgccggcc ttgctgctgg ggggtgagtt tttgagtcca acctcccgct 1380
   gctccctctg tcccgggttc tgttcccacc tctccataga gggccccacc agtgtgggtc 1440
   cctcatcctc acaggggagg tgccagctgg gacaaggaga ccagaagaga ctgaggttct 1500
   gagcggtgaa gccaccacca ggagcccaga gttggggttt gaaaaccggg gagggggggg 1560
   gtggcaggtc gccctctggg ttcaagtcca ggtctgtctg tgccttggag gggcaccgtg 1620
   gggaggtccc tttgcctctc cgtgcctcag tttcctcatc tgaacaacag gggtgcgaac 1680
   ggccccgatc ccgtgggttc ccggtggggg atccagaggc cccgtggccg ggaggggaca 1740
   ggctccttgg caggcactca gcacccgcac ccggtgtgtc cccaggcacc gcgctggcct 1800
   cggagattgt ggggggccgg cgagcgcggc cccacgcgtg gcccttcatg gtgtccctgc 1860
   agctgcgcgg aggccacttc tgcggcgcca ccctgattgc gcccaacttc gtcatgtcgg 1920
   ccgcgcactg cgtggcgaat gtgtgagtag ccgggagtgt gcgcgcccgg ctcggacccc 1980
   gcgtcccggt ctgtgaggtg ggtgggggga ggccggggcc ggggctgctg gcgggggggg 2040
   gtccgtccag ggcccgcggg gcccctcgag caccttcgcc ctcaggcccg tcgccggatg 2100
   gggacgacaa ggcgcggctg agccccgacc cccggggccg cccctgagcc ccgcctctcc 2160
   ctcttttggc agaaacgtcc gcgcggtgcg ggtggtcctg ggagcccata acctctcgcg 2220
   gcgggagccc acccggcagg tgttcgccgt gcagcgcatc ttcgaaaacg gctacgaccc 2280
   cgtaaacttg ctcaacgaca tcgtgattct ccaggtgccg ccgggcgggc gggggcgagg 2340
   ggcggaggcc agaggcctgg ggagggtgga ggcctgggga gggtggaggc tgcgacggag 2400
   gggcgcgtcg gggccgctcg tggggacctg gggtggcatc gtgggctggg tggtcccctc 2460
   tccgcgcctc ggtctgcacc tctgtgaaac gggaaaatac ccgccatggg ccgttgaggg 2520
   gttaaatgag atcctgcagg gaggccccga tctgctgtca atcaacaaac ttactgagaa 2580
   gggaggcccc gatctgttgt caatcaacaa acttactgag aagggaggcc ccgatctgtt 2640
   gtcaatcaac aaacttactg agaagggagg ccccgatctg ctgtcaatca acaaacttac 2700
   tgagaaggga ggccccgatg ttgtcaatca acaaacttac tgagaaggga ggccccgatc 2760
   tgctgtcaat caaccaaact tactgagaag ggaggccccg atctgctgtc aatcatcaaa 2820
   cttactgaga agggaggccc cgatctgctg tcaatcaaca aacttactga gaagggaggc 2880
   ccccgatctg ttgtcaatca acaaacttac tgagaaggga ggccccgatc tgctgtcaat 2940
   caacaaactt actgagattc tgtgtgtctc tccattcacc agtcctgtgg cccagggcag 3000
   gggccgcctc tgtctttggg aaaaggggca aaagtcccca cctttccacc cctgtccgcg 3060
   gcttgcagtt ctggttattt cctgggcgcc gggccccgtg gctcaggcct gtcatcccag 3120
   cactttggga ggctgaggcg ggtggatcac gaggtcaggt gttcgagacc agcctgagca 3180
   acatagtgaa accccgtctc tactaaaata cacaaaaaaa aaattagccg agtgtggttg 3240
   tgggtgcctg taatgccaac tactcaggag gctgaggaag gagaatcgct tgaaccccgg 3300
   aggcggagat tgcagtgagc tgagatcaca ccactgcact ccagcctggg tctcaaaaaa 3360
   aaaaaaaaag attcctccct gggaagggtt agagggagag tttccttgtc actaagtttt 3420
   ctcatagctc tcacccagtg cagtggcgcg atcgcagctc actacacctc catctcctgg 3480
   gctcaagcca ccctctcagc ttggaatggg gggtagctgg aaccacaggt gccaccacgt 3540
   ggtccaccac gtctggctaa tatatatata tacacacaca catacatata ttataaataa 3600
   taaatatata ttttatttaa ataaaatata taatatttat aattatttta taattataat 3660
   aatatttata taattataaa tatcatttat aattataata tttattattt tataaaataa 3720
   taaatataaa atatataaaa atatttttat aaataataaa atatatatat acacacatat 3780
   atatatattt tttgagacaa gtctcgctct gtcgcccagg ctggagcgca gtgcacaatc 3840
   tcactcactg cacctccgcc tcccaggttc aagcgattct cctgcctcag cctcccaggt 3900
   agctgggact acaggcgccc gccaccacgc ctggctaatt tttggtattg ttagtagaga 3960
   cggggtttaa ccatgttagc caggatggtc ttgatctcct gacctttgta ttggcccacc 4020
   tcagcctccc aaaatgctgg gattataggc gtgagcaccg cacctggcaa ttttttttta 4080
   ttatttttgt agacatgggg ctttgccaca ttgcccaggc tggtcttgaa tgcctggcct 4140
   ggcctaagtg atcctcctgc ctcgccctcc caaagtgctg ggcttacaag catgagccac 4200
   cgcgcccggc tgtagttttt ttgttaactg agcacctact gcttcctgca ctcaagccac 4260
   atccagggac aacctccaac gccctgagcc ttggtgacgg ctcccactct acagatgggg 4320
   aaaccgaggc ttgccttggg gagcagagtg tggggtgggt atcctgccct gcaggatccc 4380
   agaaccacag tggaacctga gatggggaaa ctgaggcccg gagaggggag ggtcatcatc 4440
   actgccccgt gtgacgcgct gacgatctgt ccccaccgcc acagctcaac gggtcggcca 4500
   ccatcaacgc caacgtgcag gtggcccagc tgccggctca gggacgccgc ctgggcaacg 4560
   gggtgcagtg cctggccatg ggctggggcc ttctgggcag gaaccgtggg atcgccagcg 4620
   tcctgcagga gctcaacgtg acggtggtga cgtccctctg ccgtcgcagc aacgtctgca 4680
   ctctcgtgag gggccggcag gccggcgtct gtttcgtacg tgccctgggt gtccctctgc 4740
   tccccacccg ctcccagccc ggtactgcag caacaggcac cgtggctaga ccctaggatg 4800
   ggacttccca accctgacac gtcggcgggc aggtgggcag ggcctcgcag tccagcttcc 4860
   ccaccttgtc tgcctccaca gggggactcc ggcagcccct tggtctgcaa cgggctaatc 4920
   cacggaattg cctccttcgt ccggggaggc tgcgcctcag ggctctaccc cgatgccttt 4980
   gccccggtgg cacagtttgt aaactggatc gactctatca tccaacgctc cgaggacaac 5040
   ccctgtcccc acccccggga cccggacccg gccagcagga cccactgaga agggctgccc 5100
   gggtcacctc agctgcccac acccacactc tccagcatct ggcacaataa acattctctg 5160
   ttttgtagaa tgtgtttgat gctccttggc tgtgtgattg ggtgttgaaa atggtcagta 5220
   ggtcgggcgt ggtggctcac acctgtaatc ccagcacttt gggaggttga ggcaggcgga 5280
   tcacttgagc tc 5292
<210> 4
   <211> 2356
   <212> DNA
   <213> Homo sapiens
<400> 4
   attctccaat cacatgatcc ctagaaatgg ggtgtggggc gagaggaagc agggaggaga 60
   gtgatttgag tagaaaagaa acacagcatt ccaggctggc cccacctcta tattgataag 120
   tagccaatgg gagcgggtag ccctgatccc tggccaatgg aaactgaggt aggcgggtca 180
   tcgcgctggg gtctgtagtc tgagcgctac ccggttgctg ctgcccaagg accgcggagt 240
   cggacgcagg cagaccatgt ggaccctggt gagctgggtg gccttaacag cagggctggt 300
   ggctggaacg cggtgcccag atggtcagtt ctgccctgtg gcctgctgcc tggaccccgg 360
   aggagccagc tacagctgct gccgtcccct tctggacaaa tggcccacaa cactgagcag 420
   gcatctgggt ggcccctgcc aggttgatgc ccactgctct gccggccact cctgcatctt 480
   taccgtctca gggacttcca gttgctgccc cttcccagag gccgtggcat gcggggatgg 540
   ccatcactgc tgcccacggg gcttccactg cagtgcagac gggcgatcct gcttccaaag 600
   atcaggtaac aactccgtgg gtgccatcca gtgccctgat agtcagttcg aatgcccgga 660
   cttctccacg tgctgtgtta tggtcgatgg ctcctggggg tgctgcccca tgccccaggc 720
   ttcctgctgt gaagacaggg tgcactgctg tccgcacggt gccttctgcg acctggttca 780
   cacccgctgc atcacaccca cgggcaccca ccccctggca aagaagctcc ctgcccagag 840
   gactaacagg gcagtggcct tgtccagctc ggtcatgtgt ccggacgcac ggtcccggtg 900
   ccctgatggt tctacctgct gtgagctgcc cagtgggaag tatggctgct gcccaatgcc 960
   caacgccacc tgctgctccg atcacctgca ctgctgcccc caagacactg tgtgtgacct 1020
   gatccagagt aagtgcctct ccaaggagaa cgctaccacg gacctcctca ctaagctgcc 1080
   tgcgcacaca gtgggggatg tgaaatgtga catggaggtg agctgcccag atggctatac 1140
   ctgctgccgt ctacagtcgg gggcctgggg ctgctgccct tttacccagg ctgtgtgctg 1200
   tgaggaccac atacactgct gtcccgcggg gtttacgtgt gacacgcaga agggtacctg 1260
   tgaacagggg ccccaccagg tgccctggat ggagaaggcc ccagctcacc tcagcctgcc 1320
   agacccacaa gccttgaaga gagatgtccc ctgtgataat gtcagcagct gtccctcctc 1380
   cgatacctgc tgccaactca cgtctgggga gtggggctgc tgtccaatcc cagaggctgt 1440
   ctgctgctcg gaccaccagc actgctgccc ccagggctac acgtgtgtag ctgaggggca 1500
   gtgtcagcga ggaagcgaga tcgtggctgg actggagaag atgcctgccc gccgggcttc 1560
   cttatcccac cccagagaca tcggctgtga ccagcacacc agctgcccgg tggggcagac 1620
   ctgctgcccg agcctgggtg ggagctgggc ctgctgccag ttgccccatg ctgtgtgctg 1680
   cgaggatcgc cagcactgct gcccggctgg ctacacctgc aacgtgaagg ctcgatcctg 1740
   cgagaaggaa gtggtctctg cccagcctgc caccttcctg gcccgtagcc ctcacgtggg 1800
   tgtgaaggac gtggagtgtg gggaaggaca cttctgccat gataaccaga cctgctgccg 1860
   agacaaccga cagggctggg cctgctgtcc ctaccgccag ggcgtctgtt gtgctgatcg 1920
   gcgccactgc tgtcctgctg gcttccgctg cgcagccagg ggtaccaagt gtttgcgcag 1980
   ggaggccccg cgctgggacg cccctttgag ggacccagcc ttgagacagc tgctgtgagg 2040
   gacagtactg aagactctgc agccctcggg accccactcg gagggtgccc tctgctcagg 2100
   cctccctagc acctccccct aaccaaattc tccctggacc ccattctgag ctccccatca 2160
   ccatgggagg tggggcctca atctaaggcc ttccctgtca gaagggggtt gtggcaaaag 2220
   ccacattaca agctgccatc ccctccccgt ttcagtggac cctgtggcca ggtgcttttc 2280
   cctatccaca ggggtgtttg tgtgtgtgcg cgtgtgcgtt tcaataaagt ttgtacactt 2340
   tcttaaaaaa aaaaaa 2356
<210> 5
   <211> 19
   <212> RNA
   <213> Artificial Sequence
<220>
   <223> BLT1 sense siRNA strand
<400> 5 caaccuacac uuccuauua 19
<210> 6
   <211> 19
   <212> RNA
   <213> Artificial Sequence
<220>
   <223> BLT1 antisense siRNA strand
<400> 6
   uaauaggaag uguagguug 19
<210> 7
   <211> 19
   <212> RNA
   <213> Artificial Sequence
<220>
   <223> BLT2 sense siRNA strand
<400> 7
   gggacuuaac auacucuua 19
<210> 8
   <211> 19
   <212> RNA
   <213> Artificial Sequence
<220>
   <223> BLT2 antisense siRNA strand
<400> 8
   uaagaguaug uuaaguccg 19
<210> 9
   <211> 21
   <212> DNA
   <213> Unknown
<220>
   <223> COL1A1 siRNA
<400> 9
   cagaagaacu gguacaucat t 21
<210> 10
   <211> 21
   <212> DNA
   <213> Unknown
<220>
   <223> COL1A1 siRNA
<400> 10
   ugauguacca guucuucugt t 21
<210> 11
   <211> 21
   <212> DNA
   <213> Unknown
<220>
   <223> P4HA1 siRNA
<400> 11
   cucuguuacg ucuccaggat t 21
<210> 12
   <211> 21
   <212> DNA
   <213> Unknown
<220>
   <223> P4HA1 siRNA
<400> 12
   uccuggagac guaacagagt t 21
<210> 13
   <211> 21
   <212> RNA
   <213> Unknown
<220>
   <223> TNF siRNA
<400> 13
   gcguggagcu gagagauaau u 21
<210> 14
   <211> 21
   <212> RNA
   <213> Unknown
<220>
   <223> TNF siRNA
<400> 14
   uuaucucuca gcuccacgcu u 21
<210> 15
   <211> 21
   <212> DNA
   <213> Unknown
<220>
   <223> ANXA11 siRNA
<400> 15
   gauucaccgu ccuagagcut t 21
<210> 16
   <211> 21
   <212> DNA
   <213> Unknown
<220>
   <223> ANXA11 siRNA
<400> 16
   agcucuagga cggugaauct t 21
<210> 17
   <211> 19
   <212> DNA
   <213> Unknown
<220>
   <223> COL1A1 PCR primer
<400> 17
   aggctggtgt gatgggatt 19
<210> 18
   <211> 20
   <212> DNA
   <213> Unknown
<220>
   <223> COL1A1 PCR primer
<400> 18
   agggccttgt tcacctctct 20
<210> 19
   <211> 24
   <212> DNA
   <213> Unknown
<220>
   <223> P4HA1 PCR primer
<400> 19
   gaaagatctg gtgacttctc tgaa 24
<210> 20
   <211> 21
   <212> DNA
   <213> Unknown
<220>
   <223> P4HA1 PCR primer
<400> 20
   ccagattctc caactcactc c 21
<210> 21
   <211> 21
   <212> DNA
   <213> Unknown
<220>
   <223> TNF PCR primer
<400> 21
   ccaggcagtc agatcatctt c 21
<210> 22
   <211> 20
   <212> DNA
   <213> Unknown
<220>
   <223> TNF PCR primer
<400> 22
   atgaggtaca ggccctctga 20
<210> 23
   <211> 20
   <212> DNA
   <213> Unknown
<220>
   <223> ANXA11 PCR primer
<400> 23
   cggcagcaga tcctactttc 20
<210> 24
   <211> 20
   <212> DNA
   <213> Unknown
<220>
   <223> ANXA11 PCR primer
<400> 24
   atcaggcagg cttcatcagt 20

## Description

### CROSS REFERENCE TO RELATED APPLICATIONS

The present Application claims the benefit of priority to U.S. Provisional Application No. 62/190,583, filed on July 9, 2015.

### STATEMENT REGARDING SEQUENCE LISTING

The Sequence Listing associated with this application is provided in text format in lieu of a paper copy, and is hereby incorporated by reference into the specification. The name of the text file containing the Sequence Listing is INMD_125_01WO_SeqList_ST25.txt. The text file is 19 kb, was created on July 7, 2016, and is being submitted electronically via EFS-Web.

### BACKGROUND OF THE INVENTION

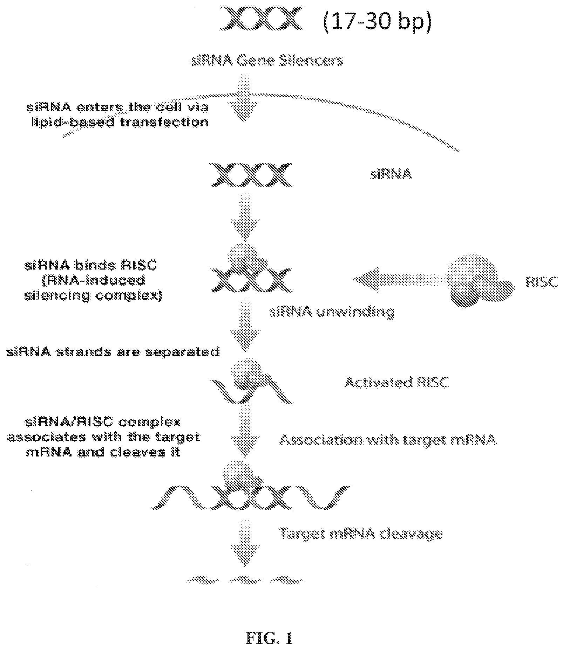

RNA interference (RNAi) via small interfering RNAs (siRNAs) targets messenger RNA (mRNA) in a target specific manner which allows for silencing of the particular gene is a targeted manner (see Figure 1 for overview). Although the precise mechanism remains unclear, RNAi is thought to begin with the cleavage of longer double-stranded RNAs into siRNAs by an RNaseIII-like enzyme, dicer. siRNAs are double-stranded RNAs (ds-RNAs) that are usually about 17 to about 30 nucleotide base pairs (bps), *e.g.,* from about 20 to about 27 bps, or about 21 to about 24 bps in length and in some instances, contain 2-nucleotide 3' overhangs, and 5' phosphate and 3' hydroxyl termini. One strand of the siRNA is incorporated into a ribonucleoprotein complex known as the RNA-induced silencing complex (RISC). RISC uses this siRNA strand to identify mRNA molecules that are at least partially complementary to the incorporated siRNA strand, and then cleaves these target mRNAs or inhibits their translation. The siRNA strand that is incorporated into RISC is known as the guide strand or the antisense strand. The other siRNA strand, known as the sense strand, is eliminated from the siRNA and is at least partially homologous to the target mRNA. In the context of the present application, the term "RNAi" or "siRNA" also includes short hairpin RNAs (shRNAs) and microRNAs (miRNAs).

Because siRNA can be designed against any mRNA target, therapeutic applications for these compounds are far ranging. Despite the potential of siRNA compounds to be successful clinically, hurdles exist to their effectiveness.

siRNA are susceptible to nuclease digestion *in* vivo. Additionally, naked siRNA constructs are limited in their ability to diffuse or be transported across cellular membranes. Delivery systems are therefore needed so that siRNA can be taken up. Although viral vectors are capable of expressing large quantities of siRNAs in an efficient manner, they are plagued with toxicity and immunogenicity issues. Moreover, injection of these vectors does not allow for siRNA specific targeting at the cellular level, for example, to combat certain diseases associated with specific cell types and tissues.

EP2199298 discloses a nanoparticle composition comprising a RNAi nucleic acid, 2-60 mol% of a cationic lipid, 5-90 mol% of a neutral lipid and 0.5-20 mol% of a PEGylated lipid. The neutral lipid may be a mixture of phospholipid and cholesterol and the composition is for inhalation to the lung.

As such, compositions and methods for delivering siRNA constructs to specific cell types and tissues are needed. The present invention addresses this and other needs.

### SUMMARY OF THE INVENTION

The present invention provides compositions comprising a RNAi compound complexed with or encapsulated by lipid particles, wherein the compositions show efficient uptake and reduction in the expression and/or activity of target mRNAs in various pulmonary cells. The invention is defined by the claims.

The invention provides a composition comprising a nucleic acid compound complexed or encapsulated by a lipid particle; wherein the lipid particle comprises: (a) a cationic lipid comprising 40 mol% to 58 mol% of the total lipid present in the composition; (b) a neutral lipid comprising 40 mol% to 50 mol% of the total lipid present in the composition; (c) a conjugated lipid comprising 0.3 mol% to 1.5 mol% of the total lipid present in the composition; and further features which are defined in claim 1.

The composition of the invention could be formulated as a dry powder, a suspension, or a nebulized spray. In some embodiments, the compositions may further comprise a propellant such as a hydrocarbon propellant.

The present invention provides for the claimed composition for use in treating a pulmonary fibrosis or sarcoidosis in a patient in need thereof, the method comprising administering to the lungs of the patient a therapeutically effective amount of the compositions described herein.

According to one aspect, administration of the present compositions downregulates the expression and/or activity of a mRNA that is over-expressed in or is genetically linked to the pulmonary disease or disorder.

In the present invention the RNAi compound targets a mRNA encoding COL1A1, or annexin A11.

In one embodiment, the effective amount of the composition is administered to the lungs of the patient via inhalation.

### BRIEF DESCRIPTION OF THE FIGURES

**Figure 1** depicts a method of action of RNAi and siRNA. Adapted from *https:*//*www.scbt.com*/*gene_silencers.html.*
**Figure 2** shows the uptake of lipid nanoparticles of the invention in macrophages and fibroblasts.
**Figure 3** shows the effect of various siRNA lipid nanoparticle formulations on the expressi on of *COL1A1.*
**Figure 4** shows the target specific reduction in the expression of *COL1A1* using siRNA lipid nanoparticle formulations.
**Figure 5** shows the target specific reduction in the expression of *P4HA1* using siRNA lipid nanoparticle formulations.
**Figure 6** shows the target specific reduction in the expression of *ANXA11* using siRNA lipid nanoparticle formulations.
**Figure 7** shows the uptake of lipid nanoparticles in macrophages and fibroblasts under fluorescence microscope.
**Figure 8** shows a schematic of inducing pulmonary fibrosis in *in vivo* mouse model of sarcoidosis.

### DETAILED DESCRIPTION OF THE INVENTION

The present invention is based in part on the discovery that treatment of localized pulmonary disorders can occur via the targeting of pulmonary phagocytic cells involved in inflammation or infection, via inhalation delivery of RNAi-lipid nanoparticle compositions to patients in need thereof.

Phagocytosis is a specific form of endocytosis involving the vascular internalization of solids such as bacteria and cellular debris. The present invention harnesses the immune system's ability to phagocytize particles as a drug delivery vehicle, by providing lipid based compositions comprising liposomes or lipid nanoparticles that are designed to be taken up by one or more phagocytes associated with host tissue damage or infection (e.g., macrophage, fibroblast). Upon delivery via inhalation and uptake, the compositions deliver the nucleic acid compounds to the cells and regulate the expression or activity of one or more target messenger RNAs (mRNAs).

Phagocytes of humans and other animals are called "professional" or "non-professional" depending on how effective they are at phagocytosis. The professional phagocytes include many types of white blood cells such as neutrophils, monocytes, macrophages, mast cells, eosinophils, basophils and dendritic cells. Although phagocytosis is a crucial element of host defense against foreign substances, as provided above, the response can also be associated with host tissue damage.

For example, neutrophils are a type of phagocytic cells that are involved in the induction of inflammation by undergoing receptor-mediated respiratory burst and degranulation. Degranulation has been implicated as a factor in pulmonary disorders, rheumatoid arthritis, and septic shock. Neutrophil degranulation depends on the activation of intracellular signaling pathways, which may be selective and dependent on nonredundant signaling pathways >Lacy 2006, Allergy Asthma, Clin. Immuno. 2(3):98-108).

In cystic fibrosis (CF) patients, neutrophils represent approximately seventy percent of the inflammatory cell population in the epithelial lining fluid (ELF), as compared to approximately one percent of the inflammatory cell population in the normal lung (Kelly et al., 1998, Expert Opin. Ther. Targets 12, pp. 145-157). Neutrophils have been shown to be ineffective in the clearance of bacteria and play a major role in the destruction of the structural matrix of the lung, for example, by the secretion of proteases that cleave and destroy lung proteins. Accordingly, the inhibition of neutrophil function at disease sites could provide an effective therapy in CF patients.

Pulmonary phagocytes, e.g., pulmonary monocytes and fibroblasts play an important role in wound healing as well as clearance of invading microorganisms. However, uncontrolled or dysregulated response of these cells can also lead to eventual development of pulmonary disorders such as pulmonary fibrosis and/or sarcoidosis. Pulmonary fibrosis is a lung disease that is refractory to treatment and carries a high mortality rate. It includes a heterogeneous group of lung disorders characterized by the progressive and irreversible destruction of lung architecture caused by scar formation that ultimately leads to organ malfunction, disruption of gas exchange, and death from respiratory failure. Idiopathic pulmonary fibrosis (IPF), a particularly severe form of pulmonary fibrosis with unknown etiology has a life expectancy of 2-6 yr after diagnosis (Wynn, JEM, 208 (7): 1339-1350, 2011; incorporated by reference herein in its entirety). Lung fibrosis can also develop after viral infections and after exposure to radiotherapy, chemotherapeutic drugs, and aerosolized environmental toxins. It also occurs in some bone marrow transplant recipients suffering from chronic graft versus host disease and in a subset of individuals with chronic inflammatory diseases like scleroderma and rheumatoid arthritis. Currently, the only effective treatment available for progressive lung fibrosis is lung transplantation. Repair of damaged tissues is a fundamental biological mechanism that allows the ordered replacement of dead or damaged cells after injury, a process critically important for survival. However, if this process becomes dysregulated, it can lead to the development of a permanent fibrotic "scar," which is characterized by the excess accumulation of extracellular matrix (ECM) components (e.g., hyaluronic acid, fibronectin, proteoglycans, and interstitial collagens) at the site of tissue injury. Consequently, fibrosis or fibrogenesis is often defined as an out of control wound healing response.

The present invention provides in one embodiment, an siRNA composition that inhibits the uncontrolled or dysregulated response of a macrophage or fibroblast in a pulmonary fibrosis patient, e.g, an IPF patient. For example, in one embodiment, the siRNA composition comprises an siRNA targeting various types of collagens and/or collagen synthesis enzymes, as discussed in further detail below. In another embodiment, the siRNA composition comprises a cytokine or cytokine receptor siRNA, as discussed in further detail below.

Wound repair has four distinct stages that include a clotting/coagulation phase, an inflammatory phase, a fibroblast migration/proliferation phase, and a final remodeling phase where normal tissue architecture is restored. In the earliest stages after tissue damage, epithelial cells and/or endothelial cells release inflammatory mediators that initiate an antifibrinolytic-coagulation cascade that triggers clotting and development of a provisional ECM. Platelet aggregation and subsequent degranulation in turn promotes blood vessel dilation and increased permeability, allowing efficient recruitment of inflammatory cells (e.g., neutrophils, macrophages, lymphocytes, and eosinophils) to the site of injury. Neutrophils are the most abundant inflammatory cell at the earliest stages of wound healing, but are quickly replaced by macrophages after neutrophil degranulation. During this initial leukocyte migration phase, activated macrophages and neutrophils debride the wound and eliminate any invading organisms. They also produce a variety of cytokines and chemokines that amplify the inflammatory response and trigger fibroblast proliferation and recruitment. Myofibroblasts are recruited from a variety of sources including local mesenchymal cells, bone marrow progenitors (called fibrocytes), and via a process called epithelial-mesenchymal transition (EMT), wherein epithelial cells transdifferentiate into fibroblast-like cells. Once fibroblasts become activated, they transform into α-smooth muscle actin-expressing myofibroblasts that secrete ECM components. Finally, in the wound maturation/remodeling phase, myofibroblasts promote wound contraction, a process where the edges of the wound migrate toward the center and epithelial/endothelial cells divide and migrate over the temporary matrix to regenerate the damaged tissue. Fibrosis develops when the wound is severe, the tissue-damaging irritant persists, or when the repair process becomes dysregulated. Thus, many stages in the wound repair process can go awry and contribute to scar formation, likely explaining the complex nature of pulmonary fibrosis. Some of the mechanisms that play a role in the development of pulmonary fibrosis are discussed in Wynn, JEM, 208(7): 1339-1350, 2011; and Todd et al., Fibrogenesis & Tissue Repair, 2012, 5(11); both of which are incorporated by reference herein in its entirety.

Sarcoidosis is a multisystem immune disorder, resulting in the formation of epitheloid granulomas throughout the body, particularly within the lungs, eyes and skin. The immune systems of affected individuals exhibit significant changes in cell numbers and cell signaling, with an increase in CD3 and CD4 positive T cells in the lungs. Activated T cells within sarcoid lungs have also been shown to over-express several cytokine receptors, including the interleukin-2 receptor (IL-2R), and produce increased amounts of cytokines, including interleukin-2 and interferon-γ (IFNγ). In addition, monocytes and macrophages are heavily involved in the formation of sarcoid granulomas and also secrete a range of cytokines that further enhance the immune response. For example alveolar macrophages secrete tumor necrosis factor α (TNFα), and interleukin-15, which has been shown to induce T cell proliferation. As such, the present invention provides treatment for sarcoidosis via inhalation of an siRNA composition that can be taken up by monocytes and macrophages present in sarcoid lungs.

The siRNA compositions provided herein are lipid nanoparticle compositions that shield the siRNA from nuclease digestion, and allow for efficient uptake by pulmonary phagocytes. The lipid nanoparticle comprises a cationic lipid, neutral lipid and a conjugated lipid such as a PEGylated lipid.

The invention provides a composition comprising a nucleic acid compound complexed or encapsulated by a lipid particle; wherein the lipid particle comprises: (a) a cationic lipid comprising 40 mol% to 58 mol% of the total lipid present in the composition; (b) a neutral lipid comprising 40 mol% to 50 mol% of the total lipid present in the composition; (c) a conjugated lipid comprising 0.3 mol% to 1.5 mol% of the total lipid present in the composition; and further features which are defined in claim1.

The compositions provided by the present invention comprise an RNAi compound complexed to or encapsulated by a lipid particle, wherein the RNAi compound targets an mRNA whose corresponding protein product plays an important role in the pathogenesis of a pulmonary disease/disorder which is pulmonary fibrosis or sarcoidosis. In one embodiment, the compositions provided by the present invention comprise an RNAi compound complexed to or encapsulated by a lipid particle, wherein the RNAi compound targets an mRNA that is over-expressed in a pulmonary disease/disorder. In another embodiment, the compositions provided by the present invention comprise an RNAi compound complexed to or encapsulated by a lipid particle, wherein the RNAi compound targets an mRNA whose corresponding gene has a nucleotide polymorphism that is genetically linked to a pulmonary disease/disorder, e.g. Annexin A11.

In one embodiment, the compositions provided by the present invention comprise an RNAi compound complexed to or encapsulated by a lipid particle, wherein the RNAi compound targets an mRNA whose corresponding protein function is associated with a phagocytic cell response, for example an inflammatory response, degranulation of a granule in a granulocyte (*e.g*., neutrophil degranulation), or recruitment of an immune cell or a granulocyte to a site of a lung infection (chemotaxis). In another embodiment, the compositions provided by the present invention comprise an RNAi compound complexed to or encapsulated by a lipid particle, wherein the RNAi compound targets an mRNA whose corresponding protein function is associated with a fibroblast response, for example, synthesis of ECM components such as collagen.

The compositions provided by the present invention comprise an RNAi compound that target a mRNA involved in collagen synthesis (namely, the COL1A1), and/or an mRNA whose corresponding gene has a nucleotide polymorphism that is genetically linked to a pulmonary disease/disorder (namely Annexin A11).

An "effective amount" or "therapeutically effective amount" of the composition is an amount of the nucleic acid compound such as an interfering RNA, or a composition comprising the same, that is sufficient to produce the desired effect, e.g., an inhibition of expression of a target sequence in comparison to the normal expression level detected in the absence of an interfering RNA. Inhibition of expression of a target gene or target sequence is achieved when the value obtained with an interfering RNA relative to the control is about 90%, 85%, 80%, 75%, 70%, 65%, 60%, 55%, 50%, 45%, 40%, 35%, 30%, 25%, 20%, 15%, 10%, 5%, or 0%. Suitable assays for measuring expression of a target gene or target sequence include, e.g., examination of protein or RNA levels using techniques known to those of skill in the art such as dot blots, northern blots, in situ hybridization, ELISA, immunoprecipitation, enzyme function, as well as phenotypic assays known to those of skill in the art.

As used herein, "complexed or encapsulated by a lipid particle" refers to a lipid particle that provides a nucleic acid compound (e.g., an interfering RNA), with full encapsulation, partial encapsulation, or both, or a lipid particle that is complexed or agglomerated with the nucleic acid compound.

The term "conjugated lipid" refers to a lipid that is coupled to a non-lipid moiety. Such conjugated lipids include, but are not limited to, polyamide oligomers (e.g., ATTA-lipid conjugates), PEG-lipid conjugates, such as PEG coupled to dialkyloxypropyls, PEG coupled to diacylglycerols, PEG coupled to cholesterol, PEG coupled to phosphatidylethanolamines, PEG conjugated to ceramides (see, e.g., U.S. Pat. No. 5,885,613), cationic PEG lipids, and mixtures thereof. PEG can be conjugated directly to the lipid or may be linked to the lipid via a linker moiety. Any linker moiety suitable for coupling the PEG to a lipid can be used including, e.g., non-ester containing linker moieties and ester-containing linker moieties. According to the invention, the conjugated lipid comprises DMG-PEG2000.

The term "neutral lipid" refers to a lipid species that exist either in an uncharged or neutral zwitterionic form at a selected pH. At physiological pH, such lipids include, for example, phospholipids such as diacylphosphatidylcholine and diacylphosphatidylethanolamine, and other lipids such as ceramide, sphingomyelin, cephalin, cholesterol, tocopherols, cerebrosides, and diacylglycerols. According to the invention, the neutral lipid comprises a mixture of i) a phospholipid and ii) cholesterol hemisuccinate (CHEMS) or tocopheryl hemisuccinate (THS).

The term "non-cationic lipid" refers to any amphipathic lipid as well as any other neutral lipid or anionic lipid. The term "non-cationic lipid" includes phospholipids, cholesterol, tocopherols, and derivatives thereof.

The term "cationic lipid" refers to any of a number of lipid species that carry a net positive charge at a selected pH, such as physiological pH (e.g., pH of about 7.0). It has been surprisingly found that cationic lipids comprising alkyl chains with multiple sites of unsaturation, e.g., at least two or three sites of unsaturation, are particularly useful for forming lipid particles with increased membrane fluidity. A number of cationic lipids and related analogs, which are also useful in the present invention, have been described in U.S. Patent Publication Nos. 20060083780 and 20060240554; U.S. Pat. Nos. 5,208,036; 5,264,618; 5,279,833; 5,283,185; 5,753,613; and 5,785,992; and PCT Publication No. WO 96/10390. According to the invention, the cationic lipid comprises 1,2-dioleoyl-3-dimethylammonium-propane (DODAP).

### Lipid particles

The present invention provides compositions comprising a nucleic acid compound complexed or encapsulated by a lipid particle and said compositions for use in treating or ameliorating one or more pulmonary fibrosis or sarcoidosis.

The lipid particle of the composition comprises a cationic lipid, a neutral lipid, and a conjugated lipid. The lipid particle of the composition comprises (a) a cationic lipid comprising 40 mol% to 58 mol% of the total lipid present in the composition; (b) a neutral lipid comprising 40 mol% to 50 mol% of the total lipid present in the composition; (c) a conjugated lipid comprising 0.3 mol% to 1.5 mol% of the total lipid present in the composition; which are further defined in claim 1.

The neutral lipid comprises or consists of a mixture of i) a phospholipid and ii) cholesterol hemisuccinate or tocopherol hemi succinate.

In various embodiments, the cationic lipid comprises one or more of the cationic lipids described in U.S. Patent Nos.: 7,341,738; 8,058,069; 9,006,417; and 9,139,554. Without wishing to be bound by theory, it is thought that the use of cationic lipid facilitates the condensation of RNAi compounds into particles, due to the electrostatic interactions between the negatively charged RNAi and the positively charged lipids.

According to the invention, the cationic lipid comprises 1,2-dioleoyl-3-dimethylammonium-propane (DODAP).

In some embodiments, the cationic lipid comprises one or more of the following cationic lipids: 1,2-dilinoleyloxy-N,N-dimethylaminopropane (DLinDMA), 1,2-dilinolenyloxy-N,N-dimethylaminopropane (DLenDMA), 2,2-dilinoleyl-4-(2-dimethylaminoethyl)-[1,3]-dioxolane (DLin-K-C2-DMA; "XTC2"), 2,2-dilinoleyl-4-(3-dimethylaminopropyl)-[1,3]-dioxolane (DLin-K-C3-DMA), 2,2-dilinoleyl-4-(4-dimethylaminobutyl)-[1,3]-dioxolane (DLin-K-C4-DMA), 2,2-dilinoleyl-5-dimethylaminomethyl-[1,3]-dioxane (DLin-K6-DMA), 2,2-dilinoleyl-4-N-methylpepiazino-[1,3]-dioxolane (DLin-K-MPZ), 2,2-dilinoleyl-4-dimethylaminomethyl-[1,3]-dioxolane (DLin-K-DMA), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3-dimethylaminopropane (DLin-S-DMA), 1-linoleoyl-2-linoleyloxy-3-dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.Cl), 1,2-dilinoleyloxy-3-(N-methylpiperazino)propane (DLin-MPZ), 3 -(N,N-dilinoleylamino)-1,2-propanediol (DLinAP), 3-(N,N-dioleylamino)-1,2-propanedio (DOAP), 1,2-dilinoleyloxo-3-(2-N,N-dimethylamino)ethoxypropane (DLin-EG-DMA), N,N-dioleyl - N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3-(N--(N',N'-dimethylaminoethane)-carbamoyl)cholesterol (DC-Chol), N-(1,2-dimyristyloxyprop-3-yl)-N,N-dimethyl-N-hydroxyethyl ammonium bromide (DMRIE), 2,3-dioleyloxy-N-[2(spermine-carboxamido)ethyl]-N,N-dimethyl-1-propanamin-iumtrifluoroacetate (DOSPA), dioctadecylamidoglycyl spermine (DOGS), 3-dimethylamino-2-(cholest-5-en-3-beta-oxybutan-4-oxy)-1-(cis,cis-9,12-oc- tadecadienoxy)propane (CLinDMA), 2-[5'-(cholest-5-en-3-beta-oxy)-3'-oxapentoxy)-3-dimethyl-1-(cis,cis-9',1- -2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), or mixtures thereof.

In other embodiments, the cationic lipid comprises one or more of the following cationic lipids: MC3, LenMC3, CP-LenMC3, γ-LenMC3, CP-γ-LenMC3, MC3MC, MC2MC, MC3 Ether, MC4 Ether, MC3 Amide, Pan-MC3, Pan-MC4, Pan MC5 described in U.S. Patent No. 9,006,417, or mixtures thereof. The synthesis of these lipids is also described in U.S. Patent No. 9,006,417.

In one embodiment, a cationic lipid includes ammonium salts of fatty acids, phospholipids and glycerides. The fatty acids include fatty acids of carbon chain lengths of 12 to 26 carbon atoms that are either saturated or unsaturated. Some specific examples include: myristylamine, palmitylamine, laurylamine and stearylamine, dilauroyl ethylphosphocholine (DLEP), dimyristoyl ethylphosphocholine (DMEP), dipalmitoyl ethylphosphocholine (DPEP) and distearoyl ethylphosphocholine (DSEP), N-(2,3-di-(9-(Z)-octadecenyloxy)-prop-1-yl-N,N,N-trimethylammoniu-m chloride (DOTMA), dioleylphosphatidylethanolamine (DOPE) and 1,2-bis(oleoyloxy)-3-(trimethylammonio) propane (DOTAP).

Many of these cationic lipids are available commercially. For example, DODAP is available commercially from Avanti Polar Lipids. Additionally, the synthesis of cationic lipids such as DLin-K-C2-DMA ("XTC2"), DLin-K-C3-DMA, DLin-K-C4-DMA, DLin-K6-DMA, and DLin-K-MPZ, as well as additional cationic lipids, is described in U.S. Provisional Application No. 61/104,212, filed Oct. 9, 2008. The synthesis of cationic lipids such as DLin-K-DMA, DLin-C-DAP, DLin-DAC, DLin-MA, DLinDAP, DLin-S-DMA, DLin-2-DMAP, DLin-TMA.Cl, DLin-TAP.Cl, DLin-MPZ, DLinAP, DOAP, and DLin-EG-DMA, as well as additional cationic lipids, is described in PCT Application No. PCT/US08/88676, filed Dec. 31, 2008. The synthesis of cationic lipids such as CLinDMA, as well as additional cationic lipids, is described in U.S. Patent Publication No. 20060240554.

In some embodiments, the cationic lipid comprises about 45 to about 50 mol% including values and subranges therebetween, of the total lipid present in the composition.

In yet some other embodiments, the cationic lipid comprises 40 to about 55 mol%, about 40 to about 50 mol%, or about 40 to 45 mol%, including values and subranges therebetween, of the total lipid present in the composition.

In yet some other embodiments, the cationic lipid comprises about 45 to about 55 mol%, or about 45 to about 50 mol%, including values and subranges therebetween, of the total lipid present in the composition.

In a particular embodiment, the cationic lipid comprises about 55 to about 58 mol%, such as about 55, 55.1, 55.2., 55.3, 55.4, 55.5, 55.6, 55.7, 55.8, 55.9, 56, 56.1, 56.2, 56.3, 56.4, 56.5, 56.6, 56.7, 56.8, 56.9, 57, 57.1, 57.2, 57.3, 57.4, 57.5, 57.6, 57.7, 57.8, 57.9, or 58 mol%, of the total lipid present in the composition. In an exemplary embodiment, the cationic lipid is DODAP and is present in an amount of about 55 to about 58 mol% of the total lipid present in the composition. In another exemplary embodiment, DODAP is present in an amount of about 57.1 mol% of the total lipid present in the composition.

In another particular embodiment, the cationic lipid comprises about 48 to about 52 mol%, such as about 48, 48.5, 49, 49.5, 50, 50.5, 51, 51.5, or 52 mol%, of the total lipid present in the composition. In an exemplary embodiment, the cationic lipid is DODAP and is present in an amount of about 48 to about 52 mol% of the total lipid present in the composition. In another exemplary embodiment, DODAP is present in an amount of about 50 mol% of the total lipid present in the composition.

In one embodiment, the cationic lipid is present in an amount of about 50 mol%, of the total lipid present in the composition. In another embodiment, the cationic lipid is present in an amount of about 57 mol%, of the total lipid present in the composition.

In some embodiments, the cationic lipid is present in an amount of about 45 mol% or about 57.1 mol% of the total lipid present in the composition.

According to the invention, a neutral lipid comprises 40 mol% to 50 mol%, including values and subranges therebetween, of the total lipid present in the composition. According to the invention, the neutral lipid present in the compositions of the invention comprises a mixture of one or more neutral lipids. Neutral lipids include, but are not limited to, phospholipids such as phosphatidylcholines and phosphatidylethanolamines, ceramide, sphingomyelin, cephalin, sterols such as cholesterol or derivatives thereof, tocopherols (e.g. methylated phenols many of which have vitamin E activity) or derivatives thereof, cerebrosides, and diacylglycerols. According to the invention, a mixture of i) a phospholipid and ii) cholesterol hemisuccinate or tocopherylhemisuccinate is present as the neutral lipid.

In a particular embodiment, tocopherol is α-tocopherol or a derivative thereof (e.g. α-tocopherol hemisuccinate). According to the invention the neutral lipid is present in an amount of 40 to 50 mol%, of the total lipid present in the composition.

In one embodiment, the neutral lipid comprises about 40 to about 42 mol%, including values therebetween, such as about 40, 40.1, 40.2, 40.3, 40.4, 40.4, 40.5, 40.6, 40.7, 40.8, 40.9, 41, 41.1, 41.2, 41.3, 41.4, 41.5, 41.6, 41.7, 41.8, 41.9, or 42 mol%, of the total lipid present in the composition. In one embodiment, the neutral lipid comprises or consists of a mixture of a phospholipid and CHEMS or THS and the mixture comprises about 40 to about 42 mol%, including values therebetween, of the total lipid present in the composition.

In some other embodiments, the neutral lipid is present in an amount of about 41 to about 43 mol%, such as about 41, 41.1, 41.2, 41.3, 41.4, 41.5, 41.6, 41.7, 41.8, 41.9, 42, 42.1, 42.2, 42.3, 42.4, 42.5, 42.6, 42.7, 42.8, 42.9, or about 43 mol%, of the total lipid present in the composition.

In another embodiment, the neutral lipid comprises or consists of a mixture of a phospholipid and cholesterol or tocopherol derivative as claimed, and the mixture comprises about 47 to about 50 mol%, including values therebetween, such as about 47, 47.1, 47.2, 47.3, 47.4, 47.5, 47.6, 47.7, 47.8, 47.9, 48, 48.1, 48.2, 48.3, 48.4, 48.5, 48.6, 48.7, 48.8, 48.9, 49, 49.1, 49.2, 49.3, 49.4, 49.5, 49.6, 49.7, 49.8, 49.9, or 50 mol%, of the total lipid present in the composition. In an exemplary embodiment, the neutral lipid comprises or consists of a mixture of a phospholipid and a cholesterol derivative such as cholesterol hemisuccinate (CHEMS), and the mixture comprises about 47 to about 50 mol%, including values therebetween, of the total lipid present in the composition. In another exemplary embodiment, the neutral lipid comprises or consists of a mixture of a phospholipid and a tocopherol derivative such as tocopherol hemisuccinate (THS), and the mixture comprises about 47 to about 50 mol%, including values therebetween, of the total lipid present in the composition.

In one embodiment, the neutral lipid is present in an amount of about 41.4 mol%, of the total lipid present in the composition. In another embodiment, the neutral lipid is present in an amount of about 42.5 mol%, of the total lipid present in the composition. In yet some other embodiments, the neutral lipid is present in an amount of about 49 mol%, of the total lipid present in the composition.

In an exemplary embodiment, the lipid particle of the composition comprises a cationic lipid, a phospholipid, CHEMS, and a conjugated lipid. In another exemplary embodiment, the lipid particle of the composition comprises a cationic lipid, a phospholipid, THS, and a conjugated lipid. In yet another exemplary embodiment, the lipid particle of the composition comprises a cationic lipid, a phospholipid, α-THS, and a conjugated lipid.

Phospholipids include, but are not limited to phosphatidylcholine (PC), phosphatidylglycerol (PG), phosphatidylinositol (PI), phosphatidylserine (PS), phosphatidylethanolamine (PE), and phosphatidic acid (PA). In one embodiment, the phospholipid is an egg phospholipid, a soya phospholipid or a hydrogenated egg and soya phospholipid. In one embodiment, the phospholipid comprises ester linkages of fatty acids in the 2 and 3 of glycerol positions containing chains of 12 to 26 carbon atoms and different head groups in the 1 position of glycerol that include choline, glycerol, inositol, serine, ethanolamine, as well as the corresponding phosphatidic acids. The chains on these fatty acids can be saturated or unsaturated, and the phospholipid can be made up of fatty acids of different chain lengths and different degrees of unsaturation. In certain embodiments, the phospholipid comprises distearoylphosphoethanolamine (DSPE), dimyristoyl phosphatidylethanolamine (DMPE), dipalmitoylphosphoethanolamine (DPPE), distearoylphosphatidylethanolamine (DSPE), dioleylphosphatidylethanolamine (DOPE), dipalmitoylphosphatidylcholine (DPPC), dimyristoylphosphatidylcholine (DMPC), dioleoylphosphatidylcholine (DOPC), distearoylphosphatidylcholine (DSPC), palmitoyl stearoylphosphatidyleholine (PSPC), diphosphatidylglycerol (DPG), dimyristoylphosphatidylglycerol (DMPG), dipalmitoylphosphatidylglycerol (DPPG), distearoylphosphatidylglycerol (DSPG), or mixture thereof.

In a particular embodiment, the phospholipid is a phosphatidylcholine (PC) or phosphatidylethanolamine (PE). In certain embodiments, the phosphatidylcholine or phosphatidylethanolamine is selected from the group consisting of distearoylphosphatidylcholine (DSPC), dioleoylphosphatidylcholine (DOPC), or distearoylphosphoethanoiamine (DSPE).

In various embodiments, the phospholipid comprises about 4 mol% to about 20 mol%, including values and subranges therebetween, of the total lipid present in the composition. In some embodiments, the phospholipid comprises about 4 to about 17 mol%, about 4 to about 15 mol%, about 4 to about 12 mol%, about 4 to about 8 mol%, about 7 to about 17 mol%, about 7 to about 15 mol%, about 7 to about 12 mol%, about 10 to about 15 mol%, about 10 to about 20 mol%, about 10 to about 17 mol%, about 12 to about 20 mol%, about 12 to about 18 mol%, about 15 to about 20 mol%, about 15 to about 18 mol%, or about 15 to about 17 mol%, including values and subranges therebetween, of the total lipid present in the composition.

In various embodiments, the phospholipid comprises about 4 to about 15 mol%, about 4 to about 10 mol%, about 10 to about 15 mol%, about 15 to about 20 mol%, or about 10 to about 20 mol%, of the total lipid present in the composition.

In one embodiment, the phospholipid comprises about 4 to about 8 mol%, including values therebetween, such as about 4, 4.5, 5, 5.5, 6, 6.5, 7, 7.1, 7.2, 7.3, 7.4, 7.5, 7.6, 7.7, 7.8, 7.9, or 8 mol%, of the total lipid present in the composition. In another embodiment, the phospholipid comprises about 15 to about 17 mol%, including values therebetween, such as about 15, 15.1, 15.2, 15.3, 15.4, 15.5, 15.6, 15.7, 15.8, 15.9, 16, 16.1, 16.2, 16.3, 16.4, 16.5, 16.6, 16.7, 16.8, 16.9, or 17 mol%, of the total lipid present in the composition. In yet another embodiment, the phospholipid comprises about 4 to about 17 mol%, including values therebetween, such as about 5, 6, 7, 8, 9, 10, 11, 12, 13, 14, 15, 16, or 16.5 mol%, of the total lipid present in the composition.

In some embodiments, the lipid particles of the compositions comprise or consist of a mixture of phospholipids. In these embodiments, the phospholipids comprise about 75, 80, 85, 90, 95, or about 100 mol%, including values therebetween, of the total lipid present in the composition. In an exemplary embodiment, the lipid particle comprises about 60, 70, or 80 mol% of phospholipid 1 and about 40, 30, or 20 mol% of phospholipid 2. For example, in one embodiment, the lipid particles comprises or consists of about 60, 65, 70, 75, or 80 mol% of 1,2-dioleoyl-sn-glycero-3-phosphoethanolamine-N-(dodecanyl) (NA-DOPE) and about 40, 35, 30, 25 or 20 mol% of DOPC.

In some embodiments, the lipid particles of the invention include sterols. Sterols for use with the invention include, but are not limited to, cholesterol, esters of cholesterol including cholesterol hemi-succinate, salts of cholesterol including cholesterol hydrogen sulfate and cholesterol sulfate, ergosterol, esters of ergosterol including ergosterol hemi-succinate, salts of ergosterol including ergosterol hydrogen sulfate and ergosterol sulfate, lanosterol, esters of lanosterol including lanosterol hemi-succinate, salts of lanosterol including lanosterol hydrogen sulfate, and lanosterol sulfate. A variety of sterols and their water soluble derivatives such as cholesterol hemisuccinate have been used to form liposomes; *see*, *e.g*., U.S. Patent No. 4,721,612.

In some embodiments, the lipid particles of the invention include methylated phenols, such as tocopherols. In one embodiment, the lipid particles include methylated phenols with vitamin E activity, e.g. α-tocopherol. The tocopherols for use with the invention include tocopherols, esters of tocopherols including tocopherol hemi-succinates (e.g. α-tocopherol hemi-succinate), salts of tocopherols including tocopherol hydrogen sulfates and tocopherol sulfates. PCT Publication No. WO 85/00968 describes a method for reducing the toxicity of drugs by encapsulating them in liposomes comprising α-tocopherol and certain derivatives thereof. Also, a variety of tocopherols and their water soluble derivatives have been used to form liposomes, *see* PCT Publication No. 87/02219. The methods described in these publications are amenable for use herein.

In a particular embodiment, the sterol used in the lipid particles of the invention is cholesterol hemisuccinate (CHEMS). In another particular embodiment, the tocopherol used in the lipid particles of the invention is tocopherol hemisuccinate (THS). In yet another particular embodiment, the lipid particles of the invention may include a mixture of CHEMS and THS.

In various embodiments, a sterol, a tocopherol, or a derivative thereof, comprises about 25 mol% to about 45 mol%, including values and ranges therebetween, of the total lipid present in the composition. In some embodiments, the sterol, tocopherol, or a derivative thereof comprises about 25 to about 40 mol%, about 25 to about 35 mol%, about 25 to about 30 mol%, about 30 to about 45 mol%, about 30 to about 40 mol%, about 30 to about 35 mol%, about 35 to about 45 mol%, or about 35 to about 40 mol%, including values and ranges therebetween, of the total lipid present in the composition. In certain embodiments, the sterol, tocopherol, or a derivative thereof comprises about 34 to about 45 mol% or about 34 to about 39 mol%, including values and ranges therebetween, of the total lipid present in the composition. According to the invention, the lipid particle comprises 40 mol% to 50 mol % of a neutral lipid comprising i) a phospholipid and ii) CHEMS or THS. Values falling outside the claimed range are not part of the invention.

In one embodiment, cholesterol, tocopherol, CHEMS or THS comprises about 25 mol% to about 45 mol%, including values and ranges therebetween, of the total lipid present in the composition. In some embodiments, cholesterol, tocopherol, CHEMS or THS comprises about 25 to about 40 mol%, about 25 to about 35 mol%, about 25 to about 30 mol%, about 30 to about 45 mol%, about 30 to about 40 mol%, about 30 to about 35 mol%, about 35 to about 45 mol%, or about 35 to about 40 mol%, including values and ranges therebetween, of the total lipid present in the composition. In certain embodiments, cholesterol, tocopherol, CHEMS or THS comprises about 34 to about 45 mol% or about 34 to about 39 mol%, including values and ranges therebetween, of the total lipid present in the composition.

In an exemplary embodiment, cholesterol, tocopherol, CHEMS or THS comprises about 34.1, 34.2, 34.3, 34.4, 34.5, 34.6, 34.7, 34.8, 34.9, 35, 35.1, 35.2, 35.3, 35.4, 35.5, 35.6, 35.7, 35.8, 35.9, 36, 36.1, 36.2, 36.3, 36.4, 36.5, 36.6, 36.7, 36.8, 36.9, 37, 37.1, 37.2, 37.3, 37.4, 37.5, 37.6, 37.7, 37.8, 37.9, 38, 38.1, 38.2, 38.3, 38.4, 38.5, 38.6, 38.7, 38.8, 38.9, or 39 mol%, of the total lipid present in the composition. In another exemplary embodiment, cholesterol, tocopherol, CHEMS or THS comprises about 25, 34.3, 34.4, 35.4, 38.5, or 45 mol%, of the total lipid present in the composition.

The lipid particles of the compositions further include a conjugated lipid. In one embodiment, the conjugated lipid is a PEGylated lipid. The PEGylated lipid, in one embodiment, comprises PEG400-PEG5000. For example, the PEGylated lipid can comprise PEG400, PEG500, PEG1000, PEG2000, PEG3000, PEG4000, or PEG5000. In a further embodiment the lipid component of the PEGylated lipid comprises cholesterol, dimyristoyl phosphatidylethanolamine (DMPE), dipalmitoyl phosphoethanolamine (DPPE), distearoylphosphatidylethanolamine (DSPE), dimyristoylglycerol glycerol (DMG), diphosphatidylglycerol (DPG) or disteraroylglycerol (DSG). In some embodiments, the PEGylated lipid is DMG-PEG2000, cholesterol-PEG2000 or DSPE-PEG2000. According to the invention, the conjugated lipid comprises DMG-PEG2000. Lipid particles not comprising DMG-PEG2000 do not form part of the invention.

Depending on its molecular weight (MW), PEG is also referred to in the art as polyethylene oxide (PEO) or polyoxyethylene (POE). The PEGylated lipid can include a branched or unbranched PEG molecule, and is not limited by a particular PEG MW. For example, the PEGylated lipid, in one embodiment, comprises a PEG molecule having a molecular weight of 300 g/mol, 400 g/mol, 500 g/mol, 1000 g/mol, 1500 g/mol, 2000 g/mol, 2500 g/mol, 3000 g/mol, 3500 g/mol, 4000 g/mol, 4500 g/mol, 5000 g/mol or 10,000 g/mol. According to the invention, the PEG has a MW of 2000 g/mol.

The conjugated lipid, for example the PEGylated lipid, can have a net-charge (e.g., cationic or anionic), or can be net-neutral. The lipids used in the PEGylated lipid component of the present invention can be synthetic, semi-synthetic or naturally-occurring lipid, including a phospholipid, a sphingolipid, a glycolipid, a ceramide, a tocopherol, a sterol, a fatty acid, or a glycoprotein such as albumin. In one embodiment, the lipid is a sterol. In a further embodiment, the sterol is cholesterol. In another embodiment, the lipid is a phospholipid described herein. In various embodiments, the PEGylated lipid of the composition provided herein comprises distearoylphosphoethanolamine (DSPE), dipalmitoylphosphatidylcholine (DPPC), dioleoylphosphatidylcholine (DOPC) dimyristoyl phosphatidylethanolamine (DMPE), dipalmitoylphosphoethanolamine (DPPE), distearoylphosphatidylethanolamine (DSPE), dimyristoylglyceroi (DMG), diphosphatidylglycerol (DPG) or disteraroylglycerol (DSG). According to the invention, the conjugated lipid comprises DMG-PEG2000. Lipid particles not comprising DMG-PEG2000 do not form part of the invention.

According to the invention, the conjugated lipid comprises a polyethyleneglycol (PEG) conjugated lipid. The PEG-conjugated lipid is PEG-l,2-Dimyristoyl-sn-glycerol (PEG-DMG) and has an average molecular weight of about 2000 daltons.

According to the invention, the conjugated lipid comprises 0.3 mol% to 1.5 mol%, such as about 0.3, 0.4, 0.5, 0.6, 0.7, 0.8, 0.9, 1.0, 1.1, 1.2, 1.3, 1.4, 1.5 mol%, of the total lipid present in the composition. In some embodiments, the conjugated lipid comprises about 1 to about 1.5 mol%, such as about 1, 1.1, 1.2, 1.3, 1.4, or about 1.5 mol%, of the total lipid present in the composition. According to the invention, the conjugated lipid comprises DMG-PEG2000.

In certain embodiments, the lipid particle of the composition comprises (a) a cationic lipid (e.g. DODAP) comprising about 50 mol% to about 57.5 mol% of the total lipid present in the composition; (b) a phospholipid (e.g., DSPC, DOPC, DSPE) comprising about 4 mol% to about 16.5 mol% of the total lipid present in the composition; (c) cholesterol hemisuccinate (CHEMS) or tocopherol hemisuccinate (THS) comprising about 25 mol% to about 45 mol%, of the total lipid present in the composition; and (d) a conjugated lipid (e.g. DMG-PEG2000) comprising about 1 mol% to about 1.5 mol% of the total lipid present in the composition.

In some embodiments, the lipid particle of the composition comprises (a) a cationic lipid (e.g. DODAP) comprising about 50 mol% to about 57.5 mol% of the total lipid present in the composition; (b) a phospholipid (e.g., DSPC, DOPC, DSPE) comprising about 4 mol% to about 16.5 mol% of the total lipid present in the composition; (c) cholesterol hemisuccinate (CHEMS) or tocopherol hemisuccinate (THS) comprising about 34 mol% to about 45 mol%, of the total lipid present in the composition; and (d) a conjugated lipid (e.g. DMG-PEG2000) comprising about 1 mol% to about 1.5 mol% of the total lipid present in the composition.

In some other embodiments, the lipid particle of the composition comprises (a) a cationic lipid (e.g. DODAP) comprising about 50 mol% to about 57.5 mol% of the total lipid present in the composition; (b) a phospholipid (e.g., DSPC, DOPC, DSPE) comprising about 4 mol% to about 16.5 mol% of the total lipid present in the composition; (c) cholesterol hemisuccinate (CHEMS) or tocopherol hemisuccinate (THS) comprising about 34 mol% to about 39 mol%, of the total lipid present in the composition; and (d) a conjugated lipid (e.g. DMG-PEG2000) comprising about 1 mol% to about 1.5 mol% of the total lipid present in the composition.

In yet some other embodiments, the lipid particle of the composition comprises (a) a cationic lipid (e.g. DODAP) comprising about 50 mol% to about 57.5 mol% of the total lipid present in the composition; (b) a phospholipid (e.g., DSPC, DOPC, DSPE) comprising about 4 mol% to about 16.5 mol% of the total lipid present in the composition; (c) cholesterol hemisuccinate (CHEMS) or tocopherol hemisuccinate (THS) comprising about 34.3 mol% of the total lipid present in the composition; and (d) a conjugated lipid (e.g. DMG-PEG2000) comprising about 1 mol% to about 1.5 mol% of the total lipid present in the composition.

In yet some other embodiments, the lipid particle of the composition comprises (a) a cationic lipid (e.g. DODAP) comprising about 50 mol% to about 57.5 mol% of the total lipid present in the composition; (b) a phospholipid (e.g., DSPC, DOPC, DSPE) comprising about 4 mol% to about 16.5 mol% of the total lipid present in the composition; (c) cholesterol hemisuccinate (CHEMS) or tocopherol hemisuccinate (THS) comprising about 25 mol% of the total lipid present in the composition; and (d) a conjugated lipid (e.g. DMG-PEG2000) comprising about 1 mol% to about 1.5 mol% of the total lipid present in the composition.

In yet some other embodiments, the lipid particle of the composition comprises (a) a cationic lipid (e.g. DODAP) comprising about 50 mol% to about 57.5 mol% of the total lipid present in the composition; (b) a phospholipid (e.g., DSPC, DOPC, DSPE) comprising about 4 mol% to about 16.5 mol% of the total lipid present in the composition; (c) cholesterol hemisuccinate (CHEMS) or tocopherol hemisuccinate (THS) comprising about 45 mol% of the total lipid present in the composition; and (d) a conjugated lipid (e.g. DMG-PEG2000) comprising about 1 mol% to about 1.5 mol% of the total lipid present in the composition.

In some embodiments, the compositions and/or lipid particles of the invention are free of anionic lipids (negatively charged lipid). However, if an anionic lipid is present, such lipids include phosphatidyl-glycerols (PGs), phosphatidic acids (PAs), phosphatidylinositols (Pis) and the phosphatidyl serines (PSs). Examples include DMPG, DPPG, DSPG, DMPA, DPP A, DSP A, DMPI, DPPI, DSPI, DMPS, DPPS and DSPS.

The compositions provided herein include an RNAi compound, complexed to, or encapsulated by a lipid component or a lipid particle. An RNAi compound is "complexed" to a lipid or a lipid component or a lipid particle and describes any composition, solution or suspension where at least about 1% by weight of the RNAi compound is associated (e.g., encapsulated or bound) with the lipid either as part of a complex, for example, as part of a microparticle, nanoparticle, micelle or liposome. The complex, in one embodiment, is formed by one or more electrostatic interactions, hydrophobic interactions, hydrogen bonds or by the encapsulation of the RNAi compound by the lipid, *e.g*., in a micelle or liposome. For example, the lipid-complexed composition, in one embodiment, comprises a plurality of liposomes, and the RNAi compound may be in the aqueous phase (encapsulated by the liposome), the hydrophobic bilayer phase, at the interfacial headgroup region of the liposomal bilayer or a combination thereof. In one embodiment, prior to administration of the composition to a patient in need thereof, at least about 5%, at least about 10%, at least about 20%, at least about 25%, at least about 50%, at least about 75%, at least about 80%, at least about 85%, at least about 90% or at least about 95% of the RNAi compound in the composition is lipid complexed. Association, in one embodiment, is measured by separation through a filter where lipid and lipid-associated drug is retained (*i.e*., in the retentate) and free drug is in the filtrate.

In one embodiment, the lipid particle is complexed to an RNAi compound. The complex, in one embodiment, is a microparticle, nanoparticle, micelle or liposome, or a combination thereof.

In one embodiment, the lipid complex is a liposome or a plurality of liposomes, and the RNAi compound is associated with the liposome surface, or present in the aqueous interior of the liposome (or plurality of liposomes). Liposomes are completely closed lipid bilayer membranes containing an entrapped aqueous volume. Liposomes may be unilamellar vesicles (possessing a single membrane bilayer) or multilamellar vesicles (onion-like structures characterized by multiple membrane bilayers, each separated from the next by an aqueous layer) or a combination thereof. The bilayer is composed of two lipid monolayers having a hydrophobic "tail" region and a hydrophilic "head" region. The structure of the membrane bilayer is such that the hydrophobic (nonpolar) "tails" of the lipid monolayers orient toward the center of the bilayer while the hydrophilic "heads" orient towards the aqueous phase.

In one embodiment, when formulated together, the RNAi compound and lipid component form a plurality of lipid particles (*e.g*., microparticles or nanoparticles). In one embodiment, the mean diameter of the plurality of lipid particles is from about 20 nm to about 2 µm, for example about 50 nm to about 1 µm, about 200 nm to about 1 µm, about 100 nm to about 800 nm, about 100 nm to about 600 nm or about 100 nm to about 500 nm.

In one lipid particle embodiment, the RNAi compound (e.g., one or more siRNAs, one or more shRNAs, one or more miRNAs, or a combination thereof) compound is present in the composition at 5 mol% - 99 mol%. In a further embodiment, the compound is present in the composition at 40 mol% - 95 mol%. In a further embodiment, the siRNA compound is present in the composition at 40 mol% - 60 mol%. In one embodiment, the siRNA compound is present in the composition at about 40 mol% or about 45 mol%.

In some embodiments, the compositions, systems and methods provided herein comprise a lipid complexed or a liposome encapsulated RNAi compound. The lipids used in the pharmaceutical compositions of the present invention as provided throughout can be synthetic, semi-synthetic or naturally-occurring lipids. As provided above, where RNAi compounds are employed, cationic lipids can be complexed thereto via electrostatic interactions.

In one embodiment, the composition may include dipalmitoylphosphatidylcholine (DPPC), a major constituent of naturally-occurring lung surfactant.

Without wishing to be bound by theory lipid microparticles, nanoparticles or liposomes, containing such lipids as cationic lipids and phosphatidylcholines, aid in the uptake of the RNAi compound by the cells in the lung (*e.g*., neutrophils, macrophages, and fibroblasts) and helps to maintain the RNAi compound in the lung.

The lipid particles of the present invention in which an interfering RNA is complexed or fully or partially encapsulated in a lipid particle can be formed by any method known in the art including, but not limited to, a continuous mixing method or a direct dilution process. Exemplary methods of producing lipid particles are disclosed in U.S. Patent No. 8,058,069.

For example, in certain embodiments, the lipid particles of the present invention are produced via a continuous mixing method, e.g., a process that includes providing an aqueous solution comprising a nucleic acid such as an interfering RNA in a first reservoir, providing an organic lipid solution in a second reservoir, and mixing the aqueous solution with the organic lipid solution such that the organic lipid solution mixes with the aqueous solution so as to substantially instantaneously produce a liposome encapsulating the nucleic acid (e.g., interfering RNA). This process and the apparatus for carrying this process are described in detail in U.S. Patent Publication No. 20040142025.

The action of continuously introducing lipid and buffer solutions into a mixing environment, such as in a mixing chamber, causes a continuous dilution of the lipid solution with the buffer solution, thereby producing a liposome substantially instantaneously upon mixing. As used herein, the phrase "continuously diluting a lipid solution with a buffer solution" (and variations) generally means that the lipid solution is diluted sufficiently rapidly in a hydration process with sufficient force to effectuate vesicle generation. By mixing the aqueous solution comprising a nucleic acid with the organic lipid solution, the organic lipid solution undergoes a continuous stepwise dilution in the presence of the buffer solution (i.e., aqueous solution) to produce a nucleic acid-lipid particle.

The lipid particles formed using the continuous mixing method typically have a size of from about 40 nm to about 150 nm, from about 50 nm to about 150 nm, from about 60 nm to about 130 nm, from about 70 nm to about 110 nm, or from about 70 nm to about 90 nm. The particles thus formed do not aggregate and are optionally sized to achieve a uniform particle size.

In another embodiment, the lipid particles of the present invention are produced via a direct dilution process that includes forming a liposome solution and immediately and directly introducing the liposome solution into a collection vessel containing a controlled amount of dilution buffer. In preferred aspects, the collection vessel includes one or more elements configured to stir the contents of the collection vessel to facilitate dilution. In one aspect, the amount of dilution buffer present in the collection vessel is substantially equal to the volume of liposome solution introduced thereto. As a non-limiting example, a liposome solution in 45% ethanol when introduced into the collection vessel containing an equal volume of dilution buffer will advantageously yield smaller particles.

In yet another embodiment, the lipid particles of the present invention are produced via a direct dilution process in which a third reservoir containing dilution buffer is fluidly coupled to a second mixing region. In this embodiment, the liposome solution formed in a first mixing region is immediately and directly mixed with dilution buffer in the second mixing region. In preferred aspects, the second mixing region includes a T-connector arranged so that the liposome solution and the dilution buffer flows meet as opposing 180° flows; however, connectors providing shallower angles can be used, e.g., from about 27° to about 180°. A pump mechanism delivers a controllable flow of buffer to the second mixing region. In one aspect, the flow rate of dilution buffer provided to the second mixing region is controlled to be substantially equal to the flow rate of liposome solution introduced thereto from the first mixing region. This embodiment advantageously allows for more control of the flow of dilution buffer mixing with the liposome solution in the second mixing region, and therefore also the concentration of liposome solution in buffer throughout the second mixing process. Such control of the dilution buffer flow rate advantageously allows for small particle size formation at reduced concentrations.

These processes and the apparatuses for carrying out these direct dilution processes are described in detail in U.S. Patent Publication No. 20070042031.

The lipid particles formed using the direct dilution process typically have a size of from about 40 nm to about 150 nm, from about 50 nm to about 150 nm, from about 60 nm to about 130 nm, from about 70 nm to about 110 nm, or from about 70 nm to about 90 nm. The particles thus formed do not aggregate and are optionally sized to achieve a uniform particle size.

If needed, the lipid particles of the invention can be sized by any of the methods available for sizing liposomes. The sizing may be conducted in order to achieve a desired size range and relatively narrow distribution of particle sizes.

Several techniques are available for sizing the particles to a desired size. One sizing method, used for liposomes and equally applicable to the present particles, is described in U.S. Pat. No. 4,737,323. Sonicating a particle suspension either by bath or probe sonication produces a progressive size reduction down to particles of less than about 50 nm in size. Homogenization is another method which relies on shearing energy to fragment larger particles into smaller ones. In a typical homogenization procedure, particles are recirculated through a standard emulsion homogenizer until selected particle sizes, typically between about 60 and about 80 nm, are observed. In both methods, the particle size distribution can be monitored by conventional laser-beam particle size discrimination, or QELS.

Extrusion of the particles through a small-pore polycarbonate membrane or an asymmetric ceramic membrane is also an effective method for reducing particle sizes to a relatively well-defined size distribution. Typically, the suspension is cycled through the membrane one or more times until the desired particle size distribution is achieved. The particles may be extruded through successively smaller-pore membranes, to achieve a gradual reduction in size.

In some embodiments, the RNAi compounds in the composition are precondensed as described in, e.g., U.S. patent application Ser. No. 09/744,103.

In other embodiments, the methods will further comprise adding non-lipid polycations which are useful to effect the lipofection of cells using the present compositions. Examples of suitable non-lipid polycations include, hexadimethrine bromide (sold under the brandname POLYBRENE RTM , from Aldrich Chemical Co., Milwaukee, Wis., USA) or other salts of hexadimethrine. Other suitable polycations include, for example, salts of poly-L-ornithine, poly-L-arginine, poly-L-lysine, poly-D-lysine, polyallylamine, and polyethyleneimine. Addition of these salts is preferably after the particles have been formed.

Liposomes can be produced by a variety of methods and the present invention is not limited to a particular type of liposomal manufacturing method. In one embodiment, one or more of the methods described in U.S. Patent Application Publication No. 2008/0089927 or WO 2013/177226 are used herein to produce the RNAi compound encapsulated lipid compositions (liposomal dispersion).

In one embodiment, the liposomal composition is formed by dissolving one or more lipids in an organic solvent forming a lipid solution, and the siRNA coacervate forms from mixing an aqueous solution of the siRNA with the lipid solution. In a further embodiment, the organic solvent is ethanol. In even a further embodiment, the one or more lipids comprise a phospholipid and a sterol or a tocopherol. The phospholipid, in one embodiment is net neutral or net cationic.

In one embodiment, liposomes are produces by sonication, extrusion, homogenization, swelling, electroformation, inverted emulsion or a reverse evaporation method. Bangham's procedure (J. Mol. Biol. (1965)) produces ordinary multilamellar vesicles (MLVs). Lenk et al. (U.S. Patent Nos. 4,522,803, 5,030,453 and 5,169,637), Fountain et al. (U.S. Patent No. 4,588,578) and Cullis et al. (U.S. Patent No. 4,975,282) disclose methods for producing multilamellar liposomes having substantially equal interlamellar solute distribution in each of their aqueous compartments. U.S. Patent No. 4,235,871 discloses preparation of oligolamellar liposomes by reverse phase evaporation. Each of the methods is amenable for use with the present invention.

Unilamellar vesicles can be produced from MLVs by a number of techniques, for example, the extrusion techniques of U.S. Patent No. 5,008,050 and U.S. Patent No. 5,059,421. Sonication and homogenization cab be so used to produce smaller unilamellar liposomes from larger liposomes (*see,* for example, Paphadjopoulos et al. (1968); Deamer and Uster (1983); and Chapman *et al.* (1968).

The liposome preparation of Bangham et al. (J. Mol. Biol. 13, 1965, pp. 238-252) involves suspending phospholipids in an organic solvent which is then evaporated to dryness leaving a phospholipid film on the reaction vessel. Next, an appropriate amount of aqueous phase is added, the 60 mixture is allowed to "swell," and the resulting liposomes which consist of multilamellar vesicles (MLVs) are dispersed by mechanical means. This preparation provides the basis for the development of the small sonicated unilamellar vesicles described by Papahadjopoulos et al. (Biochim. Biophys. Acta. 135, 1967, pp. 624-638), and large unilamellar vesicles.

Techniques for producing large unilamellar vesicles (LUVs), such as, reverse phase evaporation, infusion procedures, and detergent dilution, can be used to produce liposomes for use in the pharmaceutical compositions provided herein. A review of these and other methods for producing liposomes may be found in the text Liposomes, Marc Ostro, ed., Marcel Dekker, Inc., New York, 1983, Chapter 1. *See also* Szoka, Jr. et al., (Ann. Rev. Biophys. Bioeng. 9, 1980, p. 467).

Other techniques for making liposomes amenable for making the compositions described herein include those that form reverse-phase evaporation vesicles (REV), *see*, *e.g.*, U.S. Patent No. 4,235,871. Another class of liposomes that may be used is characterized as having substantially equal lamellar solute distribution. This class of liposomes is denominated as stable plurilamellar vesicles (SPLV) as defined in U.S. Patent No. 4,522,803, and includes monophasic vesicles as described in U.S. Patent No. 4,588,578, and frozen and thawed multilamellar vesicles (FATMLV) as described above.

The composition, in one embodiment, comprises a plurality of lipid particles with a mean diameter that is measured by a light scattering method, of approximately 0.005 microns to approximately 3.0 microns, for example, in the range about 0.1 µm to about 1.0 µm. In one embodiment, the mean diameter of the plurality of particles in the composition is about 50 nm to about 2 µm, about 50 nm to about 1.5 µm, about 50 nm to about 1 µm, 50 nm to about 900 nm, about 50 nm to about 800 nm, about 50 nm to about 700 nm, about 50 nm to about 600 nm, about 50 nm to about 500 nm. In another embodiment, the mean diameter of the plurality of particles in the composition is from about 200 nm to about 1.8 µm, from about 200 nm to about 1.7 µm, from about 200 nm to about 1.6 µm, from about 200 nm to about 1.5 µm, from about 200 nm to about 1.4 µm, from about 200 nm to about 1.3 µm, from about 200 nm to about 1.2 µm or from about 200 nm to about 1.1 µm.

The plurality of lipid particles, in one embodiment, comprises a plurality of liposomes. In one embodiment, the plurality of liposomes have a mean diameter that is measured by a light scattering method, of approximately 0.01 microns to approximately 3.0 microns, for example, in the range about 0.2 to about 1.0 microns. In one embodiment, the mean diameter of the plurality of liposomes in the composition is about 150 nm to about 2 µm, about 200 nm to about 1.9 µm, about 200 nm to about 1.8 µm, about 200 nm to about 1.7 µm, about 200 nm to about 1.6 µm, about 200 nm to about 1.5 µm, about 200 nm to about 1.4 µm, about 200 nm to about 1.3 µm, about 200 nm to about 1.2 µm, about 200 nm to about 1.1 µm, about 200 nm to about 1 µm, 200 nm to about 900 nm, about 200 nm to about 800 nm, about 200 nm to about 700 nm, about 200 nm to about 600 nm, about 200 nm to about 500 nm.

In order to minimize dose volume and reduce patient dosing time, in one embodiment, it is important that liposomal entrapment or complexing of the lipid component to the RNAi compound be highly efficient and that the lipid-to RNAi compound ratio be at as low a value as possible. In one embodiment, the weight ratio of the lipid component to RNAi compound is 2 to 1 ("lipid component to RNAi compound" or "lipid component:RNAi compound") or less (*e.g.*, from about 2:1.0 to about 0.01:1.0, or from about 2:1.0 to about 0.1:1.0). In another embodiment, the weight ratio of the lipid component to RNAi compound is 1.5 to 1.0 ("lipid component to RNAi compound" or "lipid component:RNAi compound") or less (*e.g.*, from about 1.5:1.0 to about 0.01:1.0, or from about 1.5:1 to about 0.1:1.0). In another embodiment, the weight ratio of the lipid component to RNAi compound is 1.0 to 1.0 ("lipid component to RNAi compound" or "lipid component:RNAi compound") or less (*e.g.*, from about 1.0:1.0 to about 0.01:1.0, or from about 1.0:1.0 to about 0.1:1.0), or from about 1.0:1.0 to about 0.5:1.0.

In some embodiments, the RNAi compound to lipid ratios (mass/mass ratios) in the composition will range from about 0.01 to about 0.2, from about 0.02 to about 0.1, from about 0.03 to about 0.1, or from about 0.01 to about 0.08. The ratio of the starting materials also falls within this range. In other embodiments, the preparation uses about 400 µg nucleic acid per 10 mg total lipid or a nucleic acid to lipid mass ratio of about 0.01 to about 0.08 and, more preferably, about 0.04, which corresponds to 1.25 mg of total lipid per 50 µg of nucleic acid. In other preferred embodiments, the particle has a nucleic acid:lipid mass ratio of about 0.08.

In other embodiments, the lipid to RNAi compound ratios (mass/mass ratios) in the composition will range from about 1 (1:1) to about 100 (100:1), from about 5 (5:1) to about 100 (100:1), from about 1 (1:1) to about 50 (50:1), from about 2 (2:1) to about 50 (50:1), from about 3 (3:1) to about 50 (50:1), from about 4 (4:1) to about 50 (50:1), from about 5 (5:1) to about 50 (50:1), from about 1 (1:1) to about 25 (25:1), from about 2 (2:1) to about 25 (25:1), from about 3 (3:1) to about 25 (25:1), from about 4 (4:1) to about 25 (25:1), from about 5 (5:1) to about 25 (25:1), from about 5 (5:1) to about 20 (20:1), from about 5 (5:1) to about 15 (15:1), from about 5 (5:1) to about 10 (10:1), about 5 (5:1), 6 (6:1), 7 (7:1), 8 (8:1), 9 (9:1), (10:1), 11 (11:1), 12 (12:1), 13 (13:1), 14 (14:1), or 15 (15:1). The ratio of the starting materials also falls within this range.

The composition, in one embodiment, comprises a plurality of microparticles or nanoparticles comprising one or more of the RNAi compounds (*e.g.*, siRNA, shRNA or miRNA) as described herein complexed to a lipid component, and a hydrophobic additive. In one embodiment, the hydrophobic additive (*e.g.*, an additive that is at least partially hydrophobic) is a hydrocarbon, a terpene compound or a hydrophobic lipid (*e.g.*, tocopherol, tocopherol acetate, sterol, sterol ester, alkyl ester, vitamin A acetate, a triglyceride, a phospholipid). The hydrocarbon can be aromatic, an alkane, alkene, cycloalkane or an alkyne. In one embodiment, the hydrocarbon is an alkane (*i*.*e*., a saturated hydrocarbon). In another embodiment, the hydrocarbon is a C₁₅-C₅₀ hydrocarbon. In a further embodiment, the hydrocarbon is a C₁₅, C₂₀, C₂₅, C₃₀, C₃₅, C₄₀, C₄₅ or C₅₀ hydrocarbon. In yet another embodiment, the hydrophobic additive is a C₁₅-C₂₅ hydrocarbon, C₁₅-C₃₅ hydrocarbon, C₁₅-C₄₅ hydrocarbon, C₁₅-C₂₀ hydrocarbon, C₂₀-C₂₅ hydrocarbon, C₂₅-C₃₀ hydrocarbon, C₃₀-C₃₅ hydrocarbon, C₃₅-C₄₀ hydrocarbon, C₄₀-C₄₅ hydrocarbon or a C₄₅-C₅₀ hydrocarbon.

The hydrophobic additive, when present in the composition, in one embodiment, is present at 25 mol% - 50 mol%, for example, 30 mol% - 50 mol%, 35 mol% - 45 mol%. In even a further embodiment, the hydrophobic additive is present in the composition at about 40 mol% or about 45 mol%.

In one embodiment, a composition comprising an RNAi compound (e.g., one or more siRNAs, one or more shRNAs, one or more miRNAs, or a combination thereof) compound, a lipid component, and a terpene compound *(e.g.,* the hydrophobic additive) is provided. The composition, in a further embodiment, comprises a cationic lipid, e.g., a PEGylated cationic lipid, as the lipid component. The terpene compound (hydrophobic additive), in one embodiment, is a hydrocarbon (*e.g.*, isoprene, squalaneor squalene). In another embodiment, the terpene compound is a hemiterpene (C₅H₈), monoterpene (C₁₀H₁₆), sesquiterpene (C₁₅H₂₄), diterpene (C₂₀H₃₂) (*e.g.*, cafestol, kahweol, cembrene, taxadiene), sesterterpene (C₂₅H₄₀), triterpene (C₃₀H₄₈), sesquaterpene (C₃₅H₅₆), tetraterpene (C₄₀H₆₄), polyterpene (*e.g.*, a polyisoprene with *trans* double bonds) or a norisoprenoid (*e.g.*, 3-oxo-α-ionol, 7,8-dihydroionone derivatives). The terpene compound, in another embodiment, is selected from one of the compounds provided in Table 3, below. In one embodiment, the hydrophobic additive is squalane.

| **Table 3. Terpene hydrophobic additives amenable for use in the compositions of the present invention.** | |
|---|---|
| Name | Formula |
| Isoprene | 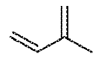 |
| Limonene | 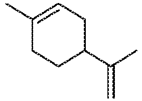 |
| humulene | 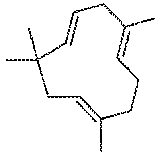 |
| farnasene | 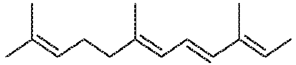 |
| squalene | 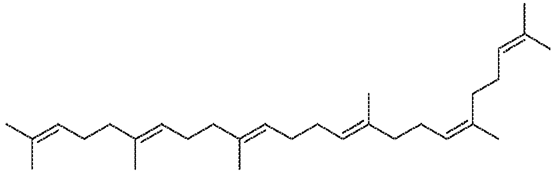 |
| squalane | 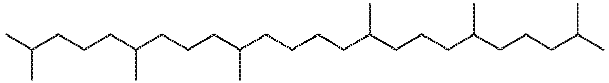 |

### RNAi compounds and their targets

The term "interfering RNA" or "RNAi" or "interfering RNA sequence" refers to single-stranded RNA (e.g., mature miRNA) or double-stranded RNA (i.e., duplex RNA such as siRNA, aiRNA, or pre-miRNA) that is capable of reducing or inhibiting the expression of a target gene or sequence (e.g., by mediating the degradation or inhibiting the translation of mRNAs which are complementary to the interfering RNA sequence). Interfering RNA thus refers to the single-stranded RNA that is complementary to a target mRNA sequence or to the double-stranded RNA formed by two complementary strands or by a single, self-complementary strand. Interfering RNA may have substantial or complete identity to the target gene or sequence, or may comprise a region of mismatch (i.e., a mismatch motif). The sequence of the interfering RNA can correspond to the full-length target gene, or a subsequence thereof.

Those of ordinary skill in the art will recognize that, in principle, either strand of an siRNA can be incorporated into RISC and function as a guide/antisense strand. It should be noted that, siRNA design (*e.g*., decreased siRNA duplex stability at the 5' end of the desired guide strand) can favor incorporation of the desired guide strand into RISC.

The antisense strand of an siRNA is the active guiding agent of the siRNA in that the antisense strand is incorporated into RISC, thus allowing RISC to identify target mRNAs with at least partial complementarity to the antisense siRNA strand for cleavage or translational repression. RISC-related cleavage of mRNAs having a sequence at least partially complementary to the guide strand leads to a decrease in the steady state level of that mRNA and of the corresponding protein encoded by this mRNA. Alternatively, RISC decreases expression of the corresponding protein via translational repression without cleavage of the target mRNA.

The present invention provides pharmaceutical compositions comprising an RNA interference (RNAi) compound complexed to or encapsulated by a lipid particle. The RNAi compound targets a messenger RNA (mRNA) whose corresponding protein function associated with a phagocytic cell response, for example an inflammatory response (*e.g*., release of one or more lipid mediators), degranulation of a granule in a granulocyte (*e.g.*, neutrophil degranulation), or recruitment of an immune cell or a granulocyte to a site of a lung infection. For example, in one embodiment, the RNAi compound targets an mRNA whose corresponding protein function is associated with granulocyte degranulation, for example, eosinophil, basophil, mast cell, or neutrophil cell degranulation is provided.

According to the invention, the RNAi compound is targeting an mRNA that encodes COL1A1 or annexin A11 (ANXA11). Embodiments of the present description in which the nucleic acid compound is not RNAi and which does not target an mRNA encoding COL1A1 or annexin A11 do not form part of the claimed invention.

An mRNA that is targeted by an RNAi compound, which is also referred to herein as a "target mRNA" means an mRNA comprising a complementary sequence or substantially complementary to an interfering RNA strand (*e.g.*, an siRNA strand). Target mRNA can be non-human animal or human mRNA. A target mRNA need not be 100% complementary to an interfering RNA strand, as long as the interfering RNA functions to silence or otherwise form a RISC complex with the target mRNA. In one embodiment, the interfering RNA strand (*e.g.*, siRNA strand) is 100% complementary, at least about 99% complementary, at least about 95% complementary, at least about 90% complementary, at least about 85% complementary, at least about 80% complementary, at least about 75% percent complementary or at least about 70% complementary to the target mRNA. Target mRNAs are described herein.

Interfering RNAs of the invention, in one embodiment, act in a catalytic manner for cleavage of target mRNA. In other words, siRNA compositions described herein are able to effect inhibition of target mRNA in substoichiometric amounts. In one embodiment, the siRNA compound, present in the composition of the invention is recycled, with 1 siRNA molecule capable of inducing cleavage of at least about 500 or at least about 1000 mRNA molecules. Accordingly, as compared to antisense therapies, significantly less siRNA is needed to provide a therapeutic effect under such cleavage conditions.

The term "siRNA" as used herein refers to a double-stranded RNAi compound (interfering RNA compound) unless otherwise noted. siRNA of the invention is a double-stranded nucleic acid molecule comprising two nucleotide strands, each strand having about 17 to about 30 nucleotides (*e.g.*, about 17, 18, 19, 20, 21, 22, 23, 24, 25, 26, 27, 28, 29 or 30 nucleotides). Besides "siRNA" molecules, other RNAi compounds are amenable for use with the present invention. Examples of other interfering RNA molecules that can interact with RISC and activate the RNA interference pathway include short hairpin RNAs (shRNAs), single-stranded siRNAs, microRNAs (miRNAs), and dicer-substrate 27-mer duplexes. For the purposes of the present invention, any RNA or RNA-like molecule (*e.g.*, an RNA molecule with a chemical modification, a DNA substitution or a non-natural nucleotide) that can interact with RISC and participate in the RNA interference pathway are referred to herein as an RNAi compound of the invention.

According to the invention, the nucleic acid compound of the compositions is an RNA interference (RNAi) compound. The RNAi compound includes a small interfering RNA (siRNA), short hairpin RNA (shRNA), and micro RNA (miRNA).

In one embodiment, the RNAi compound is an siRNA, shRNA or miRNA and is shorter than about 30 nucleotides in length, for example, to prevent nonspecific mRNA silencing. In one embodiment, the RNAi compound of the invention is about 15 to 29 nucleotides in length. In one embodiments, the siRNA sequences of the invention are about 18, about 19, about 20, about 21, about 22, about 23, about 24, about 25, about 26, about 27, about 28, or about 29 nucleotides long. The siRNA sequences of the invention may be modified, *e.g.*, chemically or comprising alternating motifs, (*see*, *e.g.*, Braasch *et al*., (2003); Chiu et al., (2003); PCT publications WO 2004/015107 and WO 02/44321, U.S. Pat. Nos. 5,898,031, and 6,107,094, US patent publications 2005/0080246, and 2005/0042647). For example, siRNA oligonucleotides may be modified with the inclusion of a 5'-phosphate moiety or 2'-O-methyl modifications.

In one embodiment, the RNAi compound is present in the composition as the double-stranded RNAi compound or single stranded RNAi compound (*e.g.*, as the siRNA compound without the need to express the interfering RNA endogenously).

As described herein, the target cell in one embodiment is a phagocyte. In one embodiment, the target cell is a granulocyte. In a further embodiment, the target cell is a neutrophil. In yet another embodiment, the target cell is selected from a neutrophil, eosinophil, basophil, mast cell, macrophage, monocyte or dendritic cell. In one embodiment, the cell is a mononuclear phagocyte. In a further embodiment, the mononuclear phagocyte is a monocyte or a macrophage. In even a further embodiment, the macrophage is an alveolar macrophage.

In one embodiment, the composition provided herein comprises one or more RNAi compounds complexed to a lipid particle. For example, two or more siRNAs, two or more shRNAs or a combination of siRNA and shRNA can be present in the composition. In one embodiment, the composition comprises a lipid particle complexed to one or more of an siRNA, shRNA or miRNA.

The RNAi compounds may comprise unmodified ribonucleotides or a combination of unmodified ribonucleotides and ribonucleotides and/or non-natural ribonucleotides.

In various embodiments, the compositions of the invention comprise an RNAi compound that targets an mRNA whose corresponding protein product plays an important role in the pathogenesis of a pulmonary disease/disorder. According to the invention, the compositions of the invention comprise an RNAi compound that targets an mRNA involved in the pathogenesis of pulmonary fibrosis or sarcoidosis.

For example, idiopathic pulmonary fibrosis (IPF) is believed to be the result of an aberrant wound healing process including/involving abnormal and excessive deposition of collagen in the pulmonary tissue. Thus, in one embodiment, the compositions of the present invention comprise RNAi compounds that target one or more mRNAs involved in the process of collagen synthesis.

It is known that COL1A1 gene encodes the pro-alpha1 chains of type I collagen whose triple helix comprises two alpha1 chains and one alpha2 chain. In one embodiment, the compositions of the invention comprise an RNAi compound, such as an siRNA, that targets the COL1A1 mRNA. In another embodiment, which is not claimed, the compositions of the invention comprise an siRNA targeting the alpha2 chain, e.g. an siRNA targeting COL1A2 mRNA.

Studies have shown that other types of collagens, such as collagen III, IV, and V are also associated with the pathogenesis of pulmonary fibrosis. Accordingly, in certain embodiments, the invention provides compositions comprising an siRNA targeting collagen types III, IV, or V; e.g. compositions comprising siRNAs targeting COL3A1, COL4A1, COL4A2, COL4A3, COL4A4, COL4A5, COL4A6, COL5A1, COL5A2, or COL5A3 mRNA. According to the invention, the RNAi compound is targeting an mRNA that encodes COL1A1 or annexin A11.

In another embodiment, the compositions of the invention comprise an RNAi compound that targets an mRNA encoding prolyl 4-hydroxylase, a key enzyme in collagen synthesis composed of two identical alpha subunits and two beta subunits. Prolyl 4-hydroxylase catalyzes the formation of 4-hydroxyproline that is essential to the proper three-dimensional folding of newly synthesized procollagen chains. In an exemplary embodiment, which is not claimed, the RNAi compound targets the P4HA1 mRNA that encodes one of several different types of alpha subunits.

TGFβ has been implicated in the pathogenesis of pulmonary fibrosis. Accordingly, in one embodiment, which is not claimed, the compositions of the invention comprise an siRNA targeting TGFβ or a receptor for TGFβ.

Sarcoidosis involves the formation of sarcoid granulomas in various organs including lungs of the patients. Monocytes and macrophages are involved in the formation of sarcoid granulomas and also secrete a range of cytokines that further enhance the immune response. For example alveolar macrophages secrete tumor necrosis factor α (TNFα) which is believed to play an important role in both formation and maintenance of sarcoid granulomas. Accordingly, in one embodiment, the compositions of the invention comprise an RNAi compound that targets the TNFα mRNA.

Additionally, a genome wide association study recently identified a single nucleotide polymorphism (SNP) in the annexin A11 gene as a potential genetic factor linked to susceptibility to sarcoidosis. In one embodiment, the compositions of the invention comprise an RNAi compound that targets the annexin A11 mRNA. In one embodiment, the RNAi compound targets the variant form of the annexin A11 mRNA that is linked to susceptibility to sarcoidosis.

In one embodiment, which is not claimed, the compositions of the invention comprise an RNAi compound that targets the receptors for cytokines and chemokines described herein. For example, in one embodiment, the compositions comprise an RNAi compound that targets a receptor for TNFα. In another embodiment, the compositions comprise an RNAi compound that targets an IL-6 receptor, IFNγ receptor, IL-12 receptor, or IL-17 receptor.

In yet some other embodiments, which are not claimed, the RNAi compound may target any number of mRNAs whose corresponding proteins are associated with the phagocytic cell processes such as an inflammation response, degranulation or recruitment of an immune cell or granulocyte to a site of lung infection (*i.e.*, chemotaxis). Phagocytic cells are implicated in numerous pulmonary disorders, and include neutrophils, basophils, eosinophils, mast cells, macrophages or monocytes, dendritic cells, and fibroblasts. The composition, for example, is a liposomal composition or a lipid nanoparticle composition as described herein.

Inflammation in the lungs of patients is characterized by persistent and excessive neutrophil infiltration. Neutrophils and other phagocytic cells have also been found to release large quantities of destructive oxidases and proteases. In one aspect, the present invention provides compositions, systems and methods to treat and/or prevent lung injury in the CF lung by inhibiting the degranulation of phagocytic cells. The compositions, systems and methods described herein, in one aspect, may be used to treat lung injury or a lung disease in a patient in need thereof by impeding the mechanism of degranulation of a granulocyte, for example, by impeding neutrophil degranulation.

The composition may comprise an RNAi compound that targets an mRNA encoding a structural component of a granule in a granulocyte, a protein that modulates or signals the production of granules (*e.g.*, a cell signaling compound), or degranulation of a granule.

The granulocyte may be a neutrophil, mast cell, basophil, eosinophil or monocyte.

In one embodiment, which is not claimed, the RNAi compound targets an mRNA whose corresponding protein function is associated with phagocytic cell degranulation. In a further embodiment, the phagocytic cell degranulation is neutrophil degranulation. In even a further embodiment, neutrophil degranulation comprises primary granule degranulation. However, the degranulation is not limited to primary granules. Rather, the RNAi compound in another embodiment, targets an mRNA whose corresponding protein function is associated with secondary granule, tertiary granule or secretory vesicle degranulation in neutrophils.

For example, in the case of neutrophil degranulation, in one embodiment, a RNAi compound may target an mRNA that encodes a protein associated with the process of degranulating a primary granule, a secondary granule, a tertiary granule or a secretory vesicle.

The RNAi compound may target an mRNA whose corresponding protein function is associated with degranulation of a primary neutrophil granule, which stores toxic mediators such as elastase, myeloperoxidase, cathepsins and defensins. Mechanisms of degranulation of neutrophils are provided in Lacy (2006). Allergy, Asthma, and Clinical Immunology 2, pp. 98-108. The mRNA expression of one or more of the targets described in Lacy as having a function in neutrophil degranulation may be targeted by one or more of the RNAi compounds of the present invention.

In certain instances, initiation and propagation of lung damage is a consequence of an exaggerated inflammatory response. Although inflammation is a physiological protective response to injury or infection and designed to facilitate repair, the inflammatory response sometimes results in further injury and organ dysfunction. For example, inflammatory chronic pulmonary disorders, chronic obstructive pulmonary disease (COPD), acute lung injury, acute respiratory distress syndrome, and cystic fibrosis are syndromes of severe pulmonary dysfunction resulting from a massive inflammatory response. One of the histological hallmarks of these chronic inflammatory pulmonary disorders is the accumulation of neutrophils in the microvasculature of the lung (Korkmaz et al. 2010). Pharmacol. Rev. 62, pp. 726-759. The present invention addresses the need of an effective treatment of one or more of these disorders, among others, *see*, *e.g.*, Tables 4-7, by providing an RNAi composition comprising a lipid component and an RNAi compound whose target is an mRNA that encodes a protein involved with neutrophil recruitment (or other phagocytic recruitment) to the site of inflammation, an inflammatory molecule such as a cytokine or chemokine, or a protein involved in neutrophil degranulation (*e.g.*, a cell fusion protein or a vesicle protein).

Neutrophils are the most abundant (40% to 75%) type of white blood cells and form an essential part of the innate immune system. Neutrophils contribute to the pathogenesis of various pulmonary disorders. The destructive features of neutrophils are highly detrimental in the settings of the lung disease microenvironment. Accordingly, without wishing to be bound by theory, the composition provided herein is thought to be effective in treating lung disease or lung injury by inhibiting the release of toxic mediators from neutrophil granules.

Neutrophils comprise multiple mediators that are released from granules. Within the primary granule, at least the following mediators can be found: elastase, myeloperoxidase, cathepsin G, α-defensins, and azurocidin 1. In one embodiment, the RNAi compound of the invention targets a myeloperoxidase (MPO), cathepsin G, a-defensin, and azurocidin 1 mRNA. The siRNA can be designed according to methods known to those of ordinary skill in the art or purchased commercially. For example, elastase (catalog nos. sc-36042, sc-36042-PR, sc-36042-SH, sc-36042-V), MPO (catalog nos. sc-43942, sc-43942-PR, sc-43942-SH, sc-43942-V), cathepsin G (catalog nos. sc-41478, sc-41478-PR, sc-41478-SH, sc-41478-V), α-defensin (catalog nos. sc-40476, sc-40476-SH, sc-40476-V) and azurocidin 1 (catalog nos. sc-42966, sc-42966-PR, sc-42966-SH, sc-42966-V) RNAi compounds are available from Santa Cruz Biotechnology (Dallas, TX), and are amenable for use with the compositions and methods described herein.

Neutrophils also secrete a number of inflammation mediators, including at least IFN-γ, tumor necrosis factor-α (TNF-α), interleukin-17 (IL-17), interferon-γ (IFN-γ) and interferon-a (IFN-α). mRNA encoding these proteins are also amenable for targeting with the RNAi compositions and methods provided herein.

Macrophages are innate immune cells that form the first line of defense against invading pathogens. Macrophages are a type of white blood cell that engulfs and digests cellular debris, foreign substances and microbes in a phagocytic process. Human macrophages are about 21 µm in diameter and are produced by the differentiation of monocytes in tissues. Alveolar macrophages are a type of macrophages found in the pulmonary alveolus and in some embodiments, mRNAs expressed by these cells are targeted by the RNAi compounds of the present invention. For example, an RNAi compound that targets an alveolar macrophage mRNA is provided.

In addition to recognizing foreign substances, phagocytosis and the destruction of the foreign substances, macrophages are also involved in antigen presentation and secretion of a wide variety of products, including enzymes, enzyme inhibitors, cytokines, chemokines, complement components, coagulation factors, and arachidonic acid intermediates (Parameswaran and Patial. (2010). Crit. Rev. Eukaryot. Gene Expr. 20, pp. 87-103). Apart from secreting such factors, macrophages also respond to these products, thus accentuating the immune response. Macrophages contribute to the pathogenesis of various pulmonary disorders and the destructive features of macrophage-mediated inflammation are highly detrimental in the setting of the lung disease microenvironment. For example, the numbers of alveolar macrophages are markedly increased in the lungs of patients with inflammatory lung disease as a result of increased recruitment, proliferation and survival. These cells secrete inflammatory mediators, oxidants, proteins and proteinases. Targeting such secretion products and mediators of macrophage recruitment to the site of inflammation via the compositions and methods described herein may provide a therapeutic strategy for the treatment of various pulmonary disorders.

Mediators and effectors of macrophages include TNF-α, IL-12, IFN-γ, IFN-α, IL-6, IL-8, IL-8 receptors (CXCR1 and CXCR2), IL-10, IL-17, IL-1β, TGF-β, iNOS, macrophage inflammatory proteins (MIPs), and C-C chemokine receptor type 5 (CCR5). In one embodiment, which is not claimed, an mRNA encoding one of these mediators/effectors is targeted by a composition and/or method described herein. In another embodiment, which is not claimed, an mRNA encoding a receptor of TNF-α, IL-12, IFN-γ, IFN-α, IL-6, IL-8, IL-10, IL-17, IL-1β, or TGF-β may be targeted by a composition and/or method described herein.

Monocytes are a type of white blood cells produced by the bone marrow, and then circulate in the bloodstream for about one to three days. After that they typically move into tissues throughout the body. Monocytes which migrate from the bloodstream to other tissues will then differentiate into tissue resident macrophages or dendritic cells. However, those monocytes in the bloodstream are also capable of phagocytosis, antigen presentation, and cytokine production, and hence involved in some diseases. Mediators and effectors of include at least TNF-α, interleukin (IL)-12, interferon (IFN)-γ, IL-6, IL-1β, IL-17, IL-10, IL-8, IL-8 receptors (CXCR1 and CXCR2), macrophage inflammatory proteins (MIPs), and CCR5. In one embodiment, which is not claimed, an mRNA encoding one of these mediators/effectors is targeted by a composition and/or method described herein.

Dendritic cells derive from monocytes and contribute to the pathogenesis of various pulmonary disorders, such as asthma and chronic obstructive pulmonary disease (COPD). The destructive features of dendritic cell-mediated inflammation are highly detrimental in the setting of the lung disease microenvironment. Mediators and effectors of dendritic cells include at least TNF-α, IL-12, IFN-γ, IL-6, IL-8 receptors (CXCR1 and CXCR2), macrophage inflammatory proteins (MIPs), and CCR5. Each of these molecules is discussed above and the mRNA of each can be targeted with one of the RNAi compositions provided herein, for example, to treat lung injury, or a pulmonary disorder such as one of the pulmonary disorders set forth in Table 4, Table 5, Table 6 or Table 7.

Eosinophils are one of the immune system components responsible for combating multicellular parasites and certain infections in vertebrates. Along with mast cells, they also control mechanisms associated with allergy and asthma. They are also involved in a number of eosinophilic pulmonary diseases including infections, drug-induced pneumonitis, inhaled toxins, systemic disorders (*e.g.,* eosinophilic granulomatosis with polyangiitis [formerly Churg-Strauss syndrome], Loeffler's syndrome), and allergic bronchopulmonary aspergillosis. Eosinophils also contribute to tropical pulmonary eosinophilia, hypereosinophilic syndromes and some lung cancers.

Eosinophils comprise receptors of lipid mediators that include at least leukotriene B₄ receptor 1 (BLT1), leukotriene B₄ receptor 2 (BLT2), cysteinyl leukotriene receptors 1 and 2 (CysLT1 and CysLT2), and platelet-activating factor receptor (PAFR). Eosinophils comprise mediators released from granules, the mediators include at least elastase and cathepsin G. Eosinophils comprise mediators and/or effectors that are involved in chemotaxis, these include at least MIP-1α (CCL3), RANTES (CCL5), CCR5 (receptor of CCL3, 4, and 5), Eotaxin-1 (CCL11), and I1-8. Eosinophils secrete inflammation mediators that include at least TNF-α, IL-12, IL-6, IL-5, IL-13, IL-10, and TGF-β. In one embodiment, which is not claimed, an mRNA encoding one of these mediators/effectors is targeted by a composition and/or method described herein.

Mast cells contain many granules rich in histamine and heparin. Mast cells are very similar in both appearance and function to basophils. They differ in that mast cells are tissue resident, *e.g*., in mucosal tissues, while basophils are found in the blood. Mast cells can be stimulated to degranulate by direct injury, cross-linking of immunoglobulin E (IgE) receptors, or complement proteins and may mediate inflammation of various diseases. Mast cells release at least the following mediators or effectors: histidine decarboxylase (HDC), Histamine H₄ receptor, leukotriene B₄ receptor 2 (BLT 2), TNF-α, IL-1β, IL-4, IL-6, granulocyte macrophage colony stimulating factor (GM-CSF), and IL-3. In one embodiment, which is not claimed, an mRNA encoding one of these mediators/effectors is targeted by a composition and/or method described herein.

Basophils are the least common of the granulocytes, representing about 0.01% to 0.3% of circulating white blood cells. Like mast cells, basophils store histamine and release it to mediate basophilic inflammation. Basophils are particularly involved in fatal asthma. Basophils release at least the following mediators or effectors: histidine decarboxylase (HDC), histamine H₄ receptor, RANTES (CCL5), IL-4, and elastase. In one embodiment, which is not claimed, an mRNA encoding one of these mediators/effectors is targeted by a composition and/or method described herein.

Although elastase, contained in primary neutrophil granules is harnessed during inflammatory responses for example, by breaking down bacterial outer membrane protein(s) and virulence factor(s), it is also destructive. Elastase disrupts tight junctions, causes proteolytic damage to tissue, breaks down cytokines and alpha proteinase inhibitor, cleaves immunoglobulin A and G (IgA and IgG), and cleaves both C3bi, a component of the complement cascade, and CR1, a receptor on neutrophils for another complement molecule involved in phagocytosis. The cleavage of IgA, IgG, C3bi, and CR1 contributes to a decrease of the ability of neutrophils to kill bacteria by phagocytosis. Accordingly, the targeting of neutrophil release of elastase with an RNAi compound of the invention, without wishing to be bound by theory, is believed to have a beneficial effect in the treatment of the pulmonary disorders described herein. Elastase mRNA sequences are known in the art, for example, AH001514.1 (SEQ ID NO:1), NM_001972.2 (SEQ ID NO:2), Y00477.1 (SEQ ID NO:3), and NM_002087.3 (SEQ ID NO:4). Accordingly, it is within the skill of one of ordinary skill in the art to design an siRNA compound that targets one of these mRNAs. One example of a commercial RNAi compound specific for elastase mRNA is provided above.

Myeloperoxidase (MPO) is a local mediator of tissue damage when released extracellularly in chronic inflammatory diseases. MPO produces hypochlorous acid (HOCl) from hydrogen peroxide (H₂O₂) and chloride anion (Cl⁻), or the equivalent from a non-chlorine halide, during the neutrophil's respiratory burst Furthermore, it oxidizes tyroside to tyrosyl radical using hydrogen peroxide as an oxidizing agent. Hypochlorous acid and tyrosyl radical are cytotoxic, so they are used by the neutrophil to kill bacteria and other pathogens, but at the same time are destructive for the host tissues. Accordingly, the targeting of neutrophil release of MPO with an RNAi compound of the invention, without wishing to be bound by theory, is believed to have a beneficial effect in the treatment of the pulmonary disorders described herein.

Cathepsin G, a serine protease stored in primary neutrophil granules, belongs to the group of lysosomal proteinases. They participate in a broad range of functions in neutrophils including clearance of internalized pathogens, proteolytic modification of cytokines and chemokines, activation as well as shedding of cell surface receptors and apoptosis. Cathepsin G induces tissue damage and permeability changes directly in acute lung injury (ALI). Accordingly, the targeting of neutrophil release of cathepsin G with an RNAi compound of the invention, without wishing to be bound by theory, is believed to have a beneficial effect in the treatment of the pulmonary disorders described herein, including ALI.

α-defensins (1, 1B, 3, 4) can cause lung damage by disrupting the capillary-epithelial barrier. In addition, elevated levels of α-defensins are found in plasma and in BAL fluid of patients with inflammatory lung disease and reach 1 mg/mL in sputum from patients with cystic fibrosis. Accordingly, the targeting of neutrophil release of α-defensin with an RNAi compound of the invention, without wishing to be bound by theory, is believed to have a beneficial effect in the treatment of the pulmonary disorders described herein.

Azurocidin 1 is an antibiotic protein found in azurophilic granule, with monocyte chemotactic and antibacterial activity. It is also a multifunctional inflammatory mediator. As provided above, the present invention provides in one embodiment, which is not claimed, a composition comprising an RNAi compound that targets azurocidin 1 mRNA.

Within a tertiary granule of neutrophils, metalloprotease 9 (MMP9) can be found. At first, the activities of proteinases that can degrade matrix, such as matrix metalloproteinases (MMPs), might be expected to resolve the excess matrix. However, some MMPs can have pro-fibrotic functions. MMP9 is one such pro-fibrotic protease. MMP9 contributes to lung tissue injury through the degradation of extracellular matrix (ECM) components. MMP9 are involved in the breakdown of extracellular matrix (ECM) in normal physiological processes, as well as in pathological processes. MMP9 contributes to the functions of neutrophils by degrading extracellular matrix, activation of IL-1β, and cleavage of several chemokines. In one embodiment, the RNAi composition provided herein comprises an RNAi compound that targets an MMP9 mRNA. The RNAi compound can be designed by one of ordinary skill in the art, *e.g*., with the knowledge of the MMP9 mRNA sequence, and RNAi design principles. Alternatively or additionally, the RNAi compound can be purchased commercially. One example of a commercial MMP9 RNAi compound is available from Santa Cruz Biotechnology (Dallas, TX) (catalog nos.: sc-29400, sc-29400-PR, sc-29400-SH, sc-29400-V).

Other pro-fibrotic MMPs include MMP-3, MMP-7, MMP-8, MMP-9, MMP-12, and MMP-13. In one embodiment, which is not claimed, the invention provides compositions wherein the RNAi compound targets one of the aforementioned pro-fibrotic MMPs.

It is thought that neutrophil degranulation is modulated by at least β-arrestins, Hck, VAMP-7, SNAP-23, and syntaxin-4. Accordingly, in one embodiment, which is not claimed, the RNAi composition provided herein comprises an RNAi compound that targets a β-arrestin mRNA, Hck mRNA, VAMP-7 mRNA, SNAP-23 mRNA and/or syntaxin-4 mRNA.

β-arrestins are required for activating signaling pathways leading to degranulation of primary and secondary granules in neutrophils. As a group of cytosolic phosphoproteins, β-arrestins uncouple activated G protein-coupled receptors (GPCR) from their associated heterotrimeric G proteins and bind directly to the cytoplasmic tail of the CXCR1 receptor, β-arrestins also associate with the primary and secondary granules in IL-8-activated neutrophils by binding to Hck (for primary granules) and Fgr (for secondary granules), respectively. Thus, β-arrestins act at two sites in the cell during chemokine activation: one site at the receptor in the plasma membrane and a second on granule membranes. Inhibiting the expression of β-arrestin protein via an RNAi compound therefore, is thought to lead to inhibition of degranulation of primary and secondary granules in neutrophils. The β-arrestin RNAi compound can be designed by one of ordinary skill in the art, *e.g*., with the knowledge of a β-arrestin mRNA sequence, and RNAi design principles. Alternatively or additionally, the β-arrestin RNAi compound can be purchased commercially. One example of a commercial β-arrestin RNAi compound is available from Santa Cruz Biotechnology (Dallas, TX) (catalog nos. sc-29741, sc-29741-PR, sc-29741-SH, sc-29741-V).

*Homo sapiens* hemopoietic cell kinase (Hck) is a tyrosine-protein kinase that belongs to the Src family of tyrosine kinases. It plays a role in neutrophil migration and in the degranulation of neutrophils. Hck translocates to the primary granules following signaling activation and mediates the granule translocation. The Hck RNAi compound can be designed by one of ordinary skill in the art, *e.g*., with the knowledge of a Hck mRNA sequence, and RNAi design principles. Alternatively or additionally, the Hck RNAi compound can be purchased commercially. One example of a commercial Hck RNAi compound is available from Santa Cruz Biotechnology (Dallas, TX) (catalog nos. sc-35536, sc-35536-PR, sc-35536-SH, sc-35536-V).

Vesicle associated membrane protein 7 (VAMP-7) is the docking protein on the membrane of granules mediating the docking process of granules onto the plasma membrane. It is involved in all primary, secondary and tertiary granules. Inhibiting the expression of VAMP-7 protein via an RNAi compound therefore, is thought to lead to inhibition of neutrophil degranulation by inhibiting the granule docking process. The VAMP-7 RNAi compound can be designed by one of ordinary skill in the art, *e.g*., with the knowledge of a VAMP-7 mRNA sequence, and RNAi design principles. Alternatively or additionally, the VAMP-7RNAi compound can be purchased commercially. Commercial VAMP-7 RNAi compounds are available from Life Technologies (catalog nos. 139515, 139516, 139517).

Synaptosomal-associated protein 23 (SNAP-23) is the docking protein on the plasma membrane mediating the docking process of granules onto the plasma membrane, forming the complex with Syntaxin-4. It is involved in primary, secondary and tertiary neutrophil granule docking. Inhibiting the expression of SNAP-23 protein via an RNAi compound therefore, is thought to lead to inhibition of neutrophil degranulation by inhibiting the granule docking process. The SNAP-23 RNAi compound can be designed by one of ordinary skill in the art, *e.g.,* with the knowledge of a SNAP-23 mRNA sequence, and RNAi design principles. Alternatively or additionally, the SNAP-23 RNAi compound can be purchased commercially. Commercial SNAP-23 RNAi compounds are available from Santa Cruz Biotechnology (Dallas, TX) (catalog nos. sc-72219, sc-72219-PR, sc-72219-SH, sc-72219-V).

Syntaxin-4 is the docking protein on the plasma membrane mediating the docking process of granules onto the plasma membrane, forming the complex with SNAP-23. It is involved in primary, secondary and tertiary neutrophil granule docking. Inhibiting the expression of syntaxin-4 protein via an RNAi compound therefore, is thought to lead to inhibition of neutrophil degranulation by inhibiting the granule docking process. The syntaxin-4 RNAi compound can be designed by one of ordinary skill in the art, *e.g.,* with the knowledge of a syntaxin-4 mRNA sequence, and RNAi design principles. Alternatively or additionally, the Syntaxin-4 RNAi compound can be purchased commercially. Commercial Syntaxin-4 RNAi compounds are available from Santa Cruz Biotechnology (Dallas, TX) (catalog nos. sc-36590, sc-36590-PR, sc-36590-SH, sc-36590-V).

The chemotaxis of granulocytes such as neutrophils allow for the invasion and localization of granulocytes into particular tissues. In one embodiment, which is not claimed, one or more chemotactic factor mRNAs is targeted by a composition and/or method described herein.

Interleukin-8 (IL-8) receptors (*e.g.,* CXCR1 and CXCR 2) are expressed on various phagocytic cells such as neutrophils, macrophages, monocytes and dendritic cells. IL-8 is a chemoattractant that attract those innate immune cells to migrate to the local inflammation sites. IL-8 binding is thought to (i) induce chemotaxis in target cells (*e.g.,* granulocytes), causing them to migrate to the site of infection and (ii) trigger the process of granulocyte degranulation. Accordingly, in one embodiment, the RNAi composition of the invention targets an mRNA that encodes IL-8 or one of its receptors. Inhibiting the expression of IL-8 or an IL-8 receptor protein via an RNAi compound therefore, is thought to lead to inhibition of granulocyte recruitment to a site of infection as well as granulocyte degranulation. The IL-8 or IL-8 receptor RNAi compound can be designed by one of ordinary skill in the art, *e.g.,* with the knowledge of a IL-8 or IL-8 receptor mRNA sequence, and RNAi design principles. Alternatively or additionally, the IL-8 or IL-8 receptor RNAi compound can be purchased commercially. Commercial IL-8 RNAi compounds are available from Santa Cruz Biotechnology (Dallas, TX) (IL8 catalog nos.: sc-39631, sc-39631-PR, sc-39631-SH, sc-39631-V; CXCR1 catalog nos.: sc-40026, sc-40026-PR, sc-40026-SH, sc-40026- V; CXCR2 catalog nos.: sc-40028, sc-40028-PR, sc-40028-SH, sc-40028-V).

G_{β2} is one of the major G_{β} subunits expressed in neutrophils that mediate neutrophil directional cell migration and infiltration. Inhibition of neutrophil directional cell migration and infiltration with an RNAi composition is used in one embodiment, to treat one of the pulmonary disorders or lung injury described herein. The G_{β2} RNAi compound can be designed by one of ordinary skill in the art, *e.g.,* with the knowledge of a G_{β2} mRNA sequence, and RNAi design principles. Alternatively or additionally, the G_{β2} RNAi compound can be purchased commercially. Commercial G_{β2} RNAi compounds are available from Santa Cruz Biotechnology (Dallas, TX) (catalog nos.: sc-41764, sc-41764-PR, sc-41764-SH, sc-41764-V).

As provided herein, in one embodiment, the siRNA compound present in the siRNA-lipid composition targets an mRNA whose corresponding protein function is as an inflammatory mediator. The inflammatory mediator, in one embodiment, is a cytokine or a chemokine. In a further embodiment, the inflammatory mediator is a cytokine. In a further embodiment, the cytokine is tumor necrosis factor-α (TNF-α). In another embodiment, the cytokine is an interleukin. In yet another embodiment, the cytokine is a chemotactic cytokine. In yet another embodiment, a receptor for TNF-α is targeted by compositions and methods of the invention. These embodiments do not form part of the claimed invention.

IFN-γ is a pro-inflammatory cytokine that is implicated in innate and adaptive immunity against viral, some bacterial, and protozoal infections. IFN-γ has also been reported to be an activator of macrophages and to recruit monocytes and neutrophils to the site of inflammation. Aberrant IFN-γ expression is associated with a number of inflammatory and autoimmune diseases. It is released from activated neutrophils as well as T cells. In one embodiment, IFN-γ mRNA is targeted with one of the RNAi compositions described herein. In another embodiment, an IFN-γ receptor mRNA is targeted with one of the RNAi compositions described herein. The IFN-γ RNAi compound can be designed by one of ordinary skill in the art, *e.g.,* with the knowledge of an IFN-γ mRNA sequence, and RNAi design principles. Alternatively or additionally, the IFN-γ RNAi compound can be purchased commercially. Commercial IFN-γ RNAi compounds are available from Santa Cruz Biotechnology (Dallas, TX) (catalog nos. sc-39606, sc-39606-PR, sc-39606-SH, sc-39606-V).

TNF-α is pro-inflammatory cytokine involved in systemic inflammation and contributes to the acute phase of immune response. Although many cells produce TNF-α, *e.g*., neutrophils discussed above, macrophages are the major producers of TNF-α and are also highly responsive to TNF-α. Dysregulation of TNF-α production, for example, TNF-α production by macrophages is associated with a variety of human diseases. TNF-α promotes the inflammatory response and in turn causes pathogenesis associated with inflammation. Thus, in one embodiment, the present invention serves to attenuate the production of TNF-α via the RNAi pathway. In another embodiment, the present invention serves to attenuate the production or actvity of a TNF-α receptor via the RNAi pathway. These embodiments do not form part of the claimed invention.

In one embodiment, which is not claimed, TNF-α mRNA is targeted with one of the RNAi compositions described herein. The TNF-α RNAi compound can be designed by one of ordinary skill in the art, *e.g.,* with the knowledge of a TNF-α mRNA sequence, and RNAi design principles. Alternatively or additionally, the TNF-α RNAi compound can be purchased commercially. Commercial TNF-α RNAi compounds are available from Santa Cruz Biotechnology (Dallas, TX) (catalog nos.: sc-37216, sc-37216-PR, sc-37216-SH, sc-37216-V).

There are six members in the interleukin 17 (IL-17) cytokine family, including IL-17A (commonly referred to as IL-17), IL-17B, IL-17C, IL-17D, IL-17E (also known as IL-25) and IL-17F. IL-17 family members, secreted by macrophages, function as proinflammatory cytokines that responds to the invasion of the immune system by extracellular pathogens and induces destruction of the pathogen's cellular matrix. IL-17 family members have a pro-inflammatory role in asthma pathogenesis, for example allergic asthma. Overexpression of IL-17F in the airway is associated with airway neutrophilia, the induction of many cytokines, an increase in airway hyperreactivity, and mucus hypersecretion.

In one embodiment, one or more IL-17 mRNAs is targeted with one of the RNAi compositions described herein. The IL-17 RNAi compound can be designed by one of ordinary skill in the art, *e.g*., with the knowledge of a IL-17 mRNA sequence, and RNAi design principles. Alternatively or additionally, the IFN-γ RNAi compound can be purchased commercially. Commercial IL-17 RNAi compounds are available from Santa Cruz Biotechnology (Dallas, TX) (catalog nos.: sc-39649, sc-39649-PR, sc-39649-SH, sc-39649-V). In one embodiment, mRNAs encoding receptors for IL-17 family members are targeted with one of the RNAi compositions described herein. These embodiments do not form part of the claimed invention.

Interferon-α (IPN-α) is a type I interferon family produced by macrophages, dendritic cells and neutrophils. In humans, there are 13 different IFN-α genes, designated as IFN-α1, -α2, -α4, -α5, -α6, -α7, -α8, -α10, -α13, -α14, -α16, -α17 and -α21. It has been reported that alveolar macrophages are the primary IFN-α producer in pulmonary infection with RNA viruses. IFN-α can activate neutrophils and in turn increase the number of neutrophils. Abnormal IFN-α production contributes to immune dysfunction and mediates tissue inflammation and organ damage. In one embodiment, an IFN-α mRNA is targeted with one of the RNAi compounds described herein. In another embodiment, an IFN-α, receptor mRNA is targeted with one of the RNAi compounds described herein. The IFN-α RNAi compound can be designed by one of ordinary skill in the art, *e.g.,* with the knowledge of an IFN-α mRNA sequence, and RNAi design principles. Alternatively or additionally, the IFN-α RNAi compound can be purchased commercially. Commercial IFN-α RNAi compounds are available from Novus Biologicals, LLC (Littleton, CO) (IFN-α - catalog no. H00003440-R01; IFN-α6 - catalog no. H00003443-R01;. IFN-α8 - catalog no. H00003445-R01; IFN-α 13 - catalog no. H00003447-R01). These embodiments do not form part of the claimed invention.

IL-3 is a cytokine that stimulates the differentiation of multipotent hematopoietic stem cells to myeloid progenitor cells. It also stimulates proliferation of all cells in the myeloid lineage (granulocytes, monocytes, and dendritic cells); and is a regulator in humoral and adaptive immunity. IL-4 induces differentiation of naive helper T cells to Th2 cells and decreases the cytokine production of Th1 cells, macrophages (IFN-γ), and dendritic cell (IL-12). Overproduction of IL-4 is associated with allergies. IL-4 promotes M2 macrophages activation and inhibits classical activation of macrophages into M1 cells. An increase in repair macrophages (M2) is coupled with secretion of IL-10 and TGF-β that result in a diminution of pathological inflammation. Release of arginase, proline, polyaminases and TGF-β by the activated M2 cell is tied with wound repair and, in adverse case, fibrosis. IL-5 is a mediator in eosinophil activation. IL-5 has been associated with the cause of several allergic diseases including allergic rhinitis and asthma, wherein a large increase in the number of circulating, airway tissue, and induced sputum eosinophils have been observed.

The present invention in one embodiment serves to attenuate the production of IL-3, IL-4 and/or IL-5 via the RNAi pathway by providing a composition comprising an RNAi compound that targets IL-3 mRNA, IL-4 mRNA and/or IL-5 mRNA complexed to or encapsulated by a lipid component. In another embodiment, the invention provides compositions and methods targeting mRNAs encoding receptors for IL-3, IL-4, and/or IL-5. In one embodiment the composition is used to treat a patient for allergic rhinitis and/or asthma. These embodiments do not form part of the claimed invention.

The IL-3, IL-4 and/or IL-5 RNAi compound can be designed by one of ordinary skill in the art, *e.g*., with the knowledge of an IL-3, IL-4 and/or IL-5 mRNA sequence, and RNAi design principles. Alternatively or additionally, the IL-3, IL-4 and/or IL-5 RNAi compound can be purchased commercially. Commercial IL-3, IL-4 and IL-5 RNAi compounds are available from Santa Cruz Biotechnology (Dallas, TX) (IL3 catalog nos.: sc-39621, sc-39621-PR, sc-39621-SH, sc-39621-V; IL-4 catalog nos: sc-39623, sc-39623-PR, sc-39623-SH, sc-39623-V, IL-5 catalog nos: sc-39625, sc-39625-PR, sc-39625-SH, sc-39625-V).

IL-13 and IL-4 exhibit a 30% of sequence similarity and have a similar structure. IL-13 has effects on immune cells that are similar to those of the closely related cytokine IL-4. IL-13 is also a mediator of the physiologic changes induced by allergic inflammation in many tissues and fibrosis pathogenesis. In one embodiment, the present invention serves to attenuate the production of IL-13 via the RNAi pathway by providing an RNAi composition comprising a lipid component and an RNAi compound, wherein the RNAi compound targets an IL-13 mRNA. The IL-13 RNAi compound can be designed by one of ordinary skill in the art, *e.g.,* with the knowledge of the IL-13 mRNA sequence, and RNAi design principles known to those of ordinary skill in the art and exemplified herein. Alternatively or additionally, the IL-13 RNAi compound can be purchased commercially. Commercial IL-13 RNAi compounds are available from OriGene (Rockville, MD) (catalog no. TR312195). In another embodiment, the invention provides compositions and methods that target an IL-13 mRNA receptor. These embodiments do not form part of the claimed invention.

IL-6 is a pro-inflammatory cytokine secreted by T cells and macrophages to stimulate immune response such as infection and post-trauma, especially bums or other tissue damage leading to inflammation. IL-6 stimulates the inflammatory and auto-immune processes in many diseases. IL-6 can also contribute to the activation signal of IL-17 production by T cells. IL-12 is a pro-inflammatory cytokine produced by phagocytes such as macrophages and dendritic cells, and directs the signal for the differentiation of naive T cells into Th1 cells. It stimulates the production of interferon-gamma (IFN-γ) and tumor necrosis factor-alpha (TNF-α) from T cells and natural killer (NK) cells, and reduces IL-4 mediated suppression of IFN-γ expression. Interlcukin-1β (IL-1β) is a pro-inflammatory cytokine produced by activated macrophages. It increases the expression of adhesion factors on endothelial cells resulting in neutrophil extravasation. IL-1β also leads to induction of cyclooxygenase type 2 and synthesis of nitric oxide. IL-8 is a chemoattractant that attract innate immune cells to migrate to the local inflammation sites, and when they approach the environment, IL-8 in turn triggers the signaling of degranulation process of neutrophils. These functions are conducted through binding of IL-8 to IL-8 receptors --- CXCR1 and CXCR2 on the membrane surface of the cells. IL-10 is an anti-inflammatory cytokine produced by M2 macrophage and some types of T cells. It has functions with multiple, pleiotropic, effects in immunoregulation and inflammation, and is capable of inhibiting synthesis of pro-inflammatory Th1 cytokines. However, it is also stimulatory towards Th2 cells and mast cells, the overstimulation of which may lead to diseases such as fibrosis. In one embodiment, the present invention serves to attenuate the production of these cytokines via the RNAi pathway by providing RNAi compositions comprising a lipid component and an RNAi compound, where the RNAi compound targets one of the aforementioned interleukin mRNAs. In a further embodiment, the interleukin mRNA is IL-6, IL-8, IL-10, IL-12, or IL-1β mRNA. In another embodiment, an mRNA encoding a receptor for IL-6, IL-8, IL-10, IL-12, or IL-1β is targeted by the compositions and methods of the invention. As with the other RNAi compounds described herein, these can be designed by one of ordinary skill in the art, *e.g*., with the knowledge of the respective cytokine mRNA sequence, and RNAi design principles known to those of ordinary skill in the art and exemplified herein. Alternatively or additionally, the cytokine RNAi compound can be purchased commercially. Commercial cytokine RNAi compounds are available from Santa Cruz Biotechnology (Dallas, TX) (IL-6 catalog nos.: sc-39627, sc-39627-PR, sc-39627-SH, sc-39627-V; IL-12 catalog nos.: sc-39640, sc-39640-PR, sc-39640-SH, sc-39640-V; IL-1β: sc-39615, sc-39615-PR, sc-39615-SH, sc-39615-V; IL-8: sc-39631, sc-39631-PR, sc-39631-SH, sc-39631-V; IL-10: sc-39635, sc-39635-PR, sc-39635-SH, sc-39635-V). These embodiments do not form part of the claimed invention.

TGF-β is a multifunctional protein that regulates cell proliferation, differentiation, apoptosis, cell cycle, embryogenesis, development, wound healing, tissue repair, angiogenesis, and tumor development. TGF-β₁ has been implicated as one of the key cytokines in the induction of fibrosis in many organs, including the lung (Lai et al. (2009). J. Environ. Pathol. Toxicol. Oncol. 28, pp. 109-119). Embodiments described herein encompass the use of a TGF-β RNAi compound or a TGF-β receptor RNAi compound in one or more of the compositions and methods described herein, *e.g*., for the treatment of a pulmonary disorder such as pulmonary fibrosis or interstitial lung disease (ILD). The TGF-β RNAi compound can be designed by one of ordinary skill in the art, *e.g.,* with the knowledge of a TGF-β mRNA sequence, and RNAi design principles. Alternatively or additionally, the TGF-β RNAi compound can be purchased commercially. Commercial TGF-β RNAi compounds are available from Santa Cruz Biotechnology (Dallas, TX) (catalog nos.: sc-270322, sc-270322-PR, sc-270322-SH, sc-270322-V). These embodiments do not form part of the claimed invention.

C-C chemokine receptor type 5 (CCR5) is a chemotaxis receptor that can bind to RANTES (a chemotactic cytokine protein also known as CCL5) and macrophage inflammatory protein (MIP) 1α and 1β (also known as CCL3 and CCL4, respectively) and has been reported to mediate inflammation. Accordingly, compositions and methods provided herein are useful for targeting CCR5 mRNA via the RNAi pathway. The CCR5 RNAi compound can be designed by one of ordinary skill in the art, *e.g*., with the knowledge of a CCR5 mRNA sequence, and RNAi design principles. Alternatively or additionally, the CCR5 RNAi compound can be purchased commercially. Commercial CCR5 RNAi compounds are available from Santa Cruz Biotechnology (Dallas, TX) (catalog nos.: sc-35062, sc-35062-PR, sc-35062-SH, sc-35062-V).

RANTES (CCL5) is chemotactic for T cells, eosinophils, and basophils and recruits them into inflammatory sites. With the help of particular cytokines (e.g., IL-2 and IFN=γ) that are released by T cells, CCL5 also induces the proliferation and activation of certain natural-killer (NK) cells to form CHAK (CC-Chemokine-activated killer) cells. RANTES has been shown to be in the respiratory secretions of asthmatics (Culley et al. (2006). J. Virol. 80, pp. 8151-8157). RANTES has also been reported to play a role in acute lung allograft rejection. Accordingly, in one embodiment, the present invention provides an RNAi compound that targets RANTES mRNA. In a further embodiment, the RNAi composition is used to treat a patient that has undergone a lung transplant or an asthma patient. Targeting RANTES with one of the RNAi compositions provided herein in another embodiment, is used for the treatment of one of the pulmonary disorders set forth in Table 4, Table 5, Table 6 and/or Table 7. The RANTES RNAi compound can be designed by one of ordinary skill in the art, *e.g.,* with the knowledge of a RANTES mRNA sequence, and RNAi design principles. Alternatively or additionally, the RANTES RNAi compound can be purchased commercially. Commercial RANTES RNAi compounds are available from Santa Cruz Biotechnology (Dallas, TX) (catalog nos.: sc-44066, sc-44066-PR, sc-44066-SH, sc-44066-V). These embodiments do not form part of the claimed invention.

Eotaxin (also designated eotaxin-1 or CCL11) is a member of the C-C or β family of chemokines which is characterized by a pair of adjacent cysteine residues. Eotaxin-1 binds to CCR2, CCR3 and CCR5. However, it has been found that eotaxin-1 has high degree selectivity for its receptor, such that they are inactive on neutrophils and monocytes, which do not express CCR3. The human eotaxin receptor, CCR3, is expressed on eosinophils, basophils, and TH2 cells. Eotaxin-1 is a chemoattractant that selectively recruits eosinophils, and therefore, is involved in allergic responses. Its presence in the serum of COPD patients has also been demonstrated (Janz-Rozyk et al. (2000). Mediators of Inflammation 9, pp. 175-179). Thus, eotaxin mRNA in one embodiment, is targeted by the RNAi composition provided herein for the treatment of a pulmonary disorder, *e.g*., a pulmonary disorder set forth in Table 4, Table 5, Table 6 or Table 7. In another embodiment, the eotaxin RNAi composition is used to treat a COPD patient or an asthma patient. The Eotaxin-1 RNAi compound can be designed by one of ordinary skill in the art, *e.g.,* with the knowledge of a Eotaxin-1 mRNA sequence, and RNAi design principles. Alternatively or additionally, the RANTES RNAi compound can be purchased commercially. Commercial Eotaxin-1 RNAi compounds are available from Santa Cruz Biotechnology (Dallas, TX) (catalog nos.: sc-43753, sc-43753-PR, sc-43753-SH, sc-43753-V). These embodiments do not form part of the claimed invention.

Macrophage inflammatory proteins 1α and 1β (MIP-1α and -1β) and macrophage inflammatory protein 2 (MIP-2) are approximately 6-8 kd, heparin binding proteins that exhibit a number of inflammatory and immunoregulatory activities, and belong to the family of chemotactic cytokines (Driscoll (1994). Exp. Lung Res. 20, pp. 473-490). They activate human granulocytes (neutrophils, eosinophils and basophils) which can lead to acute inflammation. Increased MIP expression has been observed in models of bacterial sepsis, silicosis, and oxidant-induced lung injury. Studies in humans indicate MIP-1α contributes to the inflammatory cell response associated with sarcoidosis and idiopathic pulmonary fibrosis (Driscoll (1994). Exp. Lung Res. 20, pp. 473-490).

In one embodiment, the present invention provides an RNAi composition that includes an RNAi compound that targets MIP-1α, MIP-1β or MIP-2 mRNA, for example, for the treatment of a pulmonary disorder associated with an inflammatory cell response such as sarcoidosis or idiopathic pulmonary fibrosis. In another embodiment, the present invention provides an RNAi composition that includes an RNAi compound that targets MIP-1α, MIP-1β or MIP-2, for example, for the treatment of bacterial lung sepsis, silicosis, or lung injury, *e.g*., oxidant induced lung injury. The MIP RNAi compound can be designed by one of ordinary skill in the art, *e.g.,* with the knowledge of a MIP mRNA sequence, and RNAi design principles. Alternatively or additionally, the MIP RNAi compound can be purchased commercially. Commercial MIP RNAi compounds are available from Santa Cruz Biotechnology (Dallas, TX) (MIP-1α catalog nos.: sc-43933, sc-43933-PR, sc-43933-SH, sc-43933-V; MIP-1β catalog nos.: sc-43932, sc-43932-PR, sc-43932-SH, sc-43932-V). These embodiments do not form part of the claimed invention.

Granulocyte-macrophage stimulating factor (GM-CSF) functions as a cytokine of white blood cell growth factor. GM-CSF stimulates stem cells to produce granulocytes (neutrophils, eosinophils, and basophils) and monocytes. GM-CSF is found in high levels in some inflammation sites and blocking GM-CSF expression may reduce the inflammation or damage. The present invention in one embodiment targets GM-CSF mRNA in the lung by providing an RNAi composition comprising a lipid component and an RNAi compound that targets GM-CSF mRNA as well as methods for treating a patient via pulmonary delivery of the RNA composition. The GM-CSF RNAi compounds can be designed by one of ordinary skill in the art, *e.g.,* with the knowledge of the respective GM-CSF mRNA sequence, and RNAi design principles known to those of ordinary skill in the art and exemplified herein. Alternatively or additionally, the GM-CSF RNAi compound can be purchased commercially. Commercial GM-CSF RNAi compounds are available from Santa Cruz Biotechnology (Dallas, TX) (catalog nos.: sc-39391, sc-39391-PR, sc-39391-SH, sc-39391-V). Also provided herein are compositions that target the GM-CSF receptor (*see* Santa Cruz Biotechnology catalog nos.: sc-35501, sc-35501-PR, sc-35501-SH, sc-35501-V for exemplary siRNA compound that can be used in the methods and compositions provided herein). These embodiments do not form part of the claimed invention.

Inducible nitric oxide synthase (iNOS) is the inducible isoform of nitric oxide synthase expressed in macrophage. It catalyzes the production of nitric oxide (NO) from L-arginine; and produces large amounts of NO as a defense mechanism. iNOS expression has been reported diseases with an autoimmune etiology. Disturbed regulation of NO release is associated with the pathophysiology of almost all inflammatory diseases (Hesslinger et al. (2009). Biochem Soc. Trans. 37 (Pt 4), pp. 886-89). The present invention in one embodiment, targets iNOS mRNA with an RNAi composition, in order to inhibit its inflammatory effect in various pulmonary disorders. The iNOS RNAi compound can be designed by one of ordinary skill in the art, *e.g*., with the knowledge of an iNOS mRNA sequence, and RNAi design principles. Alternatively or additionally, the iNOS RNAi compound can be purchased commercially. Commercial iNOS RNAi compounds are available from OriGene (Rockville, MD) (catalog no. TG302918). These embodiments do not form part of the claimed invention.

Histidine decarboxylase (HDC) is the enzyme that catalyzes the reaction that produces histamine from histidine (using the cofactor vitamin B6). Histamine is released by basophils and mast cells and is involved in the inflammatory response. Without wishing to be bound by theory, it is thought that histamine may be involved in immune system disorders and allergies. For example, mastocytosis is a rare disease in which there is a proliferation of mast cells that produce excess histamine. Accordingly, the present invention relates in one embodiment to an RNAi composition comprising a lipid component and a HDC RNAi compound for the treatment of mastocytosis, asthma and/or other pulmonary disorders (e.g., one of the pulmonary disorders set forth in Table 4, Table 5, Table 6 or Table 7). The HDC RNAi compound can be designed by one of ordinary skill in the art, *e.g.,* with the knowledge of a HDC mRNA sequence, and RNAi design principles. Alternatively or additionally, the HDC RNAi compound can be purchased commercially. Commercial HDC RNAi compounds are available from Santa Cruz Biotechnology (Dallas, TX) (catalog nos.: sc-45375, sc-45375-PR, sc-45375-SH sc-45375-V). These embodiments do not form part of the claimed invention.

Histamine H4 receptor responds to histamine released from either basophils or mast cells, and is involved in mediating eosinophil shape change and chemotaxis of basophils and mast cells. The present invention relates in one embodiment to an RNAi composition comprising a lipid component and a histamine H4 receptor RNAi compound for the treatment of a disorder associated with aberrant histamine H4 receptor expression. For example, in one embodiment, the pulmonary disorder is one of the pulmonary disorders set forth in Table 4, Table 5, Table 6 or Table 7. The histamine H4 receptor RNAi compound can be designed by one of ordinary skill in the art, *e.g.,* with the knowledge of a histamine H4 receptor mRNA sequence, and RNAi design principles. Alternatively or additionally, the histamine H4 receptor RNAi compound can be purchased commercially. Commercial histamine H4 receptor RNAi compounds are available from Santa Cruz Biotechnology (Dallas, TX) (catalog nos.: sc-40025, sc-40025-PR, sc-40025-SH, sc-40025-V). These embodiments do not form part of the claimed invention.

The cysteinyl leukotrienes (cys-LTs, *e.g.,* LTC4, LTD4, and LTE4) are a family of potent bioactive lipids that act through two structurally divergent G protein-coupled receptors, termed the CysLT₁ and CysLT₂ receptors. Cysteinyl leukotrienes (CysLTs) contribute to the development of airway obstruction and inflammation in asthma (Fullmer et al. (2005). Pediatr. Allergy Immunol. 16, pp. 593-601. These embodiments do not form part of the claimed invention. Accordingly, the present invention relates in one embodiment to an RNAi composition comprising a lipid component and a CysLT₁ and/or CysLT₂ RNAi compound for the treatment of lung inflammation, asthma and/or other pulmonary disorder (*e.g.,* one of the pulmonary disorders set forth in Table 3, Table 4, Table 5 or Table 6). The CysLT₁ and/or CysLT₂ RNAi compound can be designed by one of ordinary skill in the art, *e.g.*, with the knowledge of a CysLT₁ and/or CysLT₂ mRNA sequence, and RNAi design principles. Alternatively or additionally, the CysLT₁ and/or CysLT₂ RNAi compound can be purchased commercially. CysLT₁ and/or CysLT₂ RNAi compounds are available from Santa Cruz Biotechnology (Dallas, TX) (CysLT₁ catalog nos.: sc-43712, sc-43712-PR, sc-43712-SH, sc-43712-V; CysLT₂ catalog nos.: sc-43713, sc-43713-PR, sc-43713-SH, sc-43713-V). These embodiments do not form part of the claimed invention.

Platelet-activating factor (PAF) is phospholipid inflammatory mediator involved in lung inflammation. For example, it has been shown that increased levels of PAF are present in patients with acute lung injury (ALI). The PAF receptor is denoted platelet-activating factor receptor (PAFR). Embodiments herein are directed to PAFR RNAi compositions, *e.g*., for the treatment of pulmonary disorders. In one embodiment, the pulmonary disorder is associated with inflammation. In another embodiment, the pulmonary disorder is acute lung injury. In yet another embodiment, the pulmonary disorder is one of the pulmonary disorders set forth in Table 4, Table 5, Table 6 or Table 7. The PAFR RNAi compound can be designed by one of ordinary skill in the art, *e.g.*, with the knowledge of a PAFR mRNA sequence, and RNAi design principles. Alternatively or additionally, the PAFR RNAi compound can be purchased commercially. Commercial PAFR RNAi compounds are available from Santa Cruz Biotechnology (Dallas, TX) (catalog nos.: sc-40165, sc-40165-PR, sc-40165-SH, sc-40165-V). These embodiments do not form part of the claimed invention.

Lipid mediators are a class of bioactive lipids that are produced locally through specific biosynthetic pathways in response to extracellular stimuli. This class of compounds contributes to many physiological processes, and their dysregulation is associated with various diseases, especially inflammation. Leukotrienes are a type of lipid mediators and are involved in asthmatic and allergic reactions and act to sustain inflammatory reactions. The present invention in one embodiment encompasses an RNAi composition comprising an RNAi compound that targets a lipid mediator receptor mRNA present on a granulocyte, and in particular, a neutrophil. These embodiments do not form part of the claimed invention.

Neutrophils comprise lipid mediator receptors, which comprise at least the leukotriene B₄ receptor 1 (BLT1) and leukotriene B₄ receptor 2 (BLT2). Accordingly, the present invention in one embodiment encompasses an RNAi composition comprising an RNAi compound that targets BLT1 mRNA or BLT2 mRNA. BLT1 binds to the lipid mediator called Leukotriene B₄ and leads to its downstream signals in neutrophil functions. BLT2 binds to the lipid mediator Leukotriene B₄ and leads to its downstream signals in neutrophil functions. In one embodiment, which is not claimed, BLT1 or BLT2 mRNA is targeted with one of the RNAi compositions described herein. The BLT1 or BLT2 RNAi compound can be designed by one of ordinary skill in the art, *e.g*., with the knowledge of a BLT1 or BLT2 mRNA sequence, and RNAi design principles. Alternatively or additionally, the BLT1 and/or BLT2 RNAi compound can be purchased commercially. BLT1 and BLT2 RNAi compounds have been published, see for example, Hirata et al. (2013). Lipids Health Dis. 12, p. 122. The Hirata sequences, which are amenable for use herein, are as follows:
- BLT1:
   5'-CAACCUACACUUCCUAUUA-3'(sense) (SEQ ID NO: 5) and
   5'-UAAUAGGAAGUGUAGGUUG-3' (antisense) (SEQ ID NO: 6).
- BLT2:
   5'-GGGACUUAACAUACUCLUUA-3' (sense) (SEQ ID NO: 7) and
   5'-UAAGAGUAUGUUAAGUCCG-3' (antisense) (SEQ ID NO: 8).

Proteinase 3 (PR3) is a serine protease produced by neutrophils and in one embodiment; which is not claimed, PR3 mRNA is targeted by an RNAi composition of the invention. PR3 converts or activate many inflammatory molecules, such as IL-8, IL-32, IL1-β, TNF-α. It is also one of the antigens recognized by anti-neutrophil cytoplasmic antibodies (ANCAs) found in the disease granulomatosis with polyangiitis (formerly "Wegener's granulomatosis") which involves lung damage. The PR3 RNAi compound can be designed by one of ordinary skill in the art, *e.g*., with the knowledge of a PR3 mRNA sequence, and RNAi design principles. Alternatively or additionally, the PR3 RNAi compound can be purchased commercially. Commercial PR3 RNAi compounds are available from Santa Cruz Biotechnology (Dallas, TX) (catalog nos. sc-42968, sc-42968-PR, sc-42968-SH, sc-42968-V).

Vascular endothelial growth factor (VEGF) is a multifunctional cytokine that has been shown to mediate endothelial cell alterations during inflammation, neovascularization and angiogenesis. Research has shown that neutrophil-derived VEGF may regulate vascular responses during acute and chronic inflammation. In one embodiment, a VEGF receptor (VEGFR) (e.g., VEGFR-1 (Flt-1) or VEGFR-2 (Flk-1)) mRNA is targeted by an RNAi composition of the invention. Without wishing to be bound by theory, it is thought that the attenuation or elimination of VEGF binding to its receptor at the site of lung inflammation via the RNAi pathway is an effective means of treating inflammatory pulmonary disorders. Indeed, serum concentration of VEGF is high in bronchial asthma, indicating the involvement of VEGF in asthmatic inflammation. The VEGFR RNAi compound can be designed by one of ordinary skill in the art, *e.g*., with the knowledge of a VEGFR mRNA sequence, and RNAi design principles. Alternatively or additionally, the VEGFR RNAi compound can be purchased commercially. Commercial Flt-1 and Flk-1 RNAi compounds are available from Santa Cruz Biotechnology (Dallas, TX) (Flt-1: catalog nos. sc-29319, sc-29319-PR, sc-29319-SH, sc-29319-V; Flk-1: catalog nos. sc-29318, sc-29318-PR, sc-29318-SH, sc-29318-V). These embodiments do not form part of the claimed invention.

A summary of some of the mRNAs amenable for targeting with the RNAi compositions provided herein is provided in Table 1. According to the invention, the RNAi compound is targeting an mRNA that encodes COL1A1 or annexin A11.

| **Table 1. mRNA targets of the RNAi compositions of the invention according to one embodiment.** | | | | | | |
|---|---|---|---|---|---|---|
| **Neutrophil targets** | **Eosinophil targets** | **Basophil targets** | **Mast cell targets** | **Macrophage targets** | **Monocyte targets** | **Dendritic cell targets** |
| Myeloperoxidase | Elastase | HDC | HDC | TNF-α | TNF-α | TNF-α |
| Cathepsin G | Cathepsin G | Histamine H₄ receptor | Histamine H₄ receptor | IFN-γ | IL-12 | IL-12 |
| α-Defensins | MIP-1α (CCL3) or its receptor | RANTES (CCL5) or its receptor | BLT 2 | IFN-α or its receptor | IFN-γ or its receptor | IFN-γ or its receptor |
| Azurocidin 1 | RANTES (CCL5) or its receptor | IL-4 or its receptor | TNF-α or its receptor | IL-6 or its receptor | IL-6 or its receptor | IL-6 or its receptor |
| MMP9 | CCR5 | Elastase | IL-1β or its receptor | IL-1β or its receptor | IL-1β or its receptor | IL-8 receptors |
| β-arrestins | Eotaxin-1 (CCL11) or its receptor | | IL-4 or its receptor | IL-17 or its receptor | IL-17 or its receptor | MIP or its receptor |
| Hck | IL-8 (CXCL8) or its receptor | | IL-6 or its receptor | iNOS | IL-10 or its receptor | CCR5 |
| VAMP-7 | BLT1, 2 | | GM-CSF or its receptor | IL-8 | IL-8 | |
| SNAP-23 | CysLT1, 2 | | IL-3 or its receptor | IL-8 receptors | IL-8 receptors | |
| Syntaxin-4 | P AFR | | | IL-10 or its receptor | MIPs or their receptors | |
| Elastase | IL-12 or its receptor | | | MIPs or their receptors | CCR5 | |
| IL-8 receptors | IL-6 or its receptor | | | CCR5 | | |
| Gβ2 | IL-4 or its receptor | | | TGF-β or its receptor | | |
| IFN-γ or its receptor | IL-5 or its receptor | | | | | |
| TNF-α or its receptor | IL-13 or its receptor | | | | | |
| IL-17 or its receptor | IL-10 or its receptor | | | | | |
| IFN-α or its receptor | TGF-β or its receptor | | | | | |
| BLT1 | TNF-α or its receptor | | | | | |
| BLT2 | | | | | | |
| PR3 | | | | | | |
| VEGF receptors | | | | | | |

As described above, an mRNA sequence of a target mRNA described herein is useful for designing an RNAi compound of the invention. Reference human mRNA sequences for certain targets described herein are provided in Table 2 below. Although the human mRNA reference sequence numbers are provided herein, the invention also encompasses compositions that target non-human mRNA. RNAi compounds which target mRNA encoding COL1A1 or annexin A11 are encompassed by the claimed invention.

| **Table 2. Reference human mRNA sequences.** | | |
|---|---|---|
| **Target** | **mRNA Reference Sequence (Human (Homo sapiens))** | **Variant (if any)** |
| MMP9 | NM_004994.2 | |
| β-arrestin 1 | NM_004041.4 | arrestin, beta 1 (ARRB1), transcript variant 1, mRN A |
| | NM_020251.3 | arrestin, beta 1 (ARRB1), transcript variant 2, mRNA |
| β-arrestin 2 | NM_004313.3 | arrestin, beta 2 (ARRB2), transcript valiant 1, mRNA |
| | NM_199004.1 | arrestin, beta 2 (ARRB2), transcript variant 2, mRNA |
| | NM_001257328.1 | arrestin, beta 2 (ARRB2), transcript variant 3, mRNA |
| | NM_001257329.1 | arrestin, beta 2 (ARRB2), transcript variant 4, mRNA |
| | NM_001257330.1 | arrestin, beta 2 (ARRB2), transcript variant 5, mRNA |
| | NM_001257331.1 | arrestin, beta 2 (ARRB2), transcript variant 6, mRNA |
| Hck | NM_001172129.1 or NM_002110.3 | Homo sapiens HCK proto-oncogene, Src family tyrosine kinase (HCK), transcript variant 1, mRNA |
| | NM_001172130.1 or NM_001172131.1 | Homo sapiens HCK proto-oncogene, Src family tyrosine kinase (HCK), transcript variant 2, mRNA |
| | NM_001172132.1 | Homo sapiens HCK proto-oncogene, Src family tyrosine kinase (HCK), transcript variant 3, mRNA |
| | NM_001172133.1 | Homo sapiens HCK proto-oncogene, Src family tyrosine kinase (HCK), transcript variant 4, mRNA |
| VAMP-7 | NM_005638.5 | Homo sapiens vesicle-associated membrane protein 7 (VAMP7), transcript variant 1, mRNA |
| | NM_001145149.2 | Homo sapiens vesicle-associated membrane protein 7 (VAMP7), transcript variant 2, mRNA |
| | NM_001185183.1 | Homo sapiens vesicle-associated membrane protein 7 (VAMP7), transcript variant 3, mRNA |
| | XM_011531188.1 or XM_011545653.1 | Homo sapiens vesicle-associated membrane protein 7 (VAMP7), transcript variant X1, mRNA |
| SNAP-23 | NM_003825.3 | Homo sapiens synaptosomal-associated protein, 23kDa (SNAP23), transcript variant 1, mRNA |
| | NM_130798.2 | Homo sapiens synaptosomal-associated protein, 23kDa |
| | | (SNAP23), transcript variant 2, mRNA |
| syntaxin-4 | NM_001272095.1 | Homo sapiens syntaxin 4 (STX4), transcript variant 1, mRNA |
| | NM_001272096.1 | Homo sapiens syntaxin 4 (STX4), transcript variant 2, mRNA |
| | NM_004604.4 | Homo sapiens syntaxin 4 (STX4), transcript variant 3, mRNA |
| IL-8 (CXCL8) | NM_000584.3 | |
| IL-8 receptor alpha | AK312668.1 | |
| IL-8 receptor beta | AK312664.1 | |
| G_{β2} | NM_005273.3 | (Homo sapiens guanine nucleotide binding protein (G protein), beta polypeptide 2 (GNB2), mRNA) |
| IFN-γ | NM_000619.2 | |
| TNF-α | NM_000594.3 | |
| IL-17 | NM_002190.2 | |
| IFN-α 1 | NM_024013.2 | |
| IFN-α 2 | NM_000605.3 | |
| IFN-α 4 | NM_021068.2 | |
| IFN-α 7 | NM_021057.2 | |
| IFN-α 10 | NM_002171.2 | |
| IFN-α 13 | X75934.1 | |
| IFN-α 17 | NM_021268.2 | |
| IL-3 | NM_000588.3 | |
| IL-4 | NM_000589.3 | Homo sapiens interleukin 4 (IL4), transcript variant 1, mRNA |
| | NM_172348.2 | Homo sapiens interleukin 4 (IL4), transcript variant 2, mRNA |
| IL-5 | NM_000879.2 | |
| | XM_006714601.2 | Homo sapiens interleukin 5 (IL5), transcript variant X1, mRNA |
| | XM_005271988.2 | Homo sapiens interleukin 5 (IL5), transcript variant X2, mRNA |
| | XM_011543373.1 | Homo sapiens interleukin 5 (IL5), transcript variant X3, mRNA |
| | XM_011543374.1 | Homo sapiens interleukin 5 (IL5), transcript variant X4, mRNA |
| | XM_011543375.1 | Homo sapiens interleukin 5 (IL5), transcript variant X5, mRNA |
| IL-13 | NM_002188.2 | |
| IL-6 | NM_000600.3 | |
| IL-8 (CXCL8) | NM_000584.3 | |
| IL-10 | NM_000572.2 | |
| IL-12 *(consists of IL-12A subunit & IL-12B subunit below)* | | |
| IL-12A (p35) subunit | NM_000882.3 | |
| IL-12B (p40) subunit | NM_002187.2 | |
| IL-1β | NM_000576.2 | |
| CCR5 | NM_000579.3 | Homo sapiens chemokine (C-C motif) receptor 5 (gene/pseudogene) (CCR5), transcript variant A, mRNA |
| | NM_001100168.1 | Homo sapiens chemokine (C-C motif) receptor 5 (gene/pseudogene) (CCR5), transcript variant B, mRNA |
| RANTES (CCL5) | NM_002985.2 | Homo sapiens chemokine (C-C motif) ligand 5 (CCL5), transcript variant 1, mRNA |
| | NM_001278736.1 | Homo sapiens chemokine (C-C motif) ligand 5 (CCL5), transcript variant 2, mRNA |
| Eotaxin-1 (CCL11) | NM_002986.2 | |
| MIP-1α, (CCL3) | NM_002983.2 | |
| MIP-1β (CCL4) | NM_002984.3 | |
| MIP-2α (CXCL2) | NM_002089.3 | |
| MIP-2γ (CXCL14) | NM_004887.4 | |
| GM-CSF (CSF2) | NM_000758.3 | |
| iNOS | NM_000625.4 | |
| HDC | NM_001306146.1 | Homo sapiens histidine decarboxylase (HDC), transcript variant 1, mRNA |
| | NM_002112.3 | Homo sapiens histidine decarboxylase (HDC), transcript variant 2, mRNA |
| histamine H4 receptor | NM_021624.3 | Homo sapiens histamine receptor H4 (HRH4), transcript variant 1, mRNA |
| | NM_001143828.1 | Homo sapiens histamine receptor H4 (HRH4), transcript variant 2, mRNA |
| | NM_001160166.1 | Homo sapiens histamine receptor H4 (HRH4), transcript variant 3, mRNA |
| CysLT₁ | NM_001282187.1 | Homo sapiens cysteinyl leukotriene receptor 1 (CYSLTR1), transcript variant 1, mRNA |
| | NM_001282186.1 | Homo sapiens cysteinyl leukotriene receptor 1 (CYSLTR1), transcript variant 2, mRNA |
| | NM_006639.3 | Homo sapiens cysteinyl leukotriene receptor 1 (CYSLTR1), transcript variant 3, mRNA |
| | NM_001282188.1 | Homo sapiens cysteinyl leukotriene receptor 1 (CYSLTR1), transcript variant 4, mRNA |
| CysLT₂ | NM_001308465.1 | Homo sapiens cysteinyl leukotriene receptor 2 (CYSLTR2), transcript variant I, mRNA |
| | NM_001308467.1 | Homo sapiens cysteinyl leukotriene receptor 2 (CYSLTR2), transcript variant II, mRNA |
| | NM_001308468.1 | Homo sapiens cysteinyl leukotriene receptor 2 (CYSLTR2), transcript variant III, mRNA |
| | NM_001308469.1 | Homo sapiens cysteinyl leukotriene receptor 2 (CYSLTR2), transcript variant IV, mRNA |
| | NM_001308476.1 | Homo sapiens cysteinyl leukotriene receptor 2 (CYSLTR2), transcript variant V, mRNA |
| | NM_020377.3 | Homo sapiens cysteinyl leukotriene receptor 2 (CYSLTR2), transcript variant VI, mRNA |
| | NM_001308470.1 | Homo sapiens cysteinyl leukotriene receptor 2 (CYSLTR2), transcript variant VII, mRNA |
| PAFR | NM_001164721.1 | Homo sapiens platelet-activating factor receptor (PTAFR), transcript variant 1, mRNA |
| | NM_001164722.2 | Homo sapiens platelet-activating factor receptor (PTAFR), transcript variant 2, mRNA |
| | NM_000952.4 | Homo sapiens platelet-activating factor receptor (PTAFR), transcript variant 3, mRNA |
| | NM_001164723.2 | Homo sapiens platelet-activating factor receptor (PTAFR), transcript variant 4, mRNA |
| BLT1 | NM_181657.3 | Homo sapiens leukotriene B4 receptor (LTB4R), transcript variant 1, mRNA |
| | NM_001143919.2 | Homo sapiens leukotriene B4 receptor (LTB4R), transcript variant 2, mRNA |
| BLT2 | NM_019839.4 | Homo sapiens leukotriene B4 receptor 2 (LTB4R2), transcript variant 1, mRNA |
| | NM_001164692.2 | Homo sapiens leukotriene B4 receptor 2 (LTB4R2), transcript variant 2, mRNA |
| Proteinase 3 (PR3) | NM_002777.3 | |
| VEGFR-1 (Flt-1, VEGF receptor-1) | NM_002019.4 | Homo sapiens fms-related tyrosine kinase 1 (FLT1), transcript variant 1, mRNA |
| | NM_001159920.1 | Homo sapiens fms-related tyrosine kinase 1 (FLT1), transcript variant 2, mRNA |
| | NM_001160030.1 | Homo sapiens fms-related tyrosine kinase 1 (FLT1), transcript variant 3, mRNA |
| | NM_001160031.1 | Homo sapiens fms-related tyrosine kinase 1 (FLT1), transcript variant 4, mRNA |
| VEGFR-2 (Flk-1, KDR) | NM_002253.2 | |
| VEGFR-3 (Flt-4) | NM_182925.4 | Homo sapiens fms-related tyrosine kinase 4 (FLT4), transcript variant 1, mRNA |
| | NM_002020.4 | Homo sapiens fms-related tyrosine kinase 4 (FLT4), transcript variant 2, mRNA |

Treatment methods: pulmonary fibrosis or sarcoidosis are the two diseases for which the compositions of the invention are claimed to be for use of.

In one aspect of the invention, a method for treating a pulmonary disease or disorder is provided.

In one embodiment, the method for treating a pulmonary disorder comprises administering to the lungs of a patient in need thereof, one or more of the compositions described herein. The pulmonary disorder, in one embodiment, is one of the pulmonary disorders set forth in Table 4, Table 5, Table 6, Table 7, or a combination thereof.

In certain embodiments, the invention provides a method for treating pulmonary fibrosis comprising administering to the lungs of a patient in need thereof, one or more compositions of the invention. In exemplary embodiments, a method for treating pulmonary fibrosis comprises administering to the lungs of a patient in need thereof, a composition according to the invention comprising a RNAi compound complexed to or encapsulated by a lipid particle, wherein the RNAi compound targets a mRNA involved in collagen synthesis (e.g. COL1A1, P4HA1, etc.) or a cytokine production (e.g. TNFα, TGFβ, etc.).

In certain embodiments, the invention provides a method for treating sarcoidosis comprising administering to the lungs of a patient in need thereof, one or more compositions of the invention. In exemplary embodiments, a method for treating sarcoidosis comprises administering to the lungs of a patient in need thereof, a composition according to the invention comprising a RNAi compound complexed to or encapsulated by a lipid particle, wherein the RNAi compound targets a mRNA involved in collagen synthesis (e.g. COL1A1, P4HA1, etc.) or cytokine production (e.g. TNFα, TGFβ, etc.). In another embodiment, a method for treating sarcoidosis comprises administering to the lungs of a patient in need thereof, a composition according to the invention comprising a RNAi compound complexed to or encapsulated by a lipid particle, wherein the RNAi compound targets the Annexin A11 mRNA.

Administration of the RNAi compositions of the invention results in decreased expression and/or activity of target mRNAs compared to untreated cells. For example, administration of the inventive compositions downregulates the expression and/or activity of one or more mRNAs over-expressed in or genetically linked to the pulmonary disease or disorder.

In one embodiment, administration of the present compositions downregulates the expression and/or activity of a messenger RNA (mRNA) that encodes a protein associated with a phagocytic cell response. In some embodiments, the phagocytic cell is a macrophage and/or a fibroblast.

In some embodiments, administration of the composition downregulates the expression and/or activity of a mRNA encoding a cytokine, a protein associated with collagen synthesis, and/or a phospholipid-binding protein. In exemplary embodiments, administration of the compositions of the invention downregulates the expression and/or activity of a mRNA encoding TNFα, COL1A1, prolyl hydroxylase, and annexin A11.

In one embodiment, administration of the RNAi compositions of the invention results in decreased expression and/or activity of proteins encoded by target mRNAs. For example, in one embodiment, administration of the inventive compositions downregulates the production of inflammatory cytokines such as TNFα and TGFβ. In another embodiment, administration of the inventive compositions downregulates collagen synthesis.

In some embodiments, compositions of the invention reduce or inhibit the expression and/or activity of target mRNAs, such as COL1A1, P4HA1, TNFα, TGFβ, and Annexin A11 mRNAs, in cells of the patient, by about or at least about 5%, 10%, 15%, 20%, 25%, 30%, 35%, 40%, 45%, 50%, 55%, 60%, 65%, 70%, 80%, 90% or 100%, including values therebetween, compared to untreated cells.

In certain embodiments, the reduction or inhibition in the expression and/or activity of target mRNAs (e.g. COL1A1, P4HA1, TNFα, TGFβ, and Annexin A11) provided by the compositions is about 5-90%, 5-80%, 5-70%, 5-60%, 5-50%, 5-40%, 5-30%, 5-20%, 5-10%, 10-90%, 10-80%, 10-70%, 10-60%, 10-50%, 10-40%, 10-30%, 20-80%, about 20-70%, about 20-60%, about 20-50%, about 20-40%, about 30-80%, about 30-70%, about 30-60%, about 30-50%, about 40-80%, about 50-80%, about 50-70%, or about 50-60%, including values and subranges therebetween, compared to untreated cells.

In some other embodiments, there is about or at least about 2-fold, 2.5-fold, 3-fold, 3.5-fold, 4-fold, 4.5-fold, 5-fold, 6-fold, 7-fold, 8-fold, 9-fold, or about 10-fold, reduction in the expression and/or activity of target mRNAs (e.g. COL1A1, P4HA1, TNFα, TGFβ, and Annexin A11) compared to untreated cells.

In some embodiments, compositions of the invention reduce or inhibit recruitment of phagocytic cells and lymphocytes to fibrotic plaques and/or sarcoid granulomas in the organs of the patient. For example, in one embodiment, there is about or at least about 5%, 10%, 15%, 20%, 25%, 30%, 35%, 40%, 45%, 50%, 55%, 60%, 65%, 70%, 80%, 90% or 100%, including values therebetween, reduction in the recruitment of phagocytic cells and/or lymphocytes to fibrotic plaques and/or sarcoid granulomas in the organs of the patient compared to the recruitment prior to the treatment or compared to an untreated patient. In another embodiment, there is about 5-90%, 5-80%, 5-70%, 5-60%, 5-50%, 5-40%, 5-30%, 5-20%, 5-10%, 10-90%, 10-80%, 10-70%, 10-60%, 10-50%, 10-40%, 10-30%, 20-80%, about 20-70%, about 20-60%, about 20-50%), about 20-40%, about 30-80%), about 30-70%, about 30-60%, about 30-50%, about 40-80%, about 50-80%, about 50-70%, or about 50-60%, including values and subranges therebetween, reduction in the recruitment of phagocytic cells and/or lymphocytes to fibrotic plaques and/or sarcoid granulomas.

In some embodiments, compositions of the invention reduce or inhibit migration of phagocytic cells and lymphocytes from fibrotic plaques and/or sarcoid granulomas to other organs of the patient. For example, in one embodiment, there is about or at least about 5%, 10%, 15%, 20%, 25%, 30%, 35%, 40%, 45%, 50%, 55%, 60%, 65%, 70%, 80%, 90% or 100%, including values therebetween, reduction in the migration of phagocytic cells and/or lymphocytes compared to the migration prior to the treatment or compared to an untreated patient. In another embodiment, there is about 5-90%, 5-80%, 5-70%, 5-60%, 5-50%, 5-40%, 5-30%, 5-20%, 5-10%, 10-90%, 10-80%, 10-70%, 10-60%, 10-50%, 10-40%, 10-30%, 20-80%, about 20-70%, about 20-60%, about 20-50%, about 20-40%, about 30-80%, about 30-70%), about 30-60%, about 30-50%, about 40-80%, about 50-80%, about 50-70%, or about 50-60%, including values and subranges therebetween, reduction in the migration of phagocytic cells and/or lymphocytes compared to the migration prior to the treatment or compared to an untreated patient.

In some embodiments, compositions of the invention reduce or inhibit production of Th1 cytokines/chemokines in the patient compared to the production prior to the treatment or compared to an untreated patient. For example, in one embodiment, there is about or at least about 5%, 10%, 15%, 20%, 25%, 30%, 35%, 40%, 45%, 50%, 55%, 60%, 65%, 70%, 80%, 90% or 100%, including values therebetween, reduction in the production of Th1 cytokines/chemokines compared to the production prior to the treatment or compared to an untreated patient. In another embodiment, there is about 5-90%, 5-80%, 5-70%, 5-60%, 5-50%, 5-40%, 5-30%, 5-20%, 5-10%, 10-90%, 10-80%, 10-70%, 10-60%, 10-50%, 10-40%, 10-30%, 20-80%, about 20-70%, about 20-60%, about 20-50%, about 20-40%, about 30-80%, about 30-70%, about 30-60%, about 30-50%, about 40-80%, about 50-80%, about 50-70%, or about 50-60%, including values and subranges therebetween, reduction in the production of Th1 cytokines/chemokines compared to the production prior to the treatment or compared to an untreated patient.

In one embodiment, compositions of the invention reduce fibrotic scars or fibrotic plaques of pulmonary fibrosis, for example, by about or at least by about 5%, 10%, 15%, 20%, 25%, 30%, 35%, 40%, 45%, 50%, 55%, 60%, 65%, 70%, 80%, 90% or 100%, including values therebetween, compared to the fibrotic scars or plaques prior to the treatment or compared to an untreated patient. Chest X-ray, CT scans, and/or lung biopsies may be used to monitor fibrotic scars/plaques in the patient.

In one embodiment, compositions of the invention reduce sarcoid granulomas in the patient, for example, by about or at least by about 5%, 10%, 15%, 20%, 25%, 30%, 35%, 40%, 45%, 50%, 55%, 60%, 65%, 70%, 80%, 90% or 100%, including values therebetween, compared to the sarcoid granulomas prior to the treatment or compared to an untreated patient. Chest X-ray, CT scans, and/or lung biopsies may be used to monitor the status of sarcoid granulomas in the patient.

In another embodiment, compositions of the invention improve oxygen saturation in the patient's blood, for example, by about or at least by about 5%, 10%, 15%, 20%, 25%, 30%, 35%, 40%, 45%, 50%, 55%, 60%, 65%, 70%, 80%, 90% or 100%, including values therebetween, compared to the oxygen saturation levels prior to the treatment or compared to an untreated patient. An oximetry test may be used to monitor oxygen saturation.

In various embodiments, compositions of the invention are administered to the lungs of a patient via inhalation. For example, administering to the lungs via inhalation includes administering via a metered dose inhaler (MDI), nebulizer or a dry powder inhaler. Accordingly, in one aspect, the invention provides a method for treating pulmonary fibrosis or sarcoidosis comprising administering to the lungs of a patient in need thereof, via inhalation, one or more compositions of the invention.

In some embodiments, compositions of the invention are administered to the lungs of a patient intranasally or intratracheally. Intranasal administration includes administering via a metered dose inhaler (MDI), nebulizer or a dry powder inhaler. Accordingly, in one aspect, the invention provides a method for treating pulmonary fibrosis or sarcoidosis comprising intranasally or intratracheally administering to the lungs of a patient in need thereof, one or more compositions of the invention.

In some embodiments, a patient in need of a treatment using compositions of the invention may have a pre-existing pulmonary condition. For example, in one embodiment, a patient in need of a treatment using compositions of the invention is a cystic fibrosis patient, a bronchiectasis patient, or a patient with a bacterial or viral pulmonary infection. A cystic fibrosis patient, a bronchiectasis patient, or a patient with a bacterial or viral pulmonary infection may eventually develop additional pulmonary diseases such as pulmonary fibrosis or sarcoidosis.

In some embodiments, a patient in need of a treatment using compositions of the invention could have pulmonary fibrosis associated with sarcoidosis.

In some embodiments, the compositions of the invention could be used to treat a lung cancer in a patient in need thereof.

Neutrophils may be associated with various pulmonary disorders including chronic obstructive pulmonary disease (COPD), acute lung injury (ALI), cystic fibrosis (CF), bronchiectasis, and infiltrative pulmonary diseases among others. Basophils may be associated with fatal asthma. Eosinophils may be associated with allergic asthma; eosinophilic pulmonary diseases such as infections, drug-induced pneumonitis, inhaled toxins, systemic disorders (e.g., eosinophilic granulomatosis with polyangiitis, and Loeffler's syndrome); allergic bronchopulmonary aspergillosis; tropical pulmonary eosinophilia; hypereosinophilic syndromes; and lung cancers. Mast cells may be associated with various pulmonary disorders, including asthma, COPD, respiratory infections, and lung fibrosis. Macrophages may be associated with various pulmonary disorders, including COPD, CF, and sarcoidosis. Dendritic cells may be associated with various pulmonary disorders, including COPD, allergic asthma, and allergic rhinitis. The present invention provides methods for treating one or more aforementioned diseases via administration of one of the RNAi compositions of the present invention to a patient in need thereof. Furthermore, various pulmonary disorders treatable by the methods provided herein are provided in Tables 4-7.

The compositions described herein are useful for the treatment of a patient that has elevated lung phagocytic cell levels as compared to a healthy individual. To this end, the compositions described herein, in one aspect, are administered to a patient in need thereof, to inhibit production of one of the proteins of interest, *i.e.,* by RNAi. For example, in one embodiment, an effective amount of one or more of the compositions described herein is administered via a patient in need thereof, for example, a cystic fibrosis patient or a patient with α1-AT deficiency. In one embodiment, an effective amount of one of the compositions provided herein is delivered to a patient in need thereof, to treat or prevent lung damage, and/or to treat or prevent bronchiectasis. For example, in one embodiment, a method of treating a patient for bronchiectasis is provided. The method comprises, in one embodiment, administering to the lungs of the patient suffering from bronchiectasis an effective amount of one or more of the compositions described herein, for example a composition comprising siRNA complexed to a lipid (*e.g.,* a cationic lipid). In a further embodiment, the patient is a cystic fibrosis (CF) patient. In another embodiment, an effective amount of one of the compositions described herein is administered to a patient in need of treatment of α₁-AT deficiency.

The term "treating" includes: (1) preventing or delaying the appearance of clinical symptoms of the state, disorder or condition developing in the subject that may be afflicted with or predisposed to the state, disorder or condition but does not yet experience or display clinical or subclinical symptoms of the state, disorder or condition; (2) inhibiting the state, disorder or condition (*i.e.,* arresting, reducing or delaying the development of the disease, or a relapse thereof in case of maintenance treatment, of at least one clinical or subclinical symptom thereof); and/or (3) relieving the condition (*e.g.*, causing regression of the state, disorder or condition or at least one of its clinical or subclinical symptoms). The benefit to a subject to be treated is either statistically significant or at least perceptible to the subject or to the physician.

"Prophylaxis," as used herein, can mean complete prevention of an infection or disease, or prevention of the development of symptoms of that infection or disease; a delay in the onset of an infection or disease or its symptoms; or a decrease in the severity of a subsequently developed infection or disease or its symptoms.

Various pulmonary disorders can be treated by the methods and compositions provided herein. For example, in one embodiment, a method is provided for treating a patient in need thereof for a pulmonary disorder associated with tissue damage. In another embodiment, a method is provided for treating a patient in need thereof for an inflammatory pulmonary disorder.

In one embodiment, the pulmonary disorder is one of the disorders set forth in any one of Tables 4-7. Pulmonary fibrosis or sarcoidosis are present in the claims of the invention.

| **Table 4. Representative pulmonary disorders treatable by the methods of the invention, and their associated phagocytic cell type(s)** | |
|---|---|
| **Phagocytic cell type** | **Pulmonary Disorder** |
| Neutrophils | cystic fibrosis (CF) |
| | non-cystic fibrosis bronchiectasis (NCFB) |
| | idiopathic pulmonary fibrosis (IPF) |
| | secondary organizing pneumonia (BOOP) |
| | microscopic polyangiitis |
| | chronic obstructive pulmonary disease (COPD) |
| | acute lung injury (ALI) |
| | infiltrative pulmonary diseases |
| | bronchiectasis |
| Eosinophil | Allergic asthma |
| | eosinophilic pulmonary diseases [infections, drug-induced pneumonitis, inhaled toxins, systemic disorders] |
| | simple eosinophilic pneumonia (Löffler syndrome) |
| | eosinophilic granulomatosis with polyangiitis (Churg-Strauss syndrome) |
| | tropical pulmonary eosinophilia |
| | hypereosinophilic syndromes (6 subtypes) |
| | lung cancer |
| | pulmonary manifestations in inflammatory bowel diseases |
| | Wegener's granulomatosis |
| | secondary organizing pneumonia (BOOP) |
| | cystic fibrosis (CF) |
| | idiopathic pulmonary fibrosis (IPF) |
| | allergic bronchopulmonary aspergillosis (ABPA) |
| | chronic idiopathic eosinophilic pneumonia |
| | acute idiopathic eosinophilic pneumonia |
| Basophil | Fatal Asthma |
| Mast cell | Asthma |
| | COPD |
| | Lung fibrosis |
| | Respiratory infection |
| Macrophage/monocyte | Sarcoidosis |
| | Chronic beryllium disease (Berylliosis) |
| | Asbestos |
| | Pulmonary Langerhans Cells Histiocytosis (histiocytosis X) |
| | cystic fibrosis (CF) |
| | microscopic polyangiitis |
| | desquamative interstitial pneumonia (DIP) |
| | chronic obstructive pulmonary disease (COPD) |
| | acute lung injury (ALI) |
| | asbestosis |
| Dendritic cell | Pulmonary Langerhans Cells Histiocytosis (histiocytosis X) |
| | chronic obstructive pulmonary disease (COPD) |
| | allergic asthma |
| | allergic rhinitis |

| **Table 5. Representative pulmonary disorders treatable by the methods of the invention, and their associated phagocytic cell type(s)** | |
|---|---|
| **Pulmonary Disorder** | **Phagocytic cell type** |
| chronic obstructive pulmonary disease (COPD) | Neutrophils, macrophages, mast cells |
| acute lung injury (ALI) | Neutrophils |
| infiltrative pulmonary diseases | Neutrophils |
| Allergic asthma | Eosinophils, dendritic cells, mast cells |
| tropical pulmonary eosinophilia | Eosinophils |
| drug-induced pneumonitis | Eosinophils |
| Fatal asthma | Basophils |
| Respiratory infections | Mast cells |
| lung fibrosis | Mast cells |
| allergic rhinitis | Dendritic cells |

| **Table 6. Representative pulmonary disorders treatable by the methods of the invention** | |
|---|---|
| **Class of Pulmonary Disorder** | **Pulmonary Disorder** |
| **Vasculitides** | Granulomatosis with poly angiitis (Wegener's) |
| | Microscopic polyangiitis |
| | Eosinophilic granulomatosis with polyangiitis (Churg-Strauss) |
| | Behçet's disease |
| | Takayasu's arteritis |
| **Autoimmune diseases** | Anti-basement membrane syndrome |
| | Pulmonary alveolar proteinosis |
| **Disorders of genetic origin** | Lymphangioleiomyomatosis associated with tuberous sclerosis |
| | Multiple cystic lung disease in Birt-Hogg-Dubé syndrome |
| | Primary ciliary dyskinesia |
| **Other idiopathic disorders (lung limited)** | Idiopathic eosinophilic pneumonias |
| | Tracheobronchopathia osteochondroplastica |
| | Tracheobronchomegaly (Mounier-Kuhn syndrome) |
| | Idiopathic bronchiolitis |
| **Other rare diseases** | Thoracic endometriosis |
| | Langerhans' cell histiocytosis |
| **Miscellaneous** | Idiopathic pulmonary fibrosis (IPF) |
| | Chronic thromboembolic pulmonary hypertension (CTEPH) |
| | Pulmonary arterial hypertension (PAH) |
| | chronic pulmonary infections due to Pseudomonas aeruginosa in patients with cystic fibrosis (CF) aged 6 years and older. |
| | pulmonary multi-drug resistant tuberculosis (MDR-TB) |
| | α-1 antitrypsin deficiency |
| | Lymphangioleiomyomatosis |
| | Scleroderma |
| | Idiopathic chronic eosinophilic pneumonia (ICEP) |
| | Pulmonary alveolar proteinosis (PAP) |

| **Table 7. Representative pulmonary disorders treatable by the methods of the invention** | |
|---|---|
| **IDIOPATHIC INTERSTITIAL PNEUMONIAS** | **INTERSTITIAL LUNG DISEASE IN CONNECTIVE TISSUE DISEASES** |
| idiopathic pulmonary fibrosis desquamative interstitial pneumonia (DIP) respiratory bronchiolitis interstitial lung disease (RBILD) | interstitial lung disease in systemie sclerosis interstitial lung disease in rheumatoid arthritis |
| | interstitial lung diseasein idiopathic inflammatory myopathies (polymyositis, dermatomyositis, anti-synthetase syndrome) interstitial lung disease in Sjögren syndrome interstitial lung disease in mixed connective tissue disease (MCTD) |
| acute interstitial pneumonia (AIP) nospecific interstitial pneumonia (NSIP) cryptogenic organizing pneumonia (COP = idiopathic BOOP) | |
| lymphoid interstitial pneumonia (LIP) idiopathic interstitial pneumonia : unspecified | |
| | interstitial lung disease in overlap syndromes |
| | interstitial lung disease in undifferentiated connective tissue disease |
| | |
| **HYPEREOSINOPHILIC PULMONARY DISORDERS** | **ALLERGIC BRONCHOPULMONARY ASPERGILLOSIS (ABPA)PULMONARY VASCULITIS** |
| chronic idiopathic eosinophilic pneumonia acute diopathic eosinophilic pneumonia idiopatic hypereosinophilic syndrome with pulmonary manifestations hypereosinophilic lung disease: other (specify) | Wegener's granulomatosis microscopic polyangirtis Churg-Strauss syndrome pulmonary vasculitis : unspecified |
| **ALVEOLAR HEMORRHAGE SYNDROMES** | BRONCHIOLITIS OBLITERANS (in non-transplanted patients) |
| Goodpasture syndrome idiopathic pulmonary hemosideresis alveolar hemorrhage syndrome of undetermined origin alveolar hemorrhage syndrome of determined origin | **ASBESTOSIS** |
| | **SARCOIDOSIS** |
| | **CHRONIC BERYLLIUM DISEASE** |
| | |
| **PULMONARY ARTERIOVENOUS MALFORMATIONS IN HEREDITARY HEMORRHAGIC TELANGIECTASIA (HHT)** | **WATERPROOFING SPRAY PNEUMONITIS** |
| | **COMBINED PULMONARY FIBROSIS AND EMPHYSEMA** |
| **PULMONARY MANIFESTATIONS OF GASTRO-INTESTINAL DISORDERS** | combined pulmonary fibrosis and emphysema without associated connective |
| pulmonary manifestations in inflammatory bowel diseases | tissue disease |
| | combined pulmonary fibrosis and emphysema with connective tissue disease |
| severe hepatopulmonary syndrome (pa02 < 55 mmHg) | |
| | ALPHA-1-ANTITRYPSIN DEFICIENCY EMPHYSEMA |
| | |
| PULMONARY LYMPHANGIOLEIOMYOMATOSIS (LAM) | HISTIOCYTOSIS (histiocytosis X) |
| Sporadic pulmonary lymphangioleiomyomatosis (S-LAM) Pulmonary lymphangioleiomyomatosis in tuberous selerosis (TSC-LAM) | PRIMARY PULMONARY LYMPHOMA |
| | PRIMARY CILIARY DYSKINESIA (without or with situs inversus) |
| ALVEOLAR PROTEINOSIS | RARE CAUSE OF HYPERSENSITIVITY PNEUMONITIS |
| PULMONARY AMYLOIDOSIS | (all causes other than farmer's lung disease and pigeon breeder's lung disease) |

In one embodiment, a method of treating a patient for COPD is provided. The method comprises, in one embodiment, administering to the lungs of the COPD patient an effective amount of one or more of the compositions described herein via an MDI, DPI or nebulizer.

In another embodiment, methods are provided herein to treat a patient in need of treatment of leukocytosis, inflammatory lung disease, lung tissue damage, emphysema, acute respiratory distress disorder or acute lung injury. In one embodiment, the method comprises administering to a patient in need thereof via inhalation, one or more of the compositions described herein, for example, via an MDI, DPI or nebulizer.

The composition, in one embodiment, is administered directly to the lungs (i) of a CF patient to treat or prevent lung injury due to neutrophil degranulation, or degranulation of some other granulocyte such as a monocyte, eosinophil, basophil or mast cell (ii) to a patient presenting with α₁-AT deficiency, or (iii) to a patient for treatment of bronchiectasis, chronic infection, or any other pulmonary disorder that results in the elevation of lung phagocytic cell levels as compared to the levels of a healthy individual.

In one embodiment, a patient with α₁-antitrypsin deficiency is treated with a composition and method provided herein. In a further embodiment, the compositions of the invention are co-administered to the patient with an effective amount of α₁-antitrypsin.

One skilled in the art would understand that the dosing amount and dosing frequency of the compositions would very depending on the target mRNA, the efficacy of RNAi compounds, severity of the disease, age and weight of the patient, etc.

Exemplary dosing frequencies include administering the effective amount of the composition daily, every other day, once weekly, twice weekly, or three times weekly.

In one embodiment, prior to delivery of the RNAi composition to the patient in need thereof about 70% to about 100% of the RNAi compound present in the composition is liposomal complexed or present in lipid nanoparticles. In another embodiment, prior to delivery of the siRNA composition to the patient in need thereof, about 80% to about 99%, or about 85% to about 99%, or about 90% to about 99% or about 95% to about 99% or about 96% to about 99% of the siRNA present in the composition is liposomal complexed or present in lipid nanoparticles. In another embodiment, prior to delivery of the siRNA composition to the patient in need thereof, about 98% of the siRNA present in the composition is liposomal complexed or present in lipid nanoparticles.

In one embodiment, upon delivery of the composition to the lungs of a patient in need thereof, for example, via aerosolization via a nebulizer, about 20% to about 50% of the liposomal complexed (or lipid-complexed in the case of lipid nanoparticles and/or lipid microparticles) RNAi compound is released, due to shear stress on the liposomes (or lipid particles). In another embodiment, upon delivery of the composition, about 25% to about 45%, or about 30% to about 40% of the liposomal complexed(or lipid-complexed in the case of lipid nanoparticles and/or lipid microparticles) RNAi compound is released, due to shear stress on the liposomes (or lipid particles).

Compositions and delivery devices (the invention is defined by the claims. Any composition not falling within the scope of the claims is not according to the invention. Delivery devices are not claimed).

As provided herein, the present invention provides compositions, systems and methods for the treatment of diseases or disorders associated with aberrant neutrophil elastase expression and/or activity. The treatment methods comprise, in one embodiment, delivery of one of the compositions described herein to the lungs of a patient in need thereof, for example, a cystic fibrosis patient.

The compositions of the present invention may be used in any dosage dispensing device adapted for pulmonary administration. Accordingly, in one aspect, the present invention provides systems comprising one or more of the compositions described herein and an inhalation delivery device. The device, in one embodiment, is constructed to ascertain optimum metering accuracy and compatibility of its constructive elements, such as container, valve and actuator with the composition and could be based on a mechanical pump system, *e.g.,* that of a metered-dose nebulizer, dry powder inhaler, metered dose inhaler (MDI), soft mist inhaler, or a nebulizer. For example, pulmonary delivery devices include a jet nebulizer, electronic nebulizer, a soft mist inhaler, and a capsule-based dry powder inhaler, all of which are amenable for use with the compositions of the present invention.

In some embodiments, the compositions of the invention for pulmonary/inhalation/nasal/intranasal administration are formulated in the form of a dry powder formulation, a suspension formulation, a nanosuspension formulation, a microsuspension formulation, or a nebulized spray.

In certain embodiments, the compositions of the invention formulated for pulmonary/inhalation/nasal/intranasal administration comprise a propellant, e.g. a hydrocarbon propellant.

The composition, in one embodiment, is administered via a nebulizer, which provides an aerosol mist of the composition for delivery to the lungs of a subject. A nebulizer type inhalation delivery device can contain the compositions of the present invention as an aqueous solution or a suspension. In generating the nebulized spray of the compositions for inhalation, the nebulizer type delivery device may be driven ultrasonically, by compressed air, by other gases, electronically or mechanically. The ultrasonic nebulizer device usually works by imposing a rapidly oscillating waveform onto the liquid film of the composition via an electrochemical vibrating surface. At a given amplitude the waveform becomes unstable, whereby it disintegrates the liquids film, and it produces small droplets of the composition. The nebulizer device driven by air or other gases operates on the basis that a high pressure gas stream produces a local pressure drop that draws the liquid composition into the stream of gases via capillary action. This fine liquid stream is then disintegrated by shear forces.

A nebulizer type inhalation delivery device can contain the compositions of the present invention as a solution, usually aqueous, or a suspension. For example, the composition can be suspended in saline and loaded into the inhalation delivery device. In generating the nebulized spray of the compositions for inhalation, the nebulizer delivery device may be driven ultrasonically, by compressed air, by other gases, electronically or mechanically (e.g., vibrating mesh or aperture plate). Vibrating mesh nebulizers generate fine particle, low velocity aerosol, and nebulize therapeutic solutions and suspensions at a faster rate than conventional jet or ultrasonic nebulizers. Accordingly, the duration of treatment can be shortened with a vibrating mesh nebulizer, as compared to a jet or ultrasonic nebulizer. Vibrating mesh nebulizers amenable for use with the methods described herein include the Philips Respironics I-Neb®, the Omron MicroAir, the Nektar Aeroneb®, and the PARI eFlow®. Other devices that can be used with the compositions described herein include jet nebulizers (e.g., PARI LC Star, AKITA), soft mist inhalers, and capsule-based dry powder inhalers (*e.g.*, PH&T Turbospin).

The nebulizer may be portable and hand held in design, and may be equipped with a self-contained electrical unit. The nebulizer device may comprise a nozzle that has two coincident outlet channels of defined aperture size through which the liquid composition can be accelerated. This results in impaction of the two streams and atomization of the composition. The nebulizer may use a mechanical actuator to force the liquid composition through a multiorifice nozzle of defined aperture size(s) to produce an aerosol of the composition for inhalation. In the design of single dose nebulizers, blister packs containing single doses of the composition may be employed.

The device can contain, and be used to deliver, a single dose of the compositions of the invention, or the device can contain, and be used to deliver, multi-doses of the compositions of the invention.

In the present invention the nebulizer may be employed to ensure the sizing of particles is optimal for positioning of the particle within, for example, the pulmonary membrane.

A metered dose inhalator (MDI) may be employed as the inhalation delivery device for the compositions of the present invention. This device is pressurized (pMDI) and its basic structure comprises a metering valve, an actuator and a container. A propellant is used to discharge the composition from the device. Suitable propellants, *e.g*., for MDI delivery, may be selected among such gases as fluorocarbons, chlorofluorocarbons (CFCs), hydrocarbons, hydrofluorocarbons, hydrofluoroalkane propellants (*e.g.*, HFA-134a and HFA-227), nitrogen and dinitrogen oxide or mixtures thereof.

The composition may consist of particles of a defined size suspended in the pressurized propellant(s) liquid, or the composition can be in a solution or suspension of pressurized liquid propellant(s). The propellants used are primarily atmospheric friendly hydroflourocarbons (HFCs) such as 134a and 227. The inhalation delivery device, in one embodiment, delivers a single dose via, *e.g.,* a blister pack, or it may be multi dose in design. The pressurized metered dose inhalator of the inhalation system can be breath actuated to deliver an accurate dose of the lipid-containing composition. To insure accuracy of dosing, the delivery of the composition may be programmed via a microprocessor to occur at a certain point in the inhalation cycle. The MDI may be portable and hand held.

Upon nebulization, the nebulized composition (also referred to as "aerosolized composition") is in the form of aerosolized particles. The aerosolized composition can be characterized by the particle size of the aerosol, for example, by measuring the "mass median aerodynamic diameter" or "fine particle fraction" associated with the aerosolized composition. "Mass median aerodynamic diameter" or "MMAD" is normalized regarding the aerodynamic separation of aqua aerosol droplets and is determined by impactor measurements, *e.g.,* the Anderson Cascade Impactor (ACI) or the Next Generation Impactor (NGI). The gas flow rate, in one embodiment, is 28 Liter per minute for the ACI and 15 liter per minute for the NGI.

"Geometric standard deviation" or "GSD" is a measure of the spread of an aerodynamic particle size distribution. Low GSDs characterize a narrow droplet size distribution (homogeneously sized droplets), which is advantageous for targeting aerosol to the respiratory system. The average droplet size of the nebulized composition provided herein, in one embodiment is less than 5 µm or about 1 µm to about 5 µm, and has a GSD in a range of 1.0 to 2.2, or about 1.0 to about 2.2, or 1.5 to 2.2, or about 1.5 to about 2.2.

"Fine particle fraction" or "FPF," as used herein, refers to the fraction of the aerosol having a particle size less than 5 µm in diameter, as measured by cascade impaction. FPF is usually expressed as a percentage.

In one embodiment, the mass median aerodynamic diameter (MMAD) of the nebulized composition is about 1 µm to about 5 µm, or about 1 µm to about 4 µm, or about 1 µm to about 3 µm or about 1 µm to about 2 µm, as measured by the Anderson Cascade Impactor (ACI) or Next Generation Impactor (NGI). In another embodiment, the MMAD of the nebulized composition is about 5 µm or less, about 4 µm or less, about 3 µm or less, about 2 µm or less, or about 1 µm or less, as measured by cascade impaction, for example, by the ACI or NGI.

In one embodiment, the MMAD of the aerosol of the pharmaceutical composition is less than about 4.9 µm, less than about 4.5 µm, less than about 4.3 µm, less than about 4.2 µm, less than about 4.1 µm, less than about 4.0 µm or less than about 3.5 µm, as measured by cascade impaction.

In one embodiment, the MMAD of the aerosol of the pharmaceutical composition is about 1.0 µm to about 5.0 µm, about 2.0 µm to about 4.5 µm, about 2.5 µm to about 4.0 µm, about 3.0 µm to about 4.0 µm or about 3.5 µm to about 4.5 µm, as measured by cascade impaction (*e*.*g*., by the ACI or NGI).

In one embodiment, the FPF of the aerosolized composition is greater than or equal to about 50%, as measured by the ACI or NGI, greater than or equal to about 60%, as measured by the ACI or NGI or greater than or equal to about 70%, as measured by the ACI or NGI. In another embodiment, the FPF of the aerosolized composition is about 50% to about 80%, or about 50% to about 70% or about 50% to about 60%, as measured by the NGI or ACI.

In one embodiment, a metered dose inhalator (MDI) is employed as the inhalation delivery device for the compositions of the present invention. In such a situation the RNAi compound is formulated as a suspension in a propellant (*e.g.*, hydroflourocarbon) prior to loading into the MDI. The basic structure of the MDI as provided above, comprises a metering valve, an actuator and a container. A propellant is used to discharge the composition from the device. The composition may consist of particles of a defined size suspended in the pressurized propellant(s) liquid, or the composition can be in a solution or suspension of pressurized liquid propellant(s). The propellants used are primarily atmospheric friendly hydroflourocarbons (HFCs) such as 134a and 227, and may contain other co-solvents. The device of the inhalation system may deliver a single dose via, *e.g.,* a blister pack, or it may be multi dose in design. The pressurized metered dose inhalator of the inhalation system can be breath actuated to deliver an accurate dose of the lipid-containing composition. To insure accuracy of dosing, the delivery of the composition may be programmed via a microprocessor to occur at a certain point in the inhalation cycle. The MDI may be portable and hand held.

In one embodiment, a dry powder inhaler (DPI) is employed as the inhalation delivery device for the compositions of the present invention. In one embodiment, the DPI generates particles having an MMAD of from about 1 µm to about 10 µm, or about 1 µm to about 9 µm, or about 1 µm to about 8 µm, or about 1 µm to about 7 µm, or about 1 µm to about 6 µm, or about 1 µm to about 5 µm, or about 1 µm to about 4 µm, or about 1 µm to about 3 µm, or about 1 µm to about 2 µm in diameter, as measured by the NGI or ACI. In another embodiment, the DPI generates a particles having an MMAD of from about 1 µm to about 10 µm, or about 2 µm to about 10 µm, or about 3 um to about 10 µm, or about 4 µm to about 10 µm, or about 5 µm to about 10 µm, or about 6 µm to about 10 µm, or about 7 µm to about 10 µm, or about 8 µm to about 10 µm, or about 9 µm to about 10 µm, as measured by the NGI or ACI.

In one embodiment, the MMAD of the particles generated by the DPI is about 1 µm or less, about 9 µm or less, about 8 µm or less, about 7 µm or less, 6 µm or less, 5 µm or less, about 4 µm or less, about 3 µm or less, about 2 µm or less, or about 1 µm or less, as measured by the NGI or ACI.

In one embodiment, the MMAD of the particles generated by the DPI is less than about 9.9 µm, less than about 9.5 µm, less than about 9.3 µm, less than about 9.2 µm, less than about 9.1 µm, less than about 9.0 µm, less than about 8.5 µm, less than about 8.3 µm, less than about 8.2 µm, less than about 8.1 µm, less than about 8.0 µm, less than about 7.5 µm, less than about 7.3 µm, less than about 7.2 µm, less than about 7.1 µm, less than about 7.0 µm, less than about 6.5 µm, less than about 6.3 µm, less than about 6.2 µm, less than about 6.1 µm, less than about 6.0 µm, less than about 5.5 µm, less than about 5.3 µm, less than about 5.2 µm, less than about 5. 1 µm, less than about 5.0 µm, less than about 4.5 µm, less than about 4.3 µm, less than about 4.2 µm, less than about 4.1 µm, less than about 4.0 µm or less than about 3.5 µm, as measured by the NGI or ACI.

In one embodiment, the MMAD of the particles generated by the DPI is about 1.0 µm to about 10.0 µm, about 2.0 µm to about 9.5 µm, about 2.5 µm to about 9.0 µm, about 3.0 µm to about 9.0 µm, about 3.5 µm to about 8.5 µm or about 4.0 µm to about 8.0 µm.

In one embodiment, the FPF of the particulate composition generated by the DPI is greater than or equal to about 40%, as measured by the ACI or NGI, greater than or equal to about 50%, as measured by the ACI or NGI, greater than or equal to about 60%, as measured by the ACI or NGI, or greater than or equal to about 70%, as measured by the ACI or NGI. In another embodiment, the FPF of the aerosolized composition is about 40% to about 70%, or about 50% to about 70% or about 40% to about 60%, as measured by the NGI or ACI.

According to the methods of the invention, the compositions described herein are delivered to the lungs of a patient in need thereof via an inhalation delivery device. Any of the inhalation delivery devices described herein can be employed, for example, an MDI, nebulizer or dry powder inhaler can be used to deliver an effective amount of one or more of the compositions described herein to a patient in need thereof, for example, a CF patient or a patient with α1-AT deficiency.

Without wishing to be bound by theory, it is thought that the inhalation delivery methods described herein avoid systemic inactivation of the siRNA. Moreover, the inhalation methods described herein provides for efficient, direct delivery of the compositions to the phagocytic cells in the lungs.

**EXAMPLES** (examples falling outside of the scope of the claims are to be considered as reference examples and do not form part of the invention).

The present invention is further illustrated by reference to the following Examples. However, it is noted that these Examples, are illustrative and are not to be construed as restricting the scope of the invention in any way.

### Example 1- Design and Synthesis of siRNA

siRNA target sequences are specific to the gene of interest and have ∼20-50% GC content. For example, siRNAs satisfying the following conditions are capable of effective gene silencing in mammalian cells: (1) G/C at the 5' end of the sense strand; (2) A/U at the 5' end of the antisense strand; (3) at least 5 A/U residues in the first 7 bases of the 5' terminal of the antisense strand; (4) no runs of more than 9 G/C residues. Generally the mRNA target site is at least 50-200 bases downstream of the start codon to avoid regions in which regulatory proteins might bind.

The oligonucleotides include the target sequence plus the T7 RNA polymerase promoter sequence and 6 extra nucleotides upstream of the minimal promoter sequence to allow for efficient T7 RNA polymerase binding. The DNA oligonucleotides are resuspended in nuclease-free water to a final concentration of 100 pmol/µL. Each pair of DNA oligonucleotides is combined to generate either the sense strand RNA or antisense strand RNA templates by mixing 10 µL of each of the two DNA oligonucleotides, 30 µL nuclease-free water, and 50 µL 2x oligo annealing buffer for a total volume of 100 µL. This mixture is heated at 90-95 °C for 3-5 minutes, and then allowed to cool slowly to room temperature. The final concentration of annealed oligonucleotide is approximately 10 pmol/µL.

To synthesize large quantities of the siRNA, 10 µL of RiboMAX™ (Promega), 2.0 µl of the annealed oligonucleotide template DNA (10 pmol/µL), 6.0 µL, and 2.0 µL of T7 Enzyme are mixed at room temperature to a total volume of 20 µL. The 20 µL reaction may be scaled up as necessary (up to 500 µL total volume). This mixture is incubated at 37 °C for 30 minutes.

To remove the DNA template and annealing siRNA, the DNA template can be removed by digestion with DNase following the transcription reaction by adding to each transcription reaction 1 µL of RNase-free DNase and incubating the mixture for 30 minutes at 37 °C. The separate sense and antisense reactions are combined and incubated for 10 minutes at 70 °C. The mixture is then allowed to cool to room temperature (approximately 20 minutes). This step anneals the separate short sense and antisense RNA strands generating siRNA.

To purify the siRNA, 0.1 volume of 3M Sodium Acetate (pH 5.2) and 1 volume of isopropanol are added to the siRNA, and the mixture is placed on ice for 5 minutes. The reaction appears cloudy. The mixture is centrifuged at top speed in a microcentrifuge for 10 minutes. The supernatant is aspirated and the pellet is washed with 0.5 mL of cold 70% ethanol, to remove all ethanol following the wash. The pellet is air-dried for 15 min. at room temperature, and then resuspended in nuclease-free water in a volume 2-5 times the original reaction volume.

### Example 2 - Preparation of liposomal and nanoparticle formulations

To test the uptake and activity of siRNAs complexed with or encapsulated by liposomal and lipid nanoparticles of the invention, formulations 1-17 were prepared. These formulations are summarized in Table 8.

**Table 8: Summary of siRNA nanoparticle formulations**

| | Comp. 1 (molar %) | Comp. 2 (molar %) | Comp. 3 (molar %) | Comp. 4 (molar %) | Comp. 5 (molar %) |
|---|---|---|---|---|---|
| 1 | DODAP (57.1) | DSPC (7.1) | Chol (34.3) | DMG-PEG2000 (1.5) | tRNA/siRNA (0.05) |
| 2 | DODAP (57.1) | DSPC (7.1) | Chol (34.3) | DMG-PEG2000 (1.5) | tRNA/siRNA (0.025) |
| 3 | NA-DOPE (70) | DOPC (30) | | | |
| 4 | DODAP (57.1) | DSPC (7.1) | Chol (35.4) | DMG-PEG2000 (0.4) | tRNA/siRNA (0.025) |
| 5 | DODAP (57.1) | DSPE (7.1) | Chol (34.3) | DMG-PEG2000 (1.5) | tRNA/siRNA (0.025) |
| 6 | DODAP (57.1) | DSPC (7.1) | CHEMS (34.3) | DMG-PEG2000 (1.5) | tRNA/siRNA (0.025) |
| 7 | DODAP (57.1) | DSPE (7.1) | Chol (35.4) | DMG-PEG2000 (0.4) | tRNA/siRNA (0.025) |
| 8 | DODAP (57.1) | DSPE (7.1) | CHEMS (34.4) | DMG-PEG2000 (1.4) | tRNA/siRNA (0.025) |
| 9 | DODAP (70) | DSPC (4) | Chol (24.5) | DMG-PEG2000 (1.5) | tRNA/siRNA (0.025) |
| 10 | DODAP (45) | DSPC (15) | Chol (38.5) | DMG-PEG2000 (1.5) | tRNA/siRNA (0.025) |
| 11 | DODAP (70) | DSPC (4) | CHEMS (24.5) | DMG-PEG2000 (1.5) | tRNA/siRNA (0.025) |
| 12 | DODAP (45) | DSPC (15) | CHEMS (38.5) | DMG-PEG2000 (1.5) | tRNA/siRNA (0.025) |
| 13 | DODAP 57.1 | DSPC (7.1) | THS (34.3) | DMG-PEG2000 (1.5) | tRNA/siRNA (0.025) |
| 14 | DODAP (57.1) | DSPC (16.4) | CHEMS (25) | DMG-PEG2000 (1.5) | tRNA/siRNA (0.025) |
| 15 | DODAP (50) | DSPC (4) | CHEMS (45) | DMG-PEG2000 (1) | tRNA/siRNA (0.025) |
| 16 | DODAP (57.1) | DSPC (16.4) | THS (25) | DMG-PEG2000 (1.5) | tRNA/siRNA (0.025) |
| 17 | DODAP (50) | DSPC (4) | THS (45) | DMG-PEG2000 (1) | tRNA/siRNA (0.025) |

| | | | | | |
|---|---|---|---|---|---|
| DODAP: 1,2-dioleoyl-3-dimethylammonium-propane; DSPC: 1,2-distearoyl-sn-glycero-3-phosphocholine; Chol: cholesterol; DMG-PEG2000: 1,2-Dimyristoyl-sn-glycerol, methoxypolyethylene glycol; NA-DOPE: 1,2-dioleoyl-sn-glycero-3-phosphoethanolamine-N-(dodecanyl) ; DOPC: 1,2-dioleoyl-sn-glycero-3-phosphocholine; DSPE: 1,2-Distearoyl-sn-glycero-3-phosphoethanolamine; CHEMS: cholesterol hemi-succinate; THS: tocopherol hemi-succinate; tRNA: transfer ribonucleic acid; siRNA: small, interfering ribonucleic acid. | | | | | |

A wide range of cationic lipid percentages, from about 45 to 70 mol%, supported both tRNA and siRNA encapsulation into stable nanoparticles. These particles were further tested in cellular uptake and/or gene expression assays to confirm their ability to enter phagocytic cells and reduce expression of target genes.

### Example 3 - Uptake of siRNAs by phagocytic cells

To compare cellular uptake of liposomal and nanoparticle formulations by phagocytic cells found in lungs, *in vitro* uptake of particles by macrophages and fibroblasts was measured. Prior to uptake assays, THP-1 monocytes were differentiated into macrophages by 24-hour incubation with 50 ng/mL phorbol myristate acetate (PMA), followed by 24-hour incubation in fresh RPMI media. For uptake assays, differentiated macrophages or WI-38 fibroblasts cultured in Opti-MEM media containing 2% fetal bovine serum (FBS) were incubated with AF647-labeled particles (final lipid concentration of 140 µg/mL) for 1 or 4 hours, gently harvested, and washed with phosphate-buffered saline (PBS). As a surrogate for siRNA, tRNA was used to generate AF647-labeled nanoparticles used in uptake experiments. Particle uptake into individual cells was quantified by fluorescence-activated cell sorting (FACS) and normalized to the total amount of fluorescent label added per mL of media to calculate the normalized median fluorescence intensity (MFI).

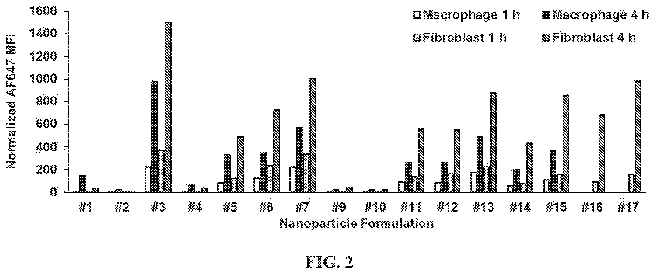

Formulation #3 composed of NA-DOPE and DOPC showed the highest uptake into both macrophages and fibroblasts (Figure 2). Other formulations containing various molar ratios of DODAP, DSPC, CHEMS, DMG-PEG2000, and RNA (formulations #6, #11, and #12) or DODAP, DSPE, cholesterol, DMG-PEG2000, and RNA (formulations #5 and #7) also exhibited good uptake into both macrophages and fibroblasts (Figure 2).

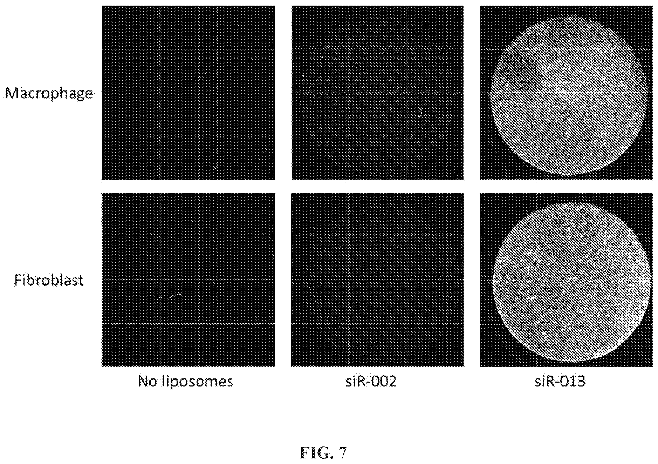

Formulation #2 showed poor uptake into both macrophages and fibroblasts (Figures 2 and 7) whereas Formulation #13 showed good uptake into both macrophages and fibroblasts (Figure 7).

### Example 4 - Activity of siRNA formulations

Several nanoparticle formulations were subsequently made with siRNA (instead of tRNA) and evaluated for ability to reduce *COL1A1* gene expression in fibroblasts. WI-38 fibroblasts were cultured for 48 hours, incubated with lipofectamine (LFC) or various siRNA formulations containing 13-100 pmol of an siRNA targeting *COL1A1* for an additional 24 hours in fresh media, and then harvested RLT lysis buffer (Qiagen). The cells were homogenized using QiaShredder columns (Qiagen), RNA was extracted using RNeasy Mini Kits (Qiagen), and total RNA was quantified using a NanoDrop spectrophotometer (Thermo). RNA was converted to cDNA with a High-Capacity cDNA Reverse Transcription Kit with RNase Inhibitor (Fisher Scientific), and *COL1A1* expression was measured using a CFX96 Real-Time PCR Detection System (BioRad). *COL1A1* expression was normalized to β-actin (*ACTB*) gene expression for each sample and then to the expression level in untreated fibroblasts. Sequences for the siRNA targeting *COL1A1* and real-time PCR primers for detecting *COL1A1* expression are shown in Table 9.

**Table 9: Summary of siRNA and real-time PCR primer sequences**

| | Gene | Sequence |
|---|---|---|
| siRNA | *COL1A1* | |
| | *P4HA1* | |
| | *TNF* | |
| | *ANXA11* | |
| Real-time PCR | *COL1A1* | |
| | *P4HA1* | |
| | *TNF* | |
| | *ANXA11* | |

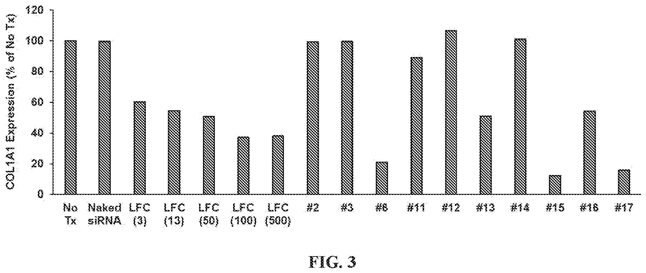

Naked siRNA targeting *COL1A1* did not lower *COL1A1* gene expression compared to untreated fibroblasts (Figure 3). 50, 100, or 500 pmol of the same siRNA formulated in lipofectamine (LFC) reduced *COL1A1* expression by more than 60% relative to untreated fibroblasts (Figure 3). Formulation #2 did not decrease *COL1A1* expression in fibroblasts (Figure 3), consistent with the poor uptake of formulation #2 into fibroblasts. Although formulations #3, #11, and #12 showed good uptake into fibroblasts, none decreased *COL1A1* expression in fibroblasts (Figure 3). In contrast, formulation #6 reduced *COL1A1* expression by nearly 80% compared to untreated fibroblasts (Figure 3).

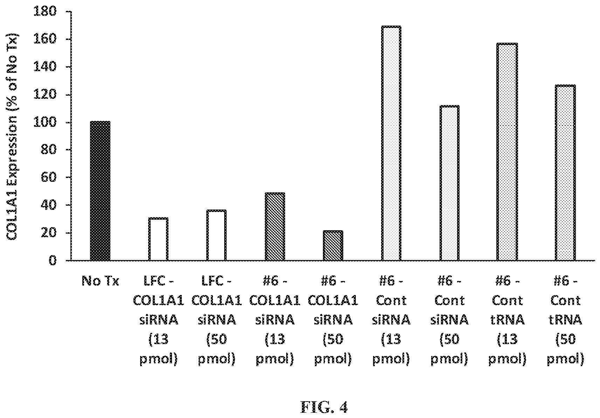

To confirm that the effects of formulation #6 on *COL1A1* expression are due to specific knock-down of the target gene, fibroblasts were incubated for 24 hours with lipofectamine (LFC) or formulation #6 containing either an siRNA targeting *COL1A1,* an siRNA targeting an irrelevant gene, or tRNA, and *COL1A1* expression was measured. Neither the tRNA nor the non-specific siRNA formulation decreased *COL1A1* expression whereas the formulations containing target-specific siRNAs decreased *COL1A1* expression (Figure 4).

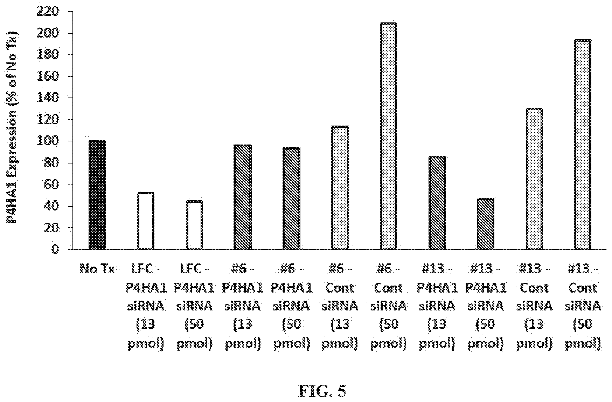

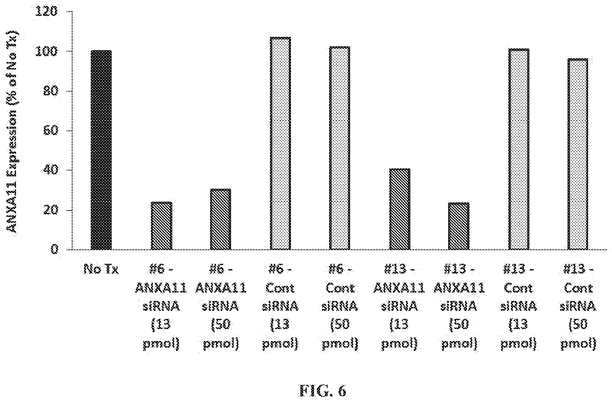

A similar experiment was performed to test the target specific knock-down of formulations #6 and #13 containing siRNAs directed to *P4HA1* and *ANXA11* mRNAs. The formulations containing control siRNAs did not decrease the expression of *P4HA1* and *ANXA11* mRNAs whereas the formulations containing target-specific siRNAs decreased *P4HA1* and *ANXA11* expression (Figures 5 and 6).

These findings demonstrate that nanoparticle-encapsulated siRNAs can be taken up by phagocytic cells to effectively reduce expression of a target gene.

### Example 5 - Effect of siRNA formulations on granuloma formation in in vivo mouse model of sarcoidosis

The ability of nanoparticle-encapsulated siRNAs to decrease granuloma formation and improve lung histopatholgy will be tested in a mouse model of sarcoidosis. An exemplary mouse model of sarcoidosis is described in McCaskill et al., Am J Respir Cell Mol Biol., 2006 Sep; 35(3): 347-356, which is incorporated herein by reference for all purposes. Specifically, *Propionibacterium acnes* (PA) is a gram-positive anaerobic bacterium implicated as a putative etiologic agent of sarcoidosis. To induce sarcoidosis in mice, heat-killed PA will be injected intraperitoneally in C57BL/6 and/or BALB/c mice. Two weeks after intraperitoneal injection, PA-sensitized mice will be challenged with heat-killed PA (e.g. 0.5 mg: 0.05 ml of 10 mg/ml suspension) intratracheally. C57BL/6 and BALB/c mice sensitized and challenged with PBS (PBS/PBS) will be used as controls. Additionally, some mice will either be sensitized to PA but not challenged (intraperitoneal PA/intratracheal PBS), or nonsensitized but challenged (intraperitoneal PBS/intratracheal PA) to determine the impact of sensitization alone as well as challenge alone.

siRNA formulations according to the invention will be administered to mice at various time points to determine the effect of formulations in improving pathophysiology of sarcoidosis, such as decrease in granuloma formation. For example, test and control siRNA formulations will be injected at day 5, day 7, day 10, day 12, and/or day 14 post intra-peritoneal sensitization and day 2, day 5, day 7, day 10, day 14, day 21, and/or day 28 post intratracheal challenge.

McCaskill et al. have shown that mice challenged with PA developed a cellular immune response characterized by elevations in Th1 cytokines/chemokines, increased numbers of lymphocytes and macrophages in lung lavage fluid, and peribronchovascular granulomatous inflammation composed of T- and B-lymphocytes and epithelioid histiocytes, all of which resemble pathophysiology of sarcoidosis.

Mice will be sacrificed at specific time points and various pathological and immunological markers, such as those described in McCaskill et al., will be tested to determine the effect of siRNA formulations on the pathophysiology of sarcoidosis. Additionally, mice will be followed for survival to determine the effect of siRNA formulation on the survival.

### Example 6 Effect of siRNA formulations in in vivo mouse model of pulmonary fibrosis (PF) associated with sarcoidosis

The ability of siRNA formulations to improve the pathophysiology of PF associated with sarcoidosis will be tested in a mouse model. An exemplary mouse model of PF associated with sarcoidosis is described in Jiang et al., Oncotarget, 2016 May; doi: 10.18632/oncotarget.9397 [Epub ahead of print], which is incorporated herein by reference for all purposes. In this model, repeated challenge with *Propionibacterium acnes* (PA) induces persistent inflammation leading to sarcoidosis followed by PF in mice.

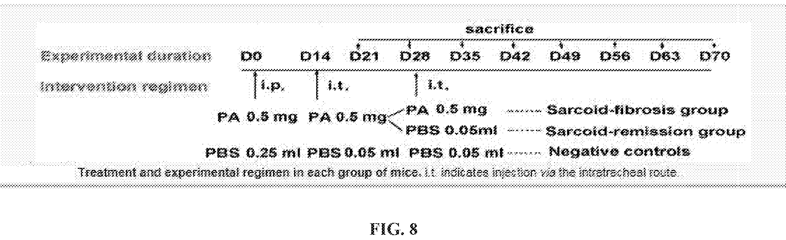

On day 0, 0.25 mL of the 2 mg/mL heat-killed PA suspension (a total of 0.5 mg) will be injected intraperitoneally into mice. On day 14, mice will be anesthetized with 1% sodium pentobarbital and challenged with 0.05 mL of the 10 mg/mL heat-killed PA suspension (a total of 0.5 mg) *via* the intratracheal route. PA inoculation and intratracheal challenge would induce sarcoid-granulomatosis in the lung. Sarcoidosis mice will be given booster challenge on day 28 with another 0.05 mL of the 10 mg/mL heat-killed PA suspension (a total of 0.5 mg) intratracheally for a second challenge; these mice will be designated sarcoid-fibrosis group. Mice administered with 0.05 mL of sterile PBS on day 28 are expected to slow the natural disease course after once PA challenging on day 14; these mice will be designated sarcoid-remission group. Mice inoculated and challenged with sterile PBS (PBS/PBS/PBS) will be used as negative controls. See Figure 8 for the schematic of the mouse model.

siRNA formulations according to the invention will be administered to mice at various time points to determine the effect of formulations in improving pathophysiology of PF associated with sarcoidosis, such as decrease in lung fibrosis and granuloma formation. For example, test and control siRNA formulations will be injected at day 5, day 7, day 10, day 12, and/or day 14 post intra-peritoneal sensitization; day 2, day 5, day 7, day 10, day 14, day 21, and/or day 28 post intratracheal challenge; and day 2, day 5, day 7, day 10, day 14, day 21, and/or day 28 post intratracheal booster dose.

Mice will be sacrificed at specific time points and various pathological and immunological markers, such as those described in McCaskill et al. and Jiang et al., will be tested to determine the effect of siRNA formulations on the pathophysiology of sarcoidosis and pulmonary fibrosis. Additionally, mice will be followed for survival to determine the effect of siRNA formulation on the survival.

### Example 7 - Effect of siRNA formulations in in vivo mouse model of pulmonary fibrosis (PF)

The ability of siRNA formulations to improve the pathophysiology of PF will be tested in a widely used experimental model of pulmonary fibrosis where bleomycin is instilled intratracheally in mice. This model is described in Izbicki et al., Int J Exp Pathol. 2002 Jun; 83(3): 111-119, and Moore and Hogaboam, Am J Physiol Lung Cell Mol Physiol. 2008 Feb; 294(2): L152-60; both of which are incorporated herein by reference for all purposes.

A single dose of bleomycin sulphate (e.g. 0.06 mg in 0.1 mL saline per animal) will be instilled intratracheally in mice on day 0. Control animals will receive 0.1 mL saline alone.

siRNA formulations according to the invention will be administered to mice at various time points to determine the effect of formulations in improving pathophysiology of PF. For example, test and control siRNA formulations will be injected at day 1, day 3, day 5, day 7, day 10, day 12, and/or day 14 post intratracheal instillation.

Mice will be sacrificed at specific time points and various pathological and immunological markers, such as those described in Izbicki et al., will be tested to determine the effect of siRNA formulations on the pathophysiology of pulmonary fibrosis. Additionally, mice will be followed for survival to determine the effect of siRNA formulation on the survival.

## Claims

1. A composition comprising a nucleic acid compound complexed or encapsulated by a lipid particle; wherein the lipid particle comprises:
(a) a cationic lipid comprising 40 mol% to 58 mol% of the total lipid present in the composition;
(b) a neutral lipid comprising 40 mol% to 50 mol% of the total lipid present in the composition; and
(c) a conjugated lipid comprising 0.3 mol% to 1.5 mol% of the total lipid present in the composition,
wherein the nucleic acid compound is an RNA interference (RNAi) compound targeting a messenger RNA (mRNA) that encodes COL1A1 or annexin A11 (ANXA11), and
wherein the cationic lipid comprises 1,2-dioleoyl-3-dimethylammonium-propane (DODAP), the neutral lipid comprises a mixture of i) a phospholipid and ii) cholesterol hemisuccinate (CHEMS) or tocopheryl hemisuccinate (THS), and the conjugated lipid comprises 1,2-dimyristoyl-sn-glycerol-polyethyleneglycol 2000 (DMG-PEG2000).

2. The composition of claim 1, wherein the cationic lipid is present in an amount of 45 to 58 mol%, of the total lipid present in the composition.

3. The composition of claim 1 or 2, wherein the phospholipid is selected from the group consisting of 1,2-distearoyl-sn-glycero-3-phosphocholine (DSPC), 1,2-dioleoyl-sn-glycero-3-phosphoethanolamine-N-(dodecanyl) (NA-DOPE), 1,2-dioleoyl-sn-glycero-3-phosphocholine (DOPC), 1,2-Distearoyl-sn- glycero-3-phosphoethanolamine (DSPE), and a mixture thereof.

4. The composition of any one of claims 1-3, wherein the cationic lipid is present in an amount of 50 to 55 mol%, of the total lipid present in the composition.

5. The composition of any one of claims 1-4, wherein the cationic lipid is present in an amount of 50 mol%, of the total lipid present in the composition.

6. The composition of any one of claims 1-3, wherein the cationic lipid is present in an amount of 55 to 58 mol%, of the total lipid present in the composition.

7. The composition of claim 6, wherein the cationic lipid is present in an amount of 57 mol%, of the total lipid present in the composition.

8. The composition of any one of claims 1-7, wherein the neutral lipid is present in an amount of 41 to 43 mol%, of the total lipid present in the composition.

9. The composition of any one of claims 1-7, wherein the neutral lipid is present in an amount of 49 mol%, of the total lipid present in the composition.

10. The composition of any one of claims 1-9, wherein the conjugated lipid is present in an amount of 1 mol% to 1.5 mol% of the total lipid present in the composition.

11. The composition of claim 1 or 2, wherein the lipid particle comprises:
(a) 1,2-dioleoyl-3-dimethylammonium-propane (DODAP) comprising 50 mol% to 57.5 mol% of the total lipid present in the composition;
(b) a phospholipid comprising 4 mol% to 16.5 mol% of the total lipid present in the composition;
(c) cholesterol hemisuccinate (CHEMS) or tocopherol hemisuccinate (THS) comprising 25 mol% to 45 mol% of the total lipid present in the composition, and
(d) 1,2-dimyristoyl-sn-glycerol-polyethyleneglycol 2000 (DMG-PEG2000) comprising 1 mol% to 1.5 mol% of the total lipid present in the composition,
wherein the phospholipid is selected from the group consisting of 1,2-distearoyl-sn-glycero-3-phosphocholine (DSPC), 1,2-dioleoyl-sn-glycero-3-phosphocholine (DOPC), and 1,2-distearoyl-sn-glycero-3-phosphoethanolamine (DSPE).

12. The composition of any one of claims 1-11, wherein the RNAi compound targeting a messenger RNA (mRNA) that encodes COL1A1 is a small interfering RNA (siRNA) comprising a sense strand comprising the nucleotide sequence of CAG AAG AAC UGG UAC AUC ATT, and an antisense strand comprising the nucleotide sequence of UGA UGU ACC AGU UCU UCU GTT.

13. The composition of any one of claims 1-11, wherein the RNAi compound targeting a messenger RNA (mRNA) that encodes ANXA11 is a small interfering RNA (siRNA) comprising a sense strand comprising the nucleotide sequence of GAU UCA CCG UCC UAG AGC UTT, and an antisense strand comprising the nucleotide sequence of AGC UCU AGG ACG GUG AAU CTT.

14. The composition of any one of claims 1-13, for use in treating a pulmonary fibrosis or sarcoidosis in a patient in need thereof by administering to the lungs of the patient a therapeutically effective amount of the composition.

15. The composition for use of claim 14, wherein the therapeutically effective amount of the composition is administered to the lungs of the patient via inhalation.

## Patentansprüche

1. Zusammensetzung, umfassend eine Nukleinsäureverbindung, die durch ein Lipidteilchen komplexiert oder eingekapselt ist; wobei das Lipidteilchen umfasst:
(a) ein kationisches Lipid, umfassend 40 Mol-% bis 58 Mol-% des Gesamtlipids, das in der Zusammensetzung vorliegt;
(b) ein neutrales Lipid, umfassend 40 Mol-% bis 50 Mol-% des Gesamtlipids, das in der Zusammensetzung vorliegt; und
(c) ein konjugiertes Lipid, umfassend 0,3 Mol-% bis 1,5 Mol-% des Gesamtlipids, das in der Zusammensetzung vorliegt,
wobei die Nukleinsäureverbindung eine RNA-Interferenz-Verbindung (RNAi-Verbindung) ist, die eine Messenger-RNA (mRNA) targetiert, die COL1A1 oder Annexin A11 (ANXA11) kodiert, und
wobei das kationische Lipid 1,2-Dioleoyl-3-dimethylammoniumpropan (DODAP) umfasst, das neutrale Lipid ein Gemisch von i) einem Phospholipid und ii) Cholesterinhemisuccinat (CHEMS) oder Tocopherylhemisuccinat (THS) umfasst und das konjugierte Lipid 1,2-Dimyristoyl-sn-glycerin-Polyethylenglykol 2000 (DMG-PEG2000) umfasst.

2. Zusammensetzung nach Anspruch 1, wobei das kationische Lipid in einer Menge von 45 bis 58 Mol-% des Gesamtlipids, das in der Zusammensetzung vorliegt, vorliegt.

3. Zusammensetzung nach Anspruch 1 oder 2, wobei das Phospholipid aus der Gruppe bestehend aus 1,2-Distearoyl-sn-glycero-3-phosphocholin (DSPC), 1,2-Dioleoyl-sn-glycero-3-phosphoethanolamin-N-(dodecanyl) (NA-DOPE), 1,2-Dioleoyl-sn-glycero-3-phosphocholin (DOPC), 1,2-Distearoyl-sn-glycero-3-phosphoethanolamin (DSPE) und einem Gemisch davon ausgewählt ist.

4. Zusammensetzung nach einem der Ansprüche 1-3, wobei das kationische Lipid in einer Menge von 50 bis 55 Mol-% des Gesamtlipids, das in der Zusammensetzung vorliegt, vorliegt.

5. Zusammensetzung nach einem der Ansprüche 1-4, wobei das kationische Lipid in einer Menge von 50 Mol-% des Gesamtlipids, das in der Zusammensetzung vorliegt, vorliegt.

6. Zusammensetzung nach einem der Ansprüche 1-3, wobei das kationische Lipid in einer Menge von 55 bis 58 Mol-% des Gesamtlipids, das in der Zusammensetzung vorliegt, vorliegt.

7. Zusammensetzung nach Anspruch 6, wobei das kationische Lipid in einer Menge von 57 Mol-% des Gesamtlipids, das in der Zusammensetzung vorliegt, vorliegt.

8. Zusammensetzung nach einem der Ansprüche 1-7, wobei das neutrale Lipid in einer Menge von 41 bis 43 Mol-% des Gesamtlipids, das in der Zusammensetzung vorliegt, vorliegt.

9. Zusammensetzung nach einem der Ansprüche 1-7, wobei das neutrale Lipid in einer Menge von 49 Mol-% des Gesamtlipids, das in der Zusammensetzung vorliegt, vorliegt.

10. Zusammensetzung nach einem der Ansprüche 1-9, wobei das konjugierte Lipid in einer Menge von 1 Mol-% bis 1,5 Mol-% des Gesamtlipids, das in der Zusammensetzung vorliegt, vorliegt.

11. Zusammensetzung nach Anspruch 1 oder 2, wobei das Lipidteilchen umfasst:
(a) 1,2-Dioleoyl-3-dimethylammoniumpropan (DODAP), umfassend 50 Mol-% bis 57,5 Mol-% des Gesamtlipids, das in der Zusammensetzung vorliegt;
(b) ein Phospholipid, umfassend 4 Mol-% bis 16,5 Mol-% des Gesamtlipids, das in der Zusammensetzung vorliegt;
(c) Cholesterinhemisuccinat (CHEMS) oder Tocopherolhemisuccinat (THS), umfassend 25 Mol-% bis 45 Mol-% des Gesamtlipids, das in der Zusammensetzung vorliegt, und
(d) 1,2-Dimyristoyl-sn-glycerin-Polyethylenglykol 2000 (DMG-PEG2000), umfassend 1 Mol-% bis 1,5 Mol-% des Gesamtlipids, das in der Zusammensetzung vorliegt,
wobei das Phospholipid aus der Gruppe bestehend aus 1,2-Distearoyl-sn-glycero-3-phosphocholin (DSPC), 1,2-Dioleoyl-sn-glycero-3-phosphocholin (DOPC) und 1,2-Distearoyl-sn-glycero-3-phosphoethanolamin (DSPE) ausgewählt ist.

12. Zusammensetzung nach einem der Ansprüche 1-11, wobei die RNAi-Verbindung, die eine Messenger-RNA (mRNA) targetiert, die COL1A1 kodiert, eine Smallinterfering-RNA (siRNA) ist, die einen Sense-Strang, umfassend die Nukleotidsequenz von CAG AAG AAC UGG UAC AUC ATT, und einen Antisense-Strang, umfassend die Nukleotidsequenz von UGA UGU ACC AGU UCU UCU GTT, umfasst.

13. Zusammensetzung nach einem der Ansprüche 1-11, wobei die RNAi-Verbindung, die eine Messenger-RNA (mRNA) targetiert, die ANXA11 kodiert, eine Smallinterfering-RNA (siRNA) ist, die einen Sense-Strang, umfassend die Nukleotidsequenz von GAU UCA CCG UCC UAG AGC UTT, und einen Antisense-Strang, umfassend die Nukleotidsequenz von AGC UCU AGG ACG GUG AAU CTT, umfasst.

14. Zusammensetzung nach einem der Ansprüche 1-13 zur Verwendung bei der Behandlung einer Lungenfibrose oder -sarkoidose bei einem Patienten, der dieser bedarf, durch Verabreichen einer therapeutisch wirksamen Menge der Zusammensetzung an die Lungen des Patienten.

15. Zusammensetzung zur Verwendung nach Anspruch 14, wobei die therapeutisch wirksame Menge der Zusammensetzung mittels Inhalation an die Lungen des Patienten verabreicht wird.

## Revendications

1. Composition comprenant un composé acide nucléique complexé ou encapsulé par une particule de lipides ; dans laquelle la particule de lipides comprend :
(a) un lipide cationique constituant 40 % en mole à 58 % en mole des lipides totaux présents dans la composition ;
(b) un lipide neutre constituant 40 % en mole à 50 % en mole des lipides totaux présents dans la composition ; et
(c) un lipide conjugué constituant 0,3 % en mole à 1,5 % en mole des lipides totaux présents dans la composition,
dans laquelle le composé acide nucléique est un composé ARN interférence (ARNi) ciblant un ARN messager (ARNm) qui code pour COL1A1 ou l'annexine A11 (ANXA11) et
dans laquelle le lipide cationique comprend du 1,2-dioléoyl-3-diméthylammonium-propane (DODAP), le lipide neutre comprend un mélange de i) un phospholipide et ii) de l'hémisuccinate de cholestérol (CHEMS) ou de l'hémisuccinate de tocophéryle (THS) et le lipide conjugué comprend du 1,2-dimyristoyl-*sn*-glycérol-polyéthylèneglycol 2000 (DMG-PEG2000).

2. Composition selon la revendication 1, dans laquelle le lipide cationique est présent en une quantité de 45 à 58 % en mole, des lipides totaux présents dans la composition.

3. Composition selon la revendication 1 ou 2, dans laquelle le phospholipide est choisi dans le groupe constitué par la 1,2-distéaroyl-sn-glycéro-3-phosphocholine (DSPC), la *N-*(dodécanyl)-1,2-dioléoyl-sn-glycéro-3-phosphoéthanolamine (NA-DOPE), la 1,2-dioléoyl-sn-glycéro-3-phosphocholine (DOPC), la 1,2-distéaroyl-sn-glycéro-3-phosphoéthanolamine (DSPE) et un mélange de celles-ci.

4. Composition selon l'une quelconque des revendications 1-3, dans laquelle le lipide cationique est présent en une quantité de 50 à 55 % en mole, des lipides totaux présents dans la composition.

5. Composition selon l'une quelconque des revendications 1-4, dans laquelle le lipide cationique est présent en une quantité de 50 % en mole, des lipides totaux présents dans la composition.

6. Composition selon l'une quelconque des revendications 1-3, dans laquelle le lipide cationique est présent en une quantité de 55 à 58 % en mole, des lipides totaux présents dans la composition.

7. Composition selon la revendication 6, dans laquelle le lipide cationique est présent en une quantité de 57 % en mole, des lipides totaux présents dans la composition.

8. Composition selon l'une quelconque des revendications 1-7, dans laquelle le lipide neutre est présent en une quantité de 41 à 43 % en mole, des lipides totaux présents dans la composition.

9. Composition selon l'une quelconque des revendications 1-7, dans laquelle le lipide neutre est présent en une quantité de 49 % en mole, des lipides totaux présents dans la composition.

10. Composition selon l'une quelconque des revendications 1-9, dans laquelle le lipide conjugué est présent en une quantité de 1 % en mole à 1,5 % en mole des lipides totaux présents dans la composition.

11. Composition selon la revendication 1 ou 2, dans laquelle la particule de lipides comprend :
(a) du 1,2-dioléoyl-3-diméthylammonium-propane (DODAP) constituant 50 % en mole à 57,5 % en mole des lipides totaux présents dans la composition ;
(b) un phospholipide constituant 4 % en mole à 16,5 % en mole des lipides totaux présents dans la composition ;
(c) de l'hémisuccinate de cholestérol (CHEMS) ou de l'hémisuccinate de tocophérol (THS) constituant 25 % en mole à 45 % en mole des lipides totaux présents dans la composition et
(d) du 1,2-dimyristoyl-sn-glycérol-polyéthylèneglycol 2000 (DMG-PEG2000) constituant 1 % en mole à 1,5 % en mole des lipides totaux présents dans la composition,
dans laquelle le phospholipide est choisi dans le groupe constitué par la 1,2-distéaroyl-sn-glycéro-3-phosphocholine (DSPC), la 1,2-dioléoyl-sn-glycéro-3-phosphocholine (DOPC) et la 1,2-distéaroyl-sn-glycéro-3-phosphoéthanolamine (DSPE).

12. Composition selon l'une quelconque des revendications 1-11, dans laquelle le composé ARNi ciblant un ARN messager (ARNm) qui code pour COL1A1 est un petit ARN interférent (pARNi) comprenant un brin sens comprenant la séquence de nucléotides CAG AAG AAC UGG UAC AUC ATT et un brin antisens comprenant la séquence de nucléotides UGA UGU ACC AGU UCU UCU GTT.

13. Composition selon l'une quelconque des revendications 1-11, dans laquelle le composé ARNi ciblant un ARN messager (ARNm) qui code pour ANXA11 est un petit ARN interférent (pARNi) comprenant un brin sens comprenant la séquence de nucléotides GAU UCA CCG UCC UAG AGC UTT et un brin antisens comprenant la séquence de nucléotides AGC UCU AGG ACG GUG AAU CTT.

14. Composition selon l'une quelconque des revendications 1-13, destinée à être utilisée en traitement d'une fibrose pulmonaire ou de la sarcoïdose chez un patient qui en a besoin par administration aux poumons du patient d'une quantité thérapeutiquement efficace de la composition.

15. Composition destinée à être utilisée selon la revendication 14, la quantité thérapeutiquement efficace de la composition étant administrée aux poumons du patient par inhalation.
